# EUROPEAN PATENT APPLICATION

(11) **EP 4 541 805 A1**
(43) Date of publication of application: **23.04.2025**
(21) Application number: 22946935.8
(22) Date of filing: 07.12.2022
(51) Int. Cl.: C07K 7/08, C07K 7/54, C12N 5/07

(54) **PEPTIDE AND PEPTIDE-CONTAINING AGENT**

(30) Priority: 15.06.2022 JP 2022096921
(71) Applicant: PeptiDream Inc., Kawasaki-shi, Kanagawa 210-0821 (JP)
(72) Inventor: SUZUKI Yoshinori, Kawasaki-shi, Kanagawa 210-0821 (JP); SHIBATA Yoshihiro, Kawasaki-shi, Kanagawa 210-0821 (JP)
(74) Representative: van Dam, Vincent
(86) International application number: PCT/JP2022/045052
(87) International publication number: WO 2023/243120

(57) **Abstract**

[Problem] To provide a novel peptide having a BMP4 binding ability.

[Solution] The present invention pertains to a peptide and a peptide-containing agent. The peptide according to the present invention has an amino acid sequence of E-R-V-F4COO-Atp-D-R-Bph-P-Y-P-Bph-Y-F4COO-F4COO-S-C (SEQ ID NO: 1) or has an amino acid sequence resulting from substitution, addition, deletion, or insertion at 1-15 amino acid residues selected from the group consisting of amino acid residues at positions 1, 2, 4, 5, 6, 7, 8, 9, 10, 11, 12, 13, 14, 15, and 16 in said amino acid sequence.

## Description

### TECHNICAL FIELD

The present invention relates to a peptide, an agent containing the peptide, and the like.

### BACKGROUND ART

Stem cells having pluripotency, such as embryonic stem cells (ES cells) and induced pluripotent stem cells (iPS cells), have been reported so far. Regenerative medicine and the creation of disease models have been attempted using cells that have been differentiated and induced from these pluripotent stem cells.

Growth factors are important signaling molecules involved in embryogenesis, tissue repair, and the regulation of intercellular communication, and many growth factors have been identified that play important roles in the survival, self-renewal, differentiation, and dedifferentiation of stem cells. By combining such growth factors, stem cell culture can be regulated in various ways.

Bone morphogenetic proteins (BMPs) are secreted signaling molecules that belong to the TGF-β superfamily. BMPs were originally discovered as regulators of cartilage and bone formation. It is revealed that there are at least 20 types of BMPs, which act as growth factors. Noggin is a secreted protein that has BMP inhibitory activity. Noggin binds to BMPs themselves and prevents BMPs from binding to their receptors, thereby inhibiting BMP signal transduction and affecting the differentiation and induction of cells. For example, when human ES cells are cultured in a Noggin-added culture medium, the action of BMP-2 endogenously produced by the ES cells is inhibited, thereby inhibiting the differentiation of the ES cells into extraembryonic entodermal cells and maintaining the undifferentiated state of the ES cells. Then, by continuing to culture them under neurodifferentiation culture conditions, the differentiation induction into neural cells is promoted (Patent Literature 1). In addition to Noggin, Chordin, follistatin, and the like are known as secreted proteins that act as such BMP inhibitors. However, since these are proteins, there are problems such as high culture costs and poor reproducibility when they are used for cell culture.

### CITATION LIST

### PATENT LITERATURE

Patent Literature 1: WO 01/98463

### SUMMARY OF INVENTION

### TECHNICAL PROBLEM

As a result of intensive studies for the purpose of creating a peptide having BMP binding activity, the present inventors have conceived the present invention. The present invention provides a novel peptide, various agents containing the peptide, and use of the peptide.

### SOLUTION TO PROBLEM

The above problem is solved by a novel peptide or the like having a sequence set forth in SEQ ID NO: 1 or the like.

The invention described in this specification relates to the following peptide or a pharmaceutically acceptable salt thereof.

A peptide or a pharmaceutically acceptable salt thereof, the peptide including:
an amino acid sequence described below:
E-R-V-F4COO-Atp-D-R-Bph-P-Y-P-Bph-Y-F4COO-F4COO-S-C (SEQ ID NO: 1); or
an amino acid sequence resulting from substitution, addition, deletion, or insertion of 1 to 15 amino acid residues selected from the group consisting of amino acid residues at positions 1, 2, 4, 5, 6, 7, 8, 9, 10, 11, 12, 13, 14, 15, and 16 of the amino acid sequence described above.

The invention described in this specification relates to the following peptide or a pharmaceutically acceptable salt thereof.

A peptide or a pharmaceutically acceptable salt thereof, the peptide including:
an amino acid sequence described below:
X1-X2-X3-X4-X5-X6-X7-X8-X9-X10-X11-X12-X13-X14-X15-X16-X17 (SEQ ID NO: 2); or
an amino acid sequence resulting from substitution, deletion, addition, or insertion of 1 to 10 amino acid sequences from the amino acid sequence set forth in SEQ ID NO: 2,
wherein X1 is E, A, T, Q, or L,
X2 is R, K, Hgl, any polar amino acid, or any aliphatic amino acid,
X3 is V,
X4 is an amino acid having an aromatic ring that may be a heterocycle at a side chain,
X5 is any aliphatic amino acid, Y, D, Atp, Cha4tH, Gthp, or A1Ac4pip,
X6 is D, any basic amino acid, or any polar amino acid,
X7 is any basic amino acid or any polar amino acid,
X8 is biphenylalanine that may have a substituent and in which C (carbon atom) on an aromatic ring may be substituted with N (nitrogen atom),
X9 is any amino acid,
X10 is Y, Hty, or 4Py,
X11 is any amino acid,
X12 is biphenylalanine that may have a substituent and in which C (carbon atom) on an aromatic ring may be substituted with N(nitrogen atom),
X13 is unsubstituted or substituted F, 4Py, or 3Py6NH2,
X14 is F4COO, D, or G,
X15 is F4COO, Y, 4Py, 3Py6NH2, W7N, Hph, or N,
X16 is any amino acid, and
X17 is C (Cys).

The peptide including an amino acid sequence resulting from substitution, deletion, addition, or insertion of 1 to 10 amino acid sequences from the amino acid sequence set forth in SEQ ID NO: 2 preferably has BMP4 binding ability as described below. In addition, it may be a peptide or a pharmaceutically acceptable salt thereof, the peptide including an amino acid sequence resulting from substitution, deletion, addition, or insertion of 1 to 9 (1 to 8, 1 to 5, 1 to 4, 1 to 3, 2, or 1) amino acid sequences from the amino acid sequence set forth in SEQ ID NO: 2.

A preferable example of the above peptide or pharmaceutically acceptable salt thereof is a peptide or a pharmaceutically acceptable salt thereof,
wherein X1 is E,
X2 is R or any polar amino acid,
X3 is V,
X4 is unsubstituted or substituted Y or F4COO,
X5 is V or Atp,
X6 is D or KCOpipzaa,
X7 is R,
X8 is Bph,
X9 is P or S,
X10 is Y or Hty,
X11 is any aliphatic amino acid,
X12 is unsubstituted or substituted Bph,
X13 is unsubstituted or substituted F,
X14 is F4COO,
X15 is F4COO or 4Py,
X16 is any amino acid, and
X17 is C.

A preferable example of the above peptide or pharmaceutically acceptable salt thereof is a peptide or a pharmaceutically acceptable salt thereof,
wherein X1 is E, A, T, Q, or L,
X2 is R or KCOpipzaa,
X3 is V,
X4 is Y, F4F, F4COO, 4Py, H, or F4aaO,
X5 is any aliphatic amino acid, Atp, Cha4tH, Gthp, or A1Ac4pip,
X6 is D, S, or KCOpipzaa,
X7 is R or K,
X8 is 3Py6Ph, Bph, Bph4C, pBph2F, or Bph3C,
X9 is S, P, KCOpipzaa, or H,
X10 is Y or Hty,
X11 is any aliphatic amino acid or Hyp,
X12 is biphenylalanine that may have a substituent,
X13 is unsubstituted or substituted F or 4Py,
X14 is F4COO,
X15 is F4COO or 4Py,
X16 is S, and
X17 is C.

A preferable example of the above peptide or pharmaceutically acceptable salt thereof is a peptide or a pharmaceutically acceptable salt thereof,
wherein the peptide includes an amino acid sequence described below: X1-X2-X3-X4-X5-X6-X7-X8-X9-X10-X11-X12-X13-X14-X15-X16-C (SEQ ID NO: 4), or an amino acid sequence resulting from substitution, deletion, addition, or insertion of 1 to 10 amino acid sequences from the amino acid sequence set forth in SEQ ID NO: 4, and
wherein X1 is A,
X2 is R, KAc, Q, or KCOpipzaa,
X3 is V,
X4 is Y, F4F, or 4Py,
X5 is V,
X6 is D, KCOpipzaa, K, R, G, or H,
X7 is R, K, KAc, 4Py, S, or Cit,
X8 is 3Py6Ph or Bph,
X9 is P, S, K, H, G, S, or KCOpipzaa,
X10 is Y,
X11 is any aliphatic amino acid,
X12 is Bph or 3Py6Ph,
X13 is Y or F4F,
X14 is F4COO,
X15 is F4COO or 4Py, and
X16 is S.

The peptide or the pharmaceutically acceptable salt thereof, the peptide including an amino acid sequence resulting from substitution, deletion, addition, or insertion of 1 to 10 amino acid sequences from the amino acid sequence set forth in SEQ ID NO: 4, preferably has BMP4 binding ability as described below. In addition, it may be a peptide or a pharmaceutically acceptable salt thereof, the peptide including an amino acid sequence resulting from substitution, deletion, addition, or insertion of 1 to 9 (1 to 8, 1 to 5, 1 to 4, 1 to 3, 2, or 1) amino acid sequences from the amino acid sequence set forth in SEQ ID NO: 4.

A preferable example of the above peptide or pharmaceutically acceptable salt thereof is a peptide or a pharmaceutically acceptable salt thereof, having binding ability to BMP4.

A preferable example of the above peptide or pharmaceutically acceptable salt thereof is a peptide or a pharmaceutically acceptable salt thereof, having inhibitory activity of BMP4.

A preferable example of the above peptide is a peptide or a pharmaceutically acceptable salt thereof,
wherein the peptide includes an amino acid sequence set forth in any one of SEQ ID NOs: 5 to 187 and SEQ ID NOs: 243 to 421, or
wherein the peptide includes an amino acid sequence resulting from substitution, deletion, addition, or insertion of 1 to 10 amino acid sequences from the amino acid sequence set forth in any one of SEQ ID NOs: 5 to 187 and SEQ ID NOs: 243 to 421, and has binding ability to BMP4 or inhibitory activity of BMP4.

The invention described in this specification relates to the following peptide or a pharmaceutically acceptable salt thereof.

A peptide or a pharmaceutically acceptable salt thereof, the peptide including:
an amino acid sequence described below:
F-G-MeF-G-Bph-G-D-D-Cha-A-MeF-L-H-C (SEQ ID NO: 241); or
an amino acid sequence resulting from substitution, addition, deletion, or insertion of 1 to 12 amino acid residues selected from the group consisting of amino acid residues at positions 1, 3, 4, 5, 6, 7, 8, 9, 10, 11, 12, and 13 of the amino acid sequence described above.

The invention described in this specification relates to the following peptide.

A peptide or a pharmaceutically acceptable salt thereof, the peptide including:
an amino acid sequence described below:
X1-X2-X3-X4-X5-X6-X7-X8-X9-X10-X11-X12-X13-X14 (SEQ ID NO: 242); or
an amino acid sequence resulting from substitution, deletion, addition, or insertion of 1 to 12 amino acid sequences from the amino acid sequence set forth in SEQ ID NO: 242,
wherein X1 is phenylalanine that may have a substituent and in which C on an aromatic ring may be substituted with N,
X2 is G,
X3 is N-methylphenylalanine that may have a substituent,
X4 is G, an aliphatic amino acid or a polar amino acid that may be a D form,
X5 is biphenylalanine in which C on an aromatic ring may be substituted with N or phenylalanine that may have a substituent,
X6 is G, or A that may be a D form,
X7 is any polar amino acid,
X8 is any acidic amino acid,
X9 is any aliphatic amino acid,
X10 is any amino acid,
X11 is N-methylphenylalanine that may have a substituent,
X12 is any aliphatic amino acid,
X13 is any amino acid, and
X14 is C.

A preferable example of the above peptide is a peptide or a pharmaceutically acceptable salt thereof, having inhibitory activity of BMP7.

A preferable example of the above peptide is a peptide or a pharmaceutically acceptable salt thereof,
wherein the peptide includes an amino acid sequence set forth in any one of SEQ ID NOs: 322 to 421, or
wherein the peptide includes an amino acid sequence resulting from substitution, deletion, addition, or insertion of 1 to 10 amino acid sequences from the amino acid sequence set forth in any one of SEQ ID NOs: 322 to 421, and has inhibitory activity of BMP7.

A preferable example of the above peptide is a cyclic peptide or a pharmaceutically acceptable salt thereof.

A preferable example of the above peptide is a peptide or a pharmaceutically acceptable salt thereof,
wherein the peptide includes a cyclic structure in which a chloroacetylated amino acid and a cysteine residue contained in the peptide are bound.

A preferable example of the above peptide is a peptide or a pharmaceutically acceptable salt thereof, further including an additional amino acid residue sequence. An example of the additional amino acid residue sequence is a linker.

A preferable example of use of the above peptide is a BMP4 and/or BMP7 signal transduction inhibitor including the peptide according to any one of the above peptides or the pharmaceutically acceptable salt thereof.

A preferable example of use of the above peptide is a human stem cell differentiation induction regulator including the peptide according to any one of the above peptides or the pharmaceutically acceptable salt thereof.

A preferable example of use of the peptide or the pharmaceutically acceptable salt thereof, the BMP4 and/or BMP7 signal transduction inhibitor, and the human stem cell differentiation induction regulator described above is a culture medium supplement including one or two or more kinds of the above. The addition of the above to a culture medium can inhibit BMP4 and/or BMP7 signal transduction as a target or can regulate differentiation induction of human stem cells.

A preferable example of use of the peptide or the pharmaceutically acceptable salt thereof described above is a method for inhibiting signal transduction activity of BMP4 and/or BMP7 using the peptide according to any one of the above peptides or the pharmaceutically acceptable salt thereof. For example, the administration of any of the peptides described above to a target (for example, a human or a non-human animal) can inhibit signal transduction activity of BMP4 and/or BMP7 in the target. In addition, the administration of any of the peptides described above to a target cell in a test tube or in an in vivo experiment can inhibit signal transduction activity of BMP4 and/or BMP7 in the target cell.

A preferable example of use of the peptide or the pharmaceutically acceptable salt thereof described above is a method for regulating differentiation induction of human stem cells using any of the peptides or the pharmaceutically acceptable salt thereof described above. For example, the administration of any of the peptides or the pharmaceutically acceptable salt thereof described above to a target (for example, a human) can regulate differentiation induction of human stem cells in the target. In addition, the administration of any of the peptides described above to a target cell in a test tube or in an in vivo experiment can regulate differentiation induction of human stem cells in the target cell.

### ADVANTAGEOUS EFFECTS OF INVENTION

The peptide or the pharmaceutically acceptable salt thereof of the present invention has BMP4 binding ability and inhibitory activity of BMP4, preferably has BMP4 signal transduction inhibitory activity, and more preferably has activity of regulating differentiation induction of human stem cells. The peptide or the pharmaceutically acceptable salt thereof of the present invention has BMP7 signal transduction inhibitory activity, and preferably has activity of regulating differentiation induction of human stem cells. The peptide or the pharmaceutically acceptable salt thereof of the present invention can be used for, for example, a culture medium for culturing human stem cells.

### BRIEF DESCRIPTION OF DRAWINGS

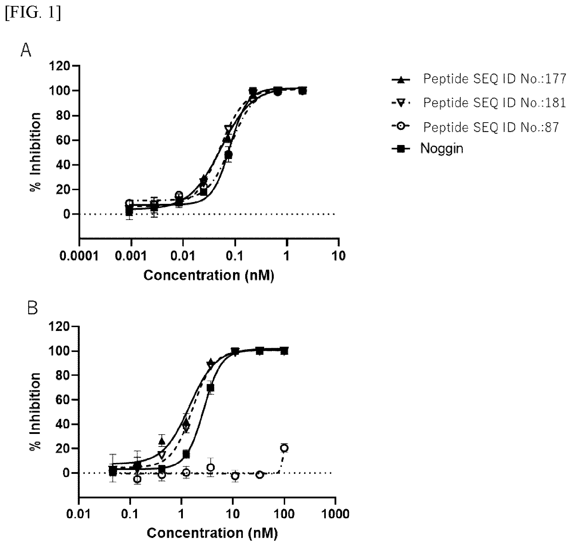

FIG. 1 is graphs showing evaluation results of BMP signal transduction inhibition caused by phosphorylation of SMAD1 using the peptide of the present invention and Noggin. A shows evaluation results of BMP4 signal transduction inhibition, and B shows evaluation results of BMP7 signal transduction inhibition. In the figure, the black triangles show BMP4 signal transduction inhibition peptide No. 177, the white triangles show BMP4 signal transduction inhibition peptide No. 181, the white circles show BMP4 signal transduction inhibition peptide No. 87, and the black squares show Noggin. The horizontal axis shows a concentration of the peptide (nM), and the vertical axis shows an inhibition rate when the phosphorylation value of SMAD1 in an unstimulated state is 100% inhibition and the maximum value of phosphorylation of SMAD1 induced by BMP is 0% inhibition.

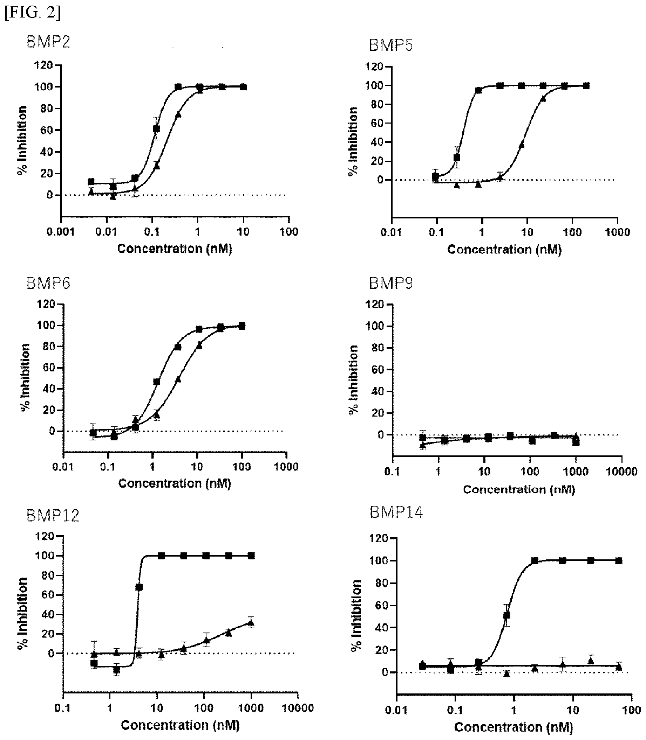

FIG. 2 is graphs showing evaluation results of BMP family signal transduction inhibition caused by phosphorylation of SMAD 1 using the peptide of the present invention and Noggin. In the figure, the triangles show BMP4 signal transduction inhibition peptide No. 177, and the squares show Noggin. The horizontal axis shows a concentration of the peptide (nM), and the vertical axis shows an inhibition rate when the phosphorylation value of SMAD1 in an unstimulated state is 100% inhibition and the maximum value of phosphorylation of SMAD1 induced by BMP is 0% inhibition.

### DESCRIPTION OF EMBODIMENTS

### 1. Abbreviations

In the present specification, the following abbreviations are used with the following meanings unless otherwise stated.

### Abbreviations (general)

Å for angstrom (unit);
BSA for bovine serum albumin;
Boc for tert-butoxycarbonyl group;
ClAc for chloroacetyl;
DCM for dichloromethane or methylene chloride;
DIPCI for N,N'-diisopropylcarbodiimide;
DIPEA or DIEA for N,N-diisopropylethylamine;
DMSO for dimethyl sulfoxide;
DMF for N,N-dimethylformamide;
DODT for 3,6-dioxa-1,8-octanedithiol;
DMEM for Dulbecco's Modified Eagle's Medium;
EC50 for 50% effective concentration;
FBS for fetal bovine serum;
Fmoc for 9-fluorenylmethyloxycarbonyl;
g for gram (unit);
HATU for O-(7-azabenzotriazol-1-yl)-N,N,N',N'-tetramethyluronium hexafluorophosphate;
HPLC for high-performance liquid chromatography;
LC-MS or LC/MS for liquid chromatography-mass spectrometer;
M for molar (unit);
mg for milligram (unit);
mL for milliliter (unit);
mM for millimolar (unit);
MeCN for acetonitrile;
min for minute (unit);
mm for millimeter (unit);
Mpe group for O-3-methyl-pent-3-yl group;
NHS for N-hydroxysuccinimide;
nm for nanometer (unit);
µL for microliter (unit);
OSu for Oxisuccinimide;
Pbf for 2,2,4,6,7-pentamethyldihydrobenzofuran-5-sulfonyl group;
PEG for polyethylene glycol;
rpm for revolutions per minute (unit);
tBu for tert-butyl group;
TFA for trifluoroacetic acid;
TIS for triisopropylsilane; and
Trt or Tr for trityl group.

### Abbreviations (non-natural amino acids)

The following abbreviations for non-natural amino acids include those in which the amino group of the main chain is protected with a general protecting group such as Boc group or Fmoc group.
3Py6NH2 or 3Py6-NH2: (S)-2-amino-3-(6-aminopyridin-3-yl)propanoic acid (CAS No.: 1269968-61-7);
Aib: 2-amino-2-methylpropanoic acid (CAS No.: 62-57-7);
aMeP: (S)-2-methylpyrrolidine-2-carboxylic acid (CAS No.: 42856-71-3);
Aze: (S)-azetidine-2-carboxylic acid (CAS No.: 2133-34-8);
Cit: L-citrulline (CAS No.: 372-75-8);
F3aa: (S)-2-amino-3-(3-(carboxymethyl)phenyl)propanoic acid (CAS No.: 1270107-31-7);
F44pip: (S)-2-amino-3-(4-(piperidin-4-yl)phenyl)propanoic acid (CAS No.: 1888705-98-3);
F4G: (S)-2-amino-3-(4-guanidinophenyl)propanoic acid (CAS No.: 59574-11-7);
Hcit: N6-carbamoyl-L-lysine (CAS No.: 1190-49-4);
Hph: (S)-2-amino-4-phenylbutanoic acid (CAS No.: 943-73-7);
Hpr: (S)-piperidine-2-carboxylic acid (CAS No.: 3105-95-1);
Hty: homo-L-tyrosine (CAS No.: 221243-01-2);
5Ind: (S)-2-amino-3-(1H-indol-5-yl)propanoic acid (CAS No.: 460096-38-2);
KCOpipzaa: N6-(4-(carboxymethyl)piperazine-1-carbonyl)-L-lysine (KISHIDA CHEMICAL CO.,LTD.);
MeA: N-methyl-L-alanine (CAS No.: 3913-67-5);
MeKCOpipzaa: N6-(4-(carboxymethyl)piperazine-1-carbonyl)-N2-methyl-L-lysine (KISHIDA CHEMICAL CO.,LTD.);
MeV: N-methyl-L-valine (CAS No.: 2480-23-1);
P4F2: (S)-4,4-difluoropyrrolidine-2-carboxylic acid (CAS No.: 52683-81-5);
Pc4F: (2S,4S)-4-fluoropyrrolidine-2-carboxylic acid (CAS No.: 2438-57-5);
Pt4F: (2S,4R)-4-fluoropyrrolidine-2-carboxylic acid (CAS No.: 2507-61-1);
W7N: (S)-2-amino-3-(1H-pyrrolo[2,3-b]pyridin-3-yl)propanoic acid (CAS No.: 49758-35-2);
Y3COO: (S)-5-(2-amino-2-carboxyethyl)-2-hydroxybenzoic acid (CAS No.: 4303-95-1);
Y3CONmm: (S)-2-amino-3-(4-hydroxy-3-(methylcarbamoyl)phenyl)propanoic acid;
OCOmPEG10000: methoxypolyethylene glycol carbonate (average molecular weight 10,000);
PEG12Me: 2,5,8,11,14,17,20,23,26,29,32,35-dodecaoxaheptatriacontane-37-amine;
3Py: (S)-2-amino-3-(pyridin-3-yl)propanoic acid (CAS No.: 64090-98-8);
3Py6Ph: (S)-2-amino-3-(6-phenylpyridin-3-yl)propanoic acid (CAS No.: 1335566-07-8);
4Py: 3-(4-pyridyl)-L-alanine (CAS No.: 37535-49-2);
4Py2NH2: (S)-2-amino-3-(2-aminopyridin-4-yl)propanoic acid (CAS No.: 1269969-46-1);
A1Ac4pip: (S)-3-(1-acetylpiperidin-4-yl)-2-aminopropanoic acid (CAS No.: 2349666-59-5);
Abu: (S)-2-aminobutanoic acid (CAS No.: 1492-24-6);
Acpe: (S)-2-amino-3-cyclopentylpropanoic acid (CAS No.: 99295-82-6);
Ahp: (S)-2-aminoheptanoic acid (CAS No.: 44902-02-5);
Atp: (2S)-2-amino-3-(oxan-4-yl)propanoic acid (CAS No.: 1344910-91-3);
bA: 3-aminopropionic acid (CAS No.: 107-95-9);
Bph: (S)-3-([1,1'-biphenyl]-4-yl)-2-aminopropanoic acid (CAS No.: 155760-02-4);
Bph3C: (S)-2-amino-3-(3'-chloro-[1,1'-biphenyl]-4-yl)propanoic acid (Fmoc protecting body CAS No.: 1380437-47-7);
Bph3F: (S)-2-amino-3-(3'-fluoro-[1,1'-biphenyl]-4-yl)propanoic acid (CAS No.: 1380437-41-1);
Bph3Me: (S)-2-amino-3-(3'-methyl-[1,1'-biphenyl]-4-yl)propanoic acid (CAS No.: 1241680-67-0);
Bph3Ome: (S)-2-amino-3-(3'-methoxy-[1,1'-biphenyl]-4-yl)propanoic acid (CAS No.: 1417316-70-1);
Bph4C: (S)-2-amino-3-(4'-chloro-[1,1'-biphenyl]-4-yl)propanoic acid (CAS No.: 1270097-85-2);
Bph4F: (S)-2-amino-3-(4'-fluoro-[1,1'-biphenyl]-4-yl)propanoic acid (CAS No.: 2230870-07-0);
Cba: (S)-2-amino-3-cyclobutylpropanoic acid (CAS No.: 1201593-65-8);
CF3A: (S)-2-amino-4,4,4-trifluorobutanoic acid (CAS No.: 15960-05-1);
Cha: (S)-2-amino-3-cyclohexylpropanoic acid (CAS No.: 27527-05-5);
Cha4tH: (S)-2-amino-3-((1r,4S)-4-hydroxycyclohexyl)propanoic acid (CAS No.: 221243-22-7);
Chg: (S)-2-amino-2-cyclohexylacetic acid (CAS No.: 14328-51-9);
da: D-alanine (CAS No.: 338-69-2);
de: D-glutamic acid (CAS No.: 6893-26-1);
ds: D-serine (CAS No.: 312-84-5);
F3C: (S)-2-amino-3-(3-chlorophenyl)propanoic acid (CAS No.: 80126-51-8);
F3Me: (S)-2-amino-3-(m-toluyl)propanoic acid (CAS No.: 114926-37-3);
F42Py: (S)-2-amino-3-(4-(pyridin-2-yl)phenyl)propanoic acid (CAS No.: 1336207-47-6);
F43Py: (S)-2-amino-3-(4-(pyridin-3-yl)phenyl)propanoic acid (CAS No.: 916911-74-5);
F44Py: (S)-2-amino-3-(4-(pyridin-4-yl)phenyl)propanoic acid (Fmoc protecting body CAS No.: 1282042-56-1);
F44thp: (S)-2-amino-3-(4-(tetrahydro-2H-pyran-4-yl)phenyl)propanoic acid (methyl ester protecting body CAS No.: 938197-48-9);
F4aao: (S)-2-amino-3-(4-(carboxymethoxy)phenyl)propanoic acid (CAS No.: 24558-63-2);
F4C: (S)-2-amino-3-(4-chlorophenyl)propanoic acid (CAS No.: 14173-39-8);
F4COO: (S)-4-(2-amino-2-carboxyethyl)benzoic acid (CAS No.: 126109-42-0);
F4F: (S)-2-amino-3-(4-fluorophenyl)propanoic acid (CAS No.: 1132-68-9);
F4Me: (S)-2-amino-3-(p-toluyl)propanoic acid (CAS No.: 1991-87-3);
Gcpr: (S)-2-amino-2-cyclopropylacetic acid (CAS No.: 49606-99-7);
Gthp: (S)-2-amino-2-(tetrahydro-2H-pyran-4-yl)acetic acid (CAS No.: 811842-25-8);
Hgl: L-2-aminoadipic acid (CAS No.: 1118-90-7);
Hgn: (S)-2,6-diamino-6-oxohexanoic acid (CAS No.: 7433-32-1);
Hty: homo-L-tyrosine (CAS No.: 221243-01-2);
Hyp: (2S,4R)-4-hydroxypyrrolidine-2-carboxylic acid (CAS No.: 51-35-4);
KCOpipzaa: N6-(4-(carboxymethyl)piperazine-1-carbonyl)-L-lysine (Fmoc-protected/tert-butyl ester protecting form CAS No.: 2680607-34-3);
MeF: methyl-L-phenylalanine (CAS No.: 2566-30-5);
MeF3C: (S)-3-(3-chlorophenyl)-2-(methylamino)propanoic acid (CAS No.: 2255324-91-3);
MeF3Me: (S)-2-(methylamino)-3-(m-toluyl)propanoic acid (Fmoc protecting body CAS No.: 2642331-20-0);
MeF4C: (S)-3-(4-chlorophenyl)-2-(methylamino)propanoic acid (CAS No.: 347851-70-1);
MeF4Me: (S)-2-(methylamino)-3-(p-toluyl)propanoic acid (CAS No.: 2307782-25-6);
MeY: methyl-L-tyrosine (CAS No.: 537-49-5);
P4Sh: (2S,4S)-4-hydroxypyrrolidine-2-carboxylic acid (CAS No.: 618-27-9);
pBph2F: (S)-2-amino-3-(2-fluoro-[1,1'-biphenyl]-4-yl)propanoic acid (CAS No.: 1389900-88-2); and
pBph3F: (S)-2-amino-3-(3-fluoro-[1,1'-biphenyl]-4-yl)propanoic acid.

### 2. Peptide (A)

One embodiment of the present invention relates to a peptide or a pharmaceutically acceptable salt thereof.

In one embodiment, the peptide of the present invention includes the following amino acid sequence:
E-R-V-F4COO-Atp-D-R-Bph-P-Y-P-Bph-Y-F4COO-F4COO-S-C (SEQ ID NO: 1); or
an amino acid sequence resulting from substitution, addition, deletion, or insertion of 1 to 15 amino acid residues selected from the group consisting of amino acid residues at positions 1, 2, 4, 5, 6, 7, 8, 9, 10, 11, 12, 13, 14, 15, and 16 of the above amino acid sequence.

E-R-V-F4COO-Atp-D-R-Bph-P-Y-P-Bph-Y-F4COO-F4COO-S-C (SEQ ID NO: 1) is a peptide that has been confirmed to have BMP binding activity in Examples of the present specification.

One to fifteen amino acid residues selected from the group consisting of amino acid residues at positions 1, 2, 4, 5, 6, 7, 8, 9, 10, 11, 12, 13, 14, 15, and 16 of the amino acid sequence of SEQ ID NO: 1 may have substitution, addition, deletion, or insertion. The number of amino acids that are substituted, deleted, added, and/or inserted only needs to be one or more and 10 or less, and the lower limit thereof is one. The upper limit thereof is 10, 9, 8, 7, 6, 5, 4, 3, or 2, and the minimum is one. The "substitution" of amino acids is preferable. The substitution of amino acids is suitably conservative amino acid substitution.

The term "conservative amino acid substitution" means a substitution with a functionally equivalent or similar amino acid. A conservative amino acid substitution in a peptide does not generally affect the function and/or activity of the peptide. In general, a substitution within a group can be considered structurally and functionally conservative. However, as is obvious to those skilled in the art, the role played by a particular amino acid residue can be determined by the significance in the three-dimensional structure of a molecule containing the amino acid. For example, a cysteine residue can take a less polar, oxidized (disulfide) form when compared to a reduced (thiol) form. A long aliphatic portion of an arginine side chain can constitute a structurally and functionally important feature. In addition, a side chain having an aromatic ring (of tryptophan, tyrosine, or phenylalanine) can contribute to an ion-aromatic or cation-pi interaction. In such cases, substitution of an amino acid having such a side chain with an amino acid belonging to an acidic or nonpolar group can be structurally and functionally conservative. A residue such as proline, glycine, and cysteine (a disulfide form) can have a direct effect on the conformation of the main chain and often cannot be substituted without any structural distortion.

The conservative amino acid substitution includes a specific substitution based on side chain similarity (e.g., the substitution described in L. Lehninger, Biochemistry, 2nd edition, pp. 73-75, Worth Publisher, New York (1975)) and a typical substitution as shown below. In addition, for example, natural amino acids are divided into groups on the basis of common side chain properties as follows, and the conservative amino acid substitution is preferably a substitution with an amino acid belonging to the same group as a group of a certain amino acid.

Hydrophobic (also called nonpolar) amino acids: amino acids that are hydrophobic (nonpolar) and that include alanine ("Ala" or simply "A"), glycine ("Gly" or simply "G"), valine ("Val" or simply "V"), leucine ("Leu" or simply "L"), isoleucine ("Ile" or simply "I"), proline ("Pro" or simply "P"), phenylalanine ("Phe" or simply "F"), tryptophan ("Trp" or simply "W"), tyrosine ("Tyr" or simply "Y"), and methionine ("Met" or simply "M").

Note that the hydrophobic amino acids can be further divided into the following groups.

Aliphatic amino acids: amino acids with fatty acid or hydrogen in the side chain, including Ala, Gly, Val, Ile, and Leu.

Aliphatic/branched-chain amino acids: amino acids with branched fatty acid in the side chain, including Val, Ile, and Leu.

Aromatic amino acids: amino acids with an aromatic ring in the side chain, including Trp, Tyr, and Phe.

Hydrophilic (also called polar) amino acids: amino acids that are hydrophilic (polar) and that include serine ("Ser" or simply "S"), threonine ("Thr" or simply "T"), cysteine ("Cys" or simply "C"), asparagine ("Asn" or simply "N"), glutamine ("Gln" or simply "Q"), aspartic acid ("Asp" or simply "D"), glutamic acid ("Glu" or simply "E"), lysine ("lysine"; "Lys" or simply "K"), arginine ("Arg" or simply "R"), and histidine ("His" or simply "H").

Note that the hydrophilic amino acids can be further divided into the following groups.

Acidic amino acids: amino acids with an acidic side chain, including Asp and Glu.

Basic amino acids: amino acids with a basic side chain, including Lys, Arg, and His.

Neutral amino acids: amino acids with a neutral side chain, including Ser, Thr, Asn, Gln, and Cys.

Meanwhile, Gly and Pro can be classified as "amino acids affecting the direction of the main chain", and amino acids containing a sulfur molecule in the side chain, Cys and Met, can also be classified as "sulfur-containing amino acids".

In the present specification, the "amino acids" include not only natural amino acids but also non-natural amino acids. Examples of the non-natural amino acids include N-alkylamino acids, in which the above-described natural amino acid is N-alkylated, and those in which nitrogen forming a peptide bond is modified with a branched or unbranched lower (e.g., Cl-C5, preferably C1-C3, and more preferably C1) alkyl group. The N-alkylamino acids are preferably N-ethylamino acids, N-butylamino acids, or N-methylamino acids, and more preferably N-methylamino acids. In addition, examples of the non-natural amino acids include D-type amino acids (also called D-amino acids), β-amino acids, γ-amino acids, amino acid mutants, chemically modified amino acids such as amino acid derivatives, and amino acids such as norleucine and ornithine, which are not used as building blocks of proteins in vivo. Further, examples thereof include amino acids in which a functional group is added to the side chain of a natural amino acid or another functional group is substituted in the side chain of a natural amino acid (e.g., amino acids with substitution and/or addition in, for example, a arylene or alkylene group moiety in the side chain, amino acids with an increased number of carbon atoms in an arylene, alkylene, or alkyl group in the side chain, amino acids with a substituted aromatic ring in the side chain, heterocyclized or cyclocondensed amino acids, etc.).

Note that when a structure such as a functional group is added to the side chain of a nature amino acid or is substituted in the side chain of a nature amino acid, a property different from that of a natural amino acid can be imparted. For example, A4p is an amino acid that has a piperidyl group in the side chain of alanine, but shows properties of basic polar amino acids different from alanine that belongs to the group of nonpolar amino acids due to the addition of the piperidyl group. In other words, non-natural amino acids with similar side chain properties can be included in the above-described groups into which natural amino acids are divided based on their common side chain properties. For example, N-methyl arginine (MeR), which is an amino acid obtained by methylating a nitrogen atom of the main chain of arginine that belongs to the basic amino acids, is a non-natural amino acid, but can be classified as the basic amino acids because it shows the basic properties. Thus, non-natural amino acids that show side-chain properties similar to those of a certain amino acid can also be included as targets for conservative amino acid substitution. D-amino acids such as de can be classified as D-amino acids or can be classified according to the nature of their side chains. N-methylamino acids can be classified as N-alkylamino acids or can be classified according to the nature of the side chain of the original amino acid that is not N-methylated.

In the present specification, "without limiting" and "in one embodiment" may be used synonymously.

The "pharmaceutically acceptable salt thereof " means a salt of any peptide. Examples of the pharmaceutically acceptable salt include: salts with mineral acids such as sulfuric acid, hydrochloric acid, and phosphoric acid; salts with organic acids such as acetic acid, oxalic acid, lactic acid, tartaric acid, fumaric acid, maleic acid, methanesulfonic acid, trifluoromethanesulfonic acid, and benzenesulfonic acid; salts with amines such as trimethylamine and methylamine; and salts with metal ions such as sodium ion, potassium ion, and calcium ion. Regarding a compound that contains moisture due to a change over time, such moisture is also included in the pharmaceutically acceptable salt.

The peptide of the present invention includes a peptide belonging to Group A1, a peptide belonging to Group A2, a peptide belonging to Group A3, and a peptide belonging to Group A4 described below.

In one embodiment, the peptide belonging to Group A1 of the present invention is a peptide that includes the following amino acid sequence:
E-R-V-F4COO-Atp-D-R-Bph-P-Y-P-Bph-Y-F4COO-F4COO-S-C (SEQ ID NO: 1), or
an amino acid sequence having 5 or less amino acid substitutions selected from the following (1-i) to (1-xii) in the above amino acid sequence.

(1-i) The first amino acid in the amino acid sequence set forth in SEQ ID NO: 1 is substituted with A.

(1-ii) The second amino acid in the amino acid sequence set forth in SEQ ID NO: 1 is substituted with any polar amino acid.

(1-iii) The fourth amino acid in the amino acid sequence set forth in SEQ ID NO: 1 is substituted with unsubstituted or substituted Y.

(1-iv) The fifth amino acid in the amino acid sequence set forth in SEQ ID NO: 1 is substituted with V.

(1-v) The sixth amino acid in the amino acid sequence set forth in SEQ ID NO: 1 is substituted with KCOpipzaa.

(1-vi) The nineth amino acid in the amino acid sequence set forth in SEQ ID NO: 1 is substituted with S.

(1-vii) The 10th amino acid in the amino acid sequence set forth in SEQ ID NO: 1 is substituted with Hty.

(1-viii) The 11th amino acid in the amino acid sequence set forth in SEQ ID NO: 1 is substituted with any aliphatic amino acid.

(1-ix) The 12th amino acid in the amino acid sequence set forth in SEQ ID NO: 1 is substituted with substituted Bph.

(1-x) The 13th amino acid in the amino acid sequence set forth in SEQ ID NO: 1 is substituted with F4F.

(1-xi) The 15th amino acid in the amino acid sequence set forth in SEQ ID NO: 1 is substituted with 4Py.

(1-xii) The 16th amino acid in the amino acid sequence set forth in SEQ ID NO: 1 is substituted with any amino acid other than S.

Although not limited, any polar amino acid in the above (1-ii) may be Hcit or KCOpipzaa, the unsubstituted or substituted Y in the above (1-iii) may be unsubstituted Y, any aliphatic amino acid in the above (1-viii) may be V, and any amino acid other than S in the above (1-xii) may be H.

In addition, although not limited, the peptide belonging to Group A1 of the present invention may be a peptide that includes an amino acid sequence having 4 or less, 3 or less, 2 or less, or 1 amino acid substitutions selected from the above (1-i) to (1-xii) in the amino acid sequence set forth in SEQ ID NO: 1.

In addition, in one embodiment, the peptide belonging to Group A2 of the present invention is a peptide that includes the following amino acid sequence:
E-R-V-Y-V-D-R-3Py6Ph-S-Y-V-Bph-Y-F4COO-F4COO-S-C (SEQ ID NO: 2), or
an amino acid sequence having 5 or less amino acid substitutions selected from the following (2-i) to (2-xi) in the above amino acid sequence.

(2-i) The first amino acid in the amino acid sequence set forth in SEQ ID NO: 2 is substituted with A.

(2-ii) The second amino acid in the amino acid sequence set forth in SEQ ID NO: 2 is substituted with KCOpipzaa.

(2-iii) The fourth amino acid in the amino acid sequence set forth in SEQ ID NO: 2 is substituted with F4F or F4COO.

(2-iv) The sixth amino acid in the amino acid sequence set forth in SEQ ID NO: 2 is substituted with KCOpipzaa.

(2-v) The seventh amino acid in the amino acid sequence set forth in SEQ ID NO: 2 is substituted with K.

(2-vi) The eighth amino acid in the amino acid sequence set forth in SEQ ID NO: 2 is substituted with Bph.

(2-vii) The ninth amino acid in the amino acid sequence set forth in SEQ ID NO: 2 is substituted with P or KCOpipzaa.

(2-viii) The 11th amino acid in the amino acid sequence set forth in SEQ ID NO: 2 is substituted with any aliphatic amino acid.

(2-ix) The 12th amino acid in the amino acid sequence set forth in SEQ ID NO: 2 is substituted with substituted Bph.

(2-x) The 13th amino acid in the amino acid sequence set forth in SEQ ID NO: 2 is substituted with substituted F.

(2-xi) The 15th amino acid in the amino acid sequence set forth in SEQ ID NO: 2 is substituted with 4Py.

Although not limited, any aliphatic amino acid in the above (2-viii) may be P, Hyp, P4Sh, Pc4F, Pt4F, P4F2, or aMePV, and the substituted F in the above (2-x) may be F4F.

In addition, although not limited, the peptide belonging to Group A2 of the present invention may be a peptide that includes an amino acid sequence having 4 or less, 3 or less, 2 or less, or 1 amino acid substitutions selected from the above (2-i) to (2-xi) in the amino acid sequence set forth in SEQ ID NO: 2.

In addition, in one embodiment, the peptide belonging to Group A3 of the present invention is a peptide that includes the following amino acid sequence:
A-R-V-Y-V-D-R-Bph-P-Y-A-Bph-Y-F4COO-F4COO-S-C (SEQ ID NO: 3), or
an amino acid sequence having 5 or less amino acid substitutions selected from the following (3-i) to (3-x) in the above amino acid sequence.

(3-i) The second amino acid in the amino acid sequence set forth in SEQ ID NO: 3 is substituted with K, KAc, Q, or KCOpipzaa.

(3-ii) The fourth amino acid in the amino acid sequence set forth in SEQ ID NO: 3 is substituted with F4F or 4Py.

(3-iii) The sixth amino acid in the amino acid sequence set forth in SEQ ID NO: 3 is substituted with KCOpipzaa, K, R, G, or H.

(3-iv) The seventh amino acid in the amino acid sequence set forth in SEQ ID NO: 3 is substituted with K, KAc, 4Py, S, or Cit.

(3-v) The eighth amino acid in the amino acid sequence set forth in SEQ ID NO: 3 is substituted with 3Py6Ph.

(3-vi) The nineth amino acid in the amino acid sequence set forth in SEQ ID NO: 3 is substituted with S, K, H, G, S, or KCOpipzaa.

(3-vii) The 11th amino acid in the amino acid sequence set forth in SEQ ID NO: 3 is substituted with any aliphatic amino acid.

(3-viii) The 12th amino acid in the amino acid sequence set forth in SEQ ID NO: 3 is substituted with 3Py6Ph.

(3-ix) The 13th amino acid in the amino acid sequence set forth in SEQ ID NO: 3 is substituted with F4F.

(3-x) The 15th amino acid in the amino acid sequence set forth in SEQ ID NO: 3 is substituted with 4Py.

Although not limited, any aliphatic amino acid in the above (3-vii) may be V, P, or A.

In addition, although not limited, the peptide belonging to Group A3 of the present invention may be a peptide that includes an amino acid sequence having 4 or less, 3 or less, 2 or less, or 1 amino acid substitutions selected from the above (3-i) to (3-x) in the amino acid sequence set forth in SEQ ID NO: 3.

In addition, in one embodiment, the peptide belonging to Group A4 of the present invention is a peptide that includes the following amino acid sequence:
F-G-MeF-G-Bph-G-D-D-Cha-A-MeF-L-H-C (SEQ ID NO: 241), or
an amino acid sequence having 4 or less amino acid substitutions selected from the following (4-i) to (4-xii) in the above amino acid sequence.

(4-i) The first amino acid in the amino acid sequence set forth in SEQ ID NO: 241 is substituted with substituted phenylalanine.

(4-ii) The third amino acid in the amino acid sequence set forth in SEQ ID NO: 241 is substituted with substituted N-methylphenylalanine.

(4-iii) The fourth amino acid in the amino acid sequence set forth in SEQ ID NO: 241 is substituted with da or ds.

(4-iv) The fifth amino acid in the amino acid sequence set forth in SEQ ID NO: 241 is substituted with biphenylalanine obtained by substituting C on the aromatic ring with N.

(4-v) The sixth amino acid in the amino acid sequence set forth in SEQ ID NO: 241 is substituted with A or da.

(4-vi) The seventh amino acid in the amino acid sequence set forth in SEQ ID NO: 241 is substituted with N.

(4-vii) The eighth amino acid in the amino acid sequence set forth in SEQ ID NO: 241 is substituted with E.

(4-viii) The nineth amino acid in the amino acid sequence set forth in SEQ ID NO: 241 is substituted with Acpe.

(4-ix) The tenth amino acid in the amino acid sequence set forth in SEQ ID NO: 241 is substituted with any polar amino acid.

(4-x) The 11th amino acid in the amino acid sequence set forth in SEQ ID NO: 241 is substituted with substituted N-methylphenylalanine.

(4-xi) The 12th amino acid in the amino acid sequence set forth in SEQ ID NO: 241 is substituted with any aliphatic amino acid.

(4-xii) The 13th amino acid in the amino acid sequence set forth in SEQ ID NO: 241 is substituted with any polar amino acid.

Although not limited, any polar amino acid in the above (4-ix) may be Hgl or Q, the number of carbon atoms of the side chain of any aliphatic amino acid in the above (4-xi) may be 7 or less, and any polar amino acid in the above (4-xii) may be P4Sh or F4COO.

In addition, although not limited, the peptide belonging to Group A4 of the present invention may be a peptide that includes an amino acid sequence having 3 or less, 2 or less or 1 amino acid substitutions selected from the above (4-i) to (4-xii) in the amino acid sequence set forth in SEQ ID NO: 241.

In one embodiment, the peptide is a cyclic peptide. The "cyclic peptide" will be described in detail in "3. Peptide (B)".

The peptide may include an additional amino acid residue in addition to the amino acid sequences of SEQ ID NOs: 5 to 187, SEQ ID NOs: 188 to 240, SEQ ID NOs: 243 to 321, and SEQ ID NOs: 322 to 421. The "additional amino acid residue" will be described in detail in "3. Peptide (B)".

The peptides belonging to Groups A1 to A3 of the present invention bind to BMP4. The peptide belonging to Group A1 of the present invention preferably further binds to BMP7. In addition, the peptide belonging to Group A2 of the present invention preferably does not bind to BMP7.

The peptides belonging to Groups A1 to A3 of the present invention have BMP4 inhibitory activity. The peptide more preferably has activity of regulating differentiation induction of human stem cells. The "binding to BMP4", "having BMP4 inhibitory activity", and "having activity of regulating differentiation induction of human stem cells" will be described in detail in "3. Peptide (B)".

The peptide belonging to Group A4 has BMP7 inhibitory activity. The peptide more preferably has activity of regulating differentiation induction of human stem cells. The "having BMP7 inhibitory activity" and "having activity of regulating differentiation induction of human stem cells" will be described in detail in "3. Peptide (B)".

### 3. Peptide (B)

One embodiment different from the above one embodiment of the present invention relates to the following peptide (B).

The peptide of the present invention in this embodiment includes the following amino acid sequence:
X1-X2-X3-X4-X5-X6-X7-X8-X9-X10-X11-X12-X13-X14-X15-X16-C (SEQ ID NO: 4),
wherein X1 is E, A, T, or Q,
X2 is R, K, Hgl, any polar amino acid, or any aliphatic amino acid,
X3 is V,
X4 is an amino acid having an aromatic ring that may be a heterocycle at a side chain,
X5 is any aliphatic amino acid, Y, D, or Atp,
X6 is D, any basic amino acid, or any polar amino acid,
X7 is any basic amino acid or any polar amino acid,
X8 is biphenylalanine that may have a substituent and in which C on an aromatic ring may be substituted with N,
X9 is any amino acid,
X10 is Y, Hty, or 4Py,
X11 is any amino acid,
X12 is biphenylalanine that may have a substituent and in which C on an aromatic ring may be substituted with N,
X13 is unsubstituted or substituted F, 4Py, or 3Py6NH2,
X14 is F4COO, D, or G,
X15 is F4COO, Y, 4Py, 3Py6NH2, W7N, Hph, or N, and
X16 is any amino acid.

The above options of X1 to X16 can be selected in any combination.

Note that the substituted F includes, for example, an amino acid having a substituent on a benzene ring having phenylalanine at the side chain, an amino acid derivative of F having a heterocyclic ring, or an amino acid derivative of F having a condensed polycyclic structure. Thus, the substituted F includes Y.

In one embodiment, X2 is R, K, Hgl, Hcit, KCOpipzaa, or Tic.

In one embodiment, X5 is V, Y, D, or Atp.

In one embodiment, X6 is D, R, H, K, KCOpipzaa, V, Y, D, or Atp.

In one embodiment, X7 is R, K, or any polar amino acid.

In one embodiment, X8 is unsubstituted Bph or unsubstituted 3Py6Ph.

In one embodiment, X12 is unsubstituted Bph or unsubstituted 3Py6Ph.

In one embodiment, X13 is unsubstituted or substituted F.

In one embodiment, X16 is S or H.

The peptide of the present invention includes a peptide belonging to Group B1, a peptide belonging to Group B2, a peptide belonging to Group B3, and a peptide belonging to Group B4, described below.

Although not limited, the peptide belonging to Group B1 of the present invention includes the following amino acid sequence:
X1-X2-X3-X4-X5-X6-X7-X8-X9-X10-X11-X12-X13-X14-X15-X16-C (SEQ ID NO: 4),
wherein X1 is E,
X2 is R or any polar amino acid,
X3 is V,
X4 is unsubstituted or substituted Y or F4COO,
X5 is V or Atp,
X6 is D or KCOpipzaa,
X7 is R,
X8 is Bph,
X9 is P or S,
X10 is Y or Hty,
X11 is any amino acid,
X12 is unsubstituted or substituted Bph,
X13 is unsubstituted or substituted F,
X14 is F4COO,
X15 is F4COO or 4Py, and
X16 is any amino acid.

The above options of X1 to X16 can be selected in any combination.

In one embodiment, X2 is R, Hcit, or KCOpipzaa.

In one embodiment, X4 is Y or F4COO.

In one embodiment, X11 is any aliphatic amino acid.

In one embodiment, X12 is Bph.

In one embodiment, X13 is Y or F4F.

In one embodiment, X15 is F4COO.

In one embodiment, X16 is S or H.

In one embodiment, X2 is KCOpipzaa, and X15 is 4Py.

Although not limited, the peptide belonging to Group B2 of the present invention includes the following amino acid sequence:
X1-X2-X3-X4-X5-X6-X7-X8-X9-X10-X11-X12-X13-X14-X15-X16-C (SEQ ID NO: 4),
wherein X1 is E or A,
X2 is R or KCOpipzaa,
X3 is V,
X4 is Y, F4F, or F4COO,
X5 is V,
X6 is D or KCOpipzaa,
X7 is R or K,
X8 is 3Py6Ph or Bph,
X9 is S, P, or KCOpipzaa,
X10 is Y,
X11 is any aliphatic amino acid,
X12 is Bph,
X13 is unsubstituted or substituted F,
X14 is F4COO,
X15 is F4COO or 4Py, and
X16 is S.

The above options of X1 to X16 can be selected in any combination.

In one embodiment, X11 is V, P, Hyp, P4Sh, Pc4F, Pt4F, P4F2, or aMeP.

In one embodiment, X12 is Bph.

In one embodiment, X13 is Y or F4F.

In one embodiment, X15 is F4COO.

In addition, although not limited, the peptide belonging to Group B3 of the present invention includes the following amino acid sequence:
X1-X2-X3-X4-X5-X6-X7-X8-X9-X10-X11-X12-X13-X14-X15-X16-C (SEQ ID NO: 4),
wherein X1 is A,
X2 is R, KAc, Q, or KCOpipzaa,
X3 is V,
X4 is Y, F4F, or 4Py,
X5 is V,
X6 is D, KCOpipzaa, K, R, G, or H,
X7 is R, K, KAc, 4Py, S, or Cit,
X8 is 3Py6Ph or Bph,
X9 is P, S, K, H, G, S, or KCOpipzaa,
X10 is Y,
X11 is any aliphatic amino acid,
X12 is Bph or 3Py6Ph,
X13 is Y or F4F,
X14 is F4COO,
X15 is F4COO or 4Py, and
X16 is S.

The above options of X1 to X16 can be selected in any combination.

In one embodiment, X11 is V, P, or A.

In addition, although not limited, the peptide belonging to Group B4 of the present invention includes the following amino acid sequence:
X1-X2-X3-X4-X5-X6-X7-X8-X9-X10-X11-X12-X13-X14 (SEQ ID NO: 242),
wherein X1 is phenylalanine that may have a substituent and in which C on an aromatic ring may be substituted with N,
X2 is G,
X3 is N-methylphenylalanine that may have a substituent,
X4 is G, an aliphatic amino acid or a polar amino acid that may be a D form,
X5 is biphenylalanine in which C on an aromatic ring may be substituted with N or phenylalanine that may have a substituent,
X6 is G, or A that may be a D form,
X7 is any polar amino acid,
X8 is any acidic amino acid,
X9 is any aliphatic amino acid,
X10 is any amino acid,
X11 is N-methylphenylalanine that may have a substituent,
X12 is any aliphatic amino acid,
X13 is any amino acid, and
X14 is C.

In one embodiment, the peptide is a cyclic peptide. The "cyclic peptide" refers to a peptide in which two amino acids are bound to form a ring in whole or in part. The peptide also includes peptides in which the amino acids form a cross-linked structure, peptides in which a cyclic structure is formed by lactam ring formation or macrocyclization reaction, and peptides having a lasso peptide-like structure. In other words, the cyclic peptide only needs to form a cyclic structure in part, and may have a linear chain portion.

There are problems that peptides generally have poor metabolic stability in vivo and their large size makes it difficult for them to permeate a cell membrane. To address such problems, a peptide cyclization technique has been adopted. It has been suggested that the peptide cyclization increases their protease resistance and thus increases their metabolic stability, and restricts conformational changes, thereby increasing their rigidity and improving their membrane permeability and affinity for a target protein.

In one embodiment, the peptide has a cyclic structure in which a chloroacetylated amino acid is bound to a cysteine residue included in the peptide. In one embodiment, the peptide has a cyclic structure in which an N-terminal amino acid (the amino acid residue at position 1) is bound to a cysteine residue included in the peptide. In one embodiment, the peptide has a cyclic structure in which an N-terminal amino acid (the amino acid residue at position 1) is bound to a cysteine residue at position 17 included in the peptide. In one embodiment, the peptide has a cyclic structure in which a chloroacetylated N-terminal amino acid (the amino acid residue at position 1) is bound to a cysteine residue at position 17 included in the peptide. The "chloroacetylation" may be "halogen acetylation" with another halogen. Further, the "acetylation" may be "acylation" with an acyl group other than an acetyl group.

In one embodiment, the peptide includes or consists of an amino acid sequence set forth in any one of SEQ ID NOs: 5 to 187, SEQ ID NOs: 188 to 240, SEQ ID NOs: 243 to 321, and SEQ ID NOs: 322 to 421. In one embodiment, the peptide is a cyclic peptide that includes or consists of an amino acid sequence set forth in any one of SEQ ID NOs: 5 to 187, SEQ ID NOs: 188 to 240, SEQ ID NOs: 243 to 321, and SEQ ID NOs: 322 to 421. The peptide is a peptide that consists of an amino acid sequence set forth in any one of SEQ ID NOs: 5 to 187, SEQ ID NOs: 188 to 240, SEQ ID NOs: 243 to 321, and SEQ ID NOs: 322 to 421, or is a peptide that consists of an amino acid sequence resulting from substitution, deletion, insertion, or addition of 1 to 10 (preferably 1 to 4,1 to 3, 2, or 1) amino acid residues therefrom. Note that, with respect to the sequence to which amino acid residues have been added, if the added portion is a linker, a sequence of many amino acid residues (for example, 1 to 10 residues) may be further added. In addition, a peptide that consists of an amino acid sequence in which these amino acid residues are substituted, deleted, inserted, or added has for example binding ability to BMP4 or preferably has BMP4 and/or BMP7 inhibitory activity.

The peptide may include an additional amino acid residue, in addition to amino acid sequences set forth in SEQ ID NO: 4 and SEQ ID NO: 242. The additional amino acid residue may be included in the peptide that forms a cyclic structure, or the amino acid residue may be further added in a linker-like manner from the cyclic peptide. The number of amide bonds (the number/length of amino acids) at the peptide or the peptide portion is not particularly limited.

In addition, an additional linker may be added from the cyclic peptide. Examples of the linker include the above-described amino acid linker (a peptide linker), a chemical linker, a fatty acid linker, a nucleic acid linker, and a sugar chain linker. The linker may be a complex of, for example, a chemical linker and a peptide linker. The chemical linker may be a PEG linker that consists of 1-24 ethylene glycol units. The linker may be a fatty acid linker containing a divalent chemical moiety induced from a fatty acid. The amino acid (peptide) linker may be a linker containing at least one amino acid. It is possible to use, for example, a glycine-rich peptide such as a peptide having the sequence [Gly-Gly-Gly-Gly-Ser]n (wherein n is 1, 2, 3, 4, 5, or 6) as described in US Patent No. 7,271,149 or a serine-rich peptide linker described in US Patent No. 5,525,491. Note that, regarding the peptide linker, an amino acid and an amino acid, or an amino acid and a chemical linker may be bound via a side chain of the amino acid. The addition of a linker may change the physical properties (e.g., solubility) of the peptide without limiting.

The linker may be added at any position. For example, it may be linked to Cys located on the C-terminal side, or may be linked to an amino acid contained in a cyclic peptide. Preferably, it is linked to Cys located on the C-terminal side, or is linked to the side chain of an amino acid contained in a cyclic peptide.

Moreover, the peptide may form a multimer via a linker or the like.

The peptides belonging to Groups B1 to B3 have binding ability to BMP4. The peptide belonging to Group B1 of the present invention preferably further has binding ability to BMP7. In addition, the peptide belonging to Group B2 of the present invention preferably does not has binding ability to BMP7.

The peptide belonging to Group B4 has BMP7 inhibitory activity. Preferably, it does not have BMP4 inhibitory activity.

### Regarding binding to BMP

The binding of the peptide and a BMP protein can be measured by any known method for measuring intermolecular binding. It can be determined in any suitable manner known per se, which includes, for example, a surface plasmon resonance (SPR) assay, a Scatchard analysis, and/or competitive binding assays such as a radioimmunoassay (RIA), an enzyme immunoassay (EIA), and a sandwich competitive assay, as well as different variants thereof known per se in the art without limiting. Although not limited, as a result of measuring the peptide and the BMP protein by such any known method for measuring the intermolecular binding (for example, the method shown in Example 15), when the peptide has a KD value of 100 nM or less, the peptide may have binding ability to the BMP protein.

Note that a novel peptide is provided in this specification. The peptide having inhibitory activity of BMP is preferably a peptide having binding activity of BMP. This is suggested by the fact that the peptide having inhibitory activity of BMP4 and/or BMP7 shown in Examples 11 and 12 has binding activity to BMP as shown in the results of Example 15 (SPR measurement). The peptide having inhibitory activity of BMP is effective in treatment or prophylaxis of various diseases involved by BMP. The peptide having binding activity of BMP is effective in various experiments on various diseases involved by BMP. The bone morphogenetic protein 4 (BMP4) mainly acts on the formation and development of bone and cartilage, and is also related to various bone diseases (Japanese Patent No. 6516724). BMP4 is an active substance of a BMP signal transduction pathway (Japanese Patent No. 7120585). The bone morphogenetic protein 7 (BMP-7) is an active substance of a BMP signal transduction pathway (Japanese Patent No. 7120585).

### Regarding having BMP4 inhibitory activity

The BMP4 inhibitory activity can be evaluated by phosphorylation of SMAD1 by a BMP4 stimulus. As a specific method, the evaluation may be performed based on, for example, the method described in Example 11. As a BMP4 activity inhibitor, Noggin is known. The peptide having BMP4 inhibitory activity preferably has the same level of BMP4 inhibitory activity as known BMP4 activity inhibitors.

In the present specification, the BMP4 inhibitory activity and the BMP4 signal transduction inhibitory activity are synonymous.

### Regarding having BMP7 inhibitory activity

The BMP7 inhibitory activity can be evaluated by phosphorylation of SMAD1 by a BMP7 stimulus. As a specific method, the evaluation may be performed based on, for example, the method described in Example 12. As a BMP7 activity inhibitor, Noggin is known. The peptide having BMP7 inhibitory activity preferably has the same level of BMP7 inhibitory activity as known BMP7 activity inhibitors.

In the present specification, the BMP7 inhibitory activity and the BMP7 signal transduction inhibitory activity are synonymous.

### Regarding having activity of regulating differentiation induction of human stem cells

As described above, having the activity of regulating differentiation induction of human stem cells (inhibitory activity) can be evaluated by evaluating having the BMP4 and/or BMP7 inhibitory activity.

Unless otherwise stated, the "peptide" in the present specification includes both "2. Peptide (A)" and "3. Peptide (B)". Moreover, in the present specification, the peptide also includes a pharmaceutically acceptable salt thereof.

### 4. Production of Peptide

The peptide of the present invention can be produced by any known method for producing the peptide as described below, for example.

A chemical synthesis method such as a liquid-phase method, a solid-phase method, and a hybrid method in which liquid-phase and solid-phase methods are combined; and
a genetic recombinant method.

In the solid-phase method, for example, a hydroxyl group of a hydroxylated resin is esterified with a carboxy group of a first amino acid, an α-amino group of which is protected by a protecting group (usually the C-terminal amino acid of the peptide as a target). The esterification catalyst used can be a known dehydration-condensation agent such as 1-mesitylenesulfonyl-3-nitro-1,2,4-triazole (MSNT), dicyclohexylcarbodiimide (DCC), and diisopropylcarbodiimide (DIC).

Next, the protecting group of the α-amino group of the first amino acid is eliminated, a second amino acid in which all functional groups other than the carboxy group of the main chain are protected is added, and the carboxyl group is activated to bind the first and second amino acids. Further, the α-amino group of the second amino acid is deprotected, a third amino acid in which all functional groups other than the carboxy group of the main chain are protected is added, and the carboxyl group is activated to bind the second and third amino acids. This procedure is repeated until a peptide having a target length is synthesized, and all the functional groups are then deprotected.

Examples of the resin for solid-phase synthesis include Merrifield resin, MBHA resin, Cl-Trt resin, SASRIN resin, Wang resin, Rink amide resin, HMFS resin, Amino-PEGA resin (Merck), and HMPA-PEGA resin (Merck). These resins can be washed with a solvent (e.g., dimethylformamide (DMF), 2-propanol, methylene chloride) before use.

Examples of the protecting group for the α-amino group include a benzyloxycarbonyl (Cbz or Z) group, a tert-butoxycarbonyl (Boc) group, a 9-fluorenylmethyloxycarbonyl (Fmoc) group, a benzyl group, an allyl group, and an allyloxycarbonyl (Alloc) group. The Cbz group can be deprotected, for example, with hydrofluoric acid or by hydrogenation, the Boc group can be deprotected with trifluoroacetic acid (TFA), and the Fmoc group can be deprotected by treatment with piperidine or pyrrolidine.

The α-carboxy group can be protected using, for example, methyl ester, ethyl ester, allyl ester, benzyl ester, tert-butyl ester, or cyclohexyl ester.

The carboxy group can be activated using a condensation agent. Examples of the condensation agent include dicyclohexylcarbodiimide (DCC), diisopropylcarbodiimide (DIC), 1-ethyl-3-(3-dimethylaminopropyl)carbodiimide (EDC or WSC), (1H-benzotriazol-1-yloxy)tris(dimethylamino)phosphonium hexafluorophosphate (BOP), and 1-[bis(dimethylamino)methyl]-1H-benzotriazolium-3-oxide hexafluorophosphate (HBTU).

The peptide chain can be cleaved from the resin by treatment with an acid such as TFA and hydrogen fluoride (HF).

The peptide can be produced by genetic recombinant method (translation synthesis system) using a nucleic acid encoding the peptide described above. The nucleic acid encoding the peptide may be DNA or RNA.

The nucleic acid encoding the peptide can be prepared by a known method or an equivalent method. For example, the nucleic acid can be synthesized with an automatic synthesizer. A restriction enzyme recognition site may be added for vector insertion of the obtained DNA. Alternatively, a base sequence encoding an amino acid sequence may be incorporated so that the resulting peptide chain can be cut out by an enzyme or other means.

To inhibit degradation by host-derived proteases, a chimeric protein expression method, in which a peptide as a target is expressed as a chimeric peptide with another peptide, can also be used. In this case, as the above nucleic acid, a nucleic acid encoding the peptide as a target and a peptide bound to the peptide as a target is used.

Then, an expression vector is prepared using the nucleic acid encoding the peptides. The nucleic acid can be inserted downstream of a promoter of the expression vector as it is, by digestion with a restriction enzyme, or by adding a linker. Examples of the vector include: an E. coli-derived plasmid (e.g., pBR322, pBR325, pUC12, pUC13, pUC18, pUC19, pUC118, pBluescript II); a Bacillus subtilis-derived plasmid (e.g., pUBI10, pTP5, pC1912, pTP4, pE194, pC194); a yeast-derived plasmid (e.g., pSH19, pSH15, YEp, YRp, YIp, YAC); a bacteriophage (e.g., e-phage, M13 phage); a virus (e.g., retrovirus, vaccinia virus, adenovirus, adeno-associated virus (AAV), cauliflower mosaic virus, tobacco mosaic virus, baculovirus); and a cosmid.

The promoter can be appropriately selected depending on the type of host. If the host is an animal cell, for example, an SV40 (simian virus 40)-derived promoter or a CMV (cytomegalovirus)-derived promoter can be used. If the host is E. coli, for example, a trp promoter, a T7 promoter, or a lac promoter can be used.

The expression vector can incorporate, for example, a nucleic acid encoding a DNA replication origin (ori), a selection marker (e.g., antibiotic resistance, nutrient requirement), an enhancer, a splicing signal, a poly-A addition signal, or a tag (e.g., FLAG, HA, GST, GFP).

Next, an appropriate host cell is transformed with the expression vector. The host can be appropriately selected in view of the relation to the vector. Examples of the host used include E. coli, Bacillus subtilis, a bacterium belonging to the genus Bacillus, yeast, an insect or insect cell, and an animal cell. Examples of the animal cell used include an HEK293T cell, a CHO cell, a COS cell, a myeloma cell, a HeLa cell, and a Vero cell. The transformation can be performed according to the type of host by a known method such as lipofection, a calcium phosphate method, electroporation, microinjection, and particle gun. The transformants may be cultured according to a routine procedure to express a peptide as a target.

The peptide is purified from the transformant culture by collecting the cultured cells, suspending the cells in an appropriate buffer solution, and then destroying the cells by, for instance, sonication or freeze-thawing to obtain a crude extract by centrifugation or filtration. If the peptide is secreted into the culture solution, the supernatant is collected.

Purification from the crude extract or culture supernatant can also be performed by a known method or an equivalent method (e.g., chlorination, dialysis, ultrafiltration, gel filtration, SDS-PAGE, ion exchange chromatography, affinity chromatography, reverse phase high-performance liquid chromatography).

A known method or an equivalent method may be used to convert the resulting peptide in a free form to a salt or in a salt form to a free form.

In one embodiment, the translation synthesis system may be a cell-free translation system. The cell-free translation system generally allows an expressed product to be obtained in a highly pure form without purification. The cell-free translation system contains, for example, ribosomal proteins, aminoacyl-tRNA synthetase (ARS), ribosomal RNA, amino acids, rRNA, GTP, ATP, translation initiation factor (IF), elongation factor (EF), termination factor (RF), and ribosome regeneration factor (RRF), as well as other factors necessary for translation. An E. coli extract or wheat germ extract may be added to increase expression efficiency. In addition, a rabbit red blood cell extract or an insect cell extract may be added.

Energy can be continuously supplied to the system containing them by using dialysis to produce several hundred µg to several mg/mL of the protein without limiting. The system may be a system including RNA polymerase for transcription from the gene DNA. As a commercially available cell-free translation system that can be used, examples of the E. coli-derived system include Roche Diagnostics' RTS-100 (registered trademark), Gene Frontier's PURESYSTEM, and NEW ENGLAND Biolabs' PURExpress In Vitro Protein Synthesis Kit; and examples of the system using wheat germ extract include those from ZOEGENE Corporation and CellFree Sciences.

In the cellular translation system, instead of aminoacyl-tRNA synthesized by natural aminoacyl-tRNA synthetase, an artificial aminoacyl-tRNA in which the desired amino acid or hydroxy acid is linked (acylated) to the tRNA may be used. Such aminoacyl-tRNA can be synthesized using an artificial ribozyme.

Examples of such a ribozyme include flexizyme (H. Murakami, H. Saito, and H. Suga, (2003), Chemistry& Biology, Vol. 10, 655-662; WO2007/066627 and others). The flexizyme is also known and called as the original Flexizyme (Fx) and a modified form such as dinitrobenzylflexizyme (dFx), enhanced Flexizyme (eFx), and aminoflexizyme (aFx).

By using the tRNA, generated by flexizyme, to which the desired amino acid or hydroxy acid is linked, the desired codon can be associated with the desired amino acid or hydroxy acid for translation. A special amino acid may be used as the desired amino acid. For example, the non-natural amino acid required for the cyclization described above can also be introduced into the peptide linked by this method.

The peptide can be chemically synthesized using various methods routinely used in the art. Examples of the method include stepwise solid-phase synthesis, semi-synthesis of peptide fragment via conformationally assisted re-ligation, and chemical ligation. The synthesis of the peptide is a chemical synthesis using various solid-phase techniques as described, for example, in K. J. Jensen, P. T. Shelton, S. L. Pedersen, Peptide Synthesis and Applications, 2nd Edition, Springer, 2013. The preferred strategy is based on a combination of a Fmoc group that temporarily protects the α-amino group and allows selective removal by a base, and a protecting group that temporarily protects the side chain functional group and is stable under de-Fmoc conditions. The selection of such a general peptide side chain is described in, for example, the above Peptide Synthesis and Applications, 2nd edition and G. B. Fields, R. L. Noble, Solid Phase Peptide Synthesis Utilizing 9-Fluorenylmethoxycarbonyl Amino Acids, Int. J. Peptide Protein Res. 35, 1990, 161-214. Examples of the preferred peptide side chain protecting group include: a benzyl, tert-butyl, and trityl (Trt) groups for the hydroxy group of serine or threonine; a 2-bromobenzyloxycarbonyl and tert-butyl groups for the hydroxy group of tyrosine; a Boc, methyltetrazole thiol (Mtt), Alloc, and ivDde groups for the amino group of the lysine side chain; a Trt and Boc groups for the imidazole group of histidine; a 2,2,4,6,7-pentamethyldihydrobenzofuran-5-sulfonyl (Pbf) group for the guanidyl group of arginine; a tert-butyl, allyl, and 3-methylpentane (Mpe) groups for the carboxyl group of, for instance, glutamic acid or aspartic acid; a Trt group for the carboxamide group of glutamine or asparagine; and a Trt and monomethoxytrityl (Mmt) groups for the thiol group of cysteine.

The peptide can be synthesized in a stepwise manner on the solid-phase resin described above. The α-amino protecting groups of the C-terminal amino acid used and all amino acids and the peptide used in the synthesis should be selectively removed in the synthetic process. Preferably, the above-described solid-phase resin is used to initiate the process. The C-terminal carboxyl group of the peptide with the N-terminus appropriately protected with Fmoc, etc. or the C-terminal carboxyl group of a Fmoc-protected amino acid is made into an activated ester with an appropriate reagent and then added to the amino group on the solid-phase resin. Subsequent elongation of the peptide chain can be achieved by sequentially repeating the removal of the N-terminal protecting group (Fmoc group), followed by condensation with a protected amino acid derivative, according to the amino acid sequence of the peptide as a target. Note that the peptide as a target can be released at the final stage. For example, as the release conditions, TFA solution containing water/silylhydride/thiol as a scavenger in TFA, as described in, for instance, Teixeira, W. E. Benckhuijsen, P. E. de Koning, A. R. P. M. Valentijn, J. W. Drijfhout, Protein Pept. Lett., 2002, 9, 379-385, can be used for the release. A typical example is TFA/Water/TIS/DODT (volume ratio 92.5:2.5:2.5:2.5).

The peptide described in the present specification can be synthesized using a single or multichannel peptide synthesizer, such as CEM's Liberty Blue synthesizer, Biotage's Syro I synthesizer, or their successors.

The carboxy group can be activated using a condensation agent. Examples of the condensation agent include dicyclohexylcarbodiimide (DCC), diisopropylcarbodiimide (DIPCDI), 1-ethyl-3-(3-dimethylaminopropyl)carbodiimide (EDC or WSC), (1H-benzotriazol-1-yloxy)tris(dimethylamino)phosphonium hexafluorophosphate (BOP), and 1-[bis(dimethylamino)methyl]-1H-benzotriazolium-3-oxide hexafluorophosphate (HBTU).

The peptide can be cyclized according to a known method. Without limiting, for example, the peptide is designed to contain two or more cysteine residues so that a cyclic structure can be formed through a disulfide bond after translation. In addition, Goto's method (Y. Goto, et al., ACS Chem. Biol. 3 120-129 (2008)) can be used to synthesize a peptide with a chloroacetyl group at the N-terminus by genetic code reprogramming technique. In the peptide, a cysteine residue containing a sulfur molecule can be placed for cyclization. This causes the spontaneous nucleophilic attack of the mercapto group on the chloroacetyl group after translation, and the peptide is cyclized through a thioether bond. Other combinations of amino acids that are bound to form a ring may be placed for cyclization within the peptide by genetic code reprogramming technique. Alternatively, the peptide may be cyclized by placing an L-2-amino adipic acid residue in the peptide and binding it to the N-terminal main chain amino group. In this way, any known cyclization method can be used without restriction.

### 5. BMP4 and/or BMP7 signal transduction inhibitor, differentiation induction regulator of human stem cells, culture medium supplement for culture, and culture medium for culture, which include peptide of present invention

The peptide of the present invention has a binding ability to BMP4. The BMP4 is known to have the function of strongly binding to its receptor, BMPR1A, transmitting signals into cells, and regulating cell differentiation and induction. The peptide of the present invention inhibits binding between BMP4 and the BMP4 receptor by binding to BMP4, and therefore has an activity of inhibiting transduction of the BMP4 signal into cells.

The peptide of the present invention has a BMP7 signal transduction inhibitory activity.

Therefore, in one embodiment, the present invention provides BMP4 and/or BMP7 signal transduction inhibitors that include the peptide.

In one embodiment, the present invention provides a composition for inhibiting BMP4 and/or BMP7 signal transduction, which includes the peptide.

In one embodiment, the present invention provides a method for inhibiting the signal transduction activity of BMP4, the method including a step of contacting the peptide with BMP4. Without being limited thereto, the step of contacting the peptide with BMP4 may be a step of contacting *in vitro.*

In one embodiment, the present invention provides a method for inhibiting the signal transduction activity of BMP7.

Because BMP4 and/or BMP7 regulate differentiation/induction of human stem cells through the signal transduction, the peptide having a BMP4 and/or BMP7 signal transduction inhibitory activity has an activity of regulating differentiation induction of human stem cells.

Therefore, in one embodiment, the present invention provides a differentiation induction regulator of human stem cells that includes the peptide.

In one embodiment, the present invention provides a composition that has an ability to regulate differentiation induction of human stem cells that includes the peptide.

In one embodiment, the present invention provides a method for regulating differentiation induction of human stem cells using the peptide. Without being limited thereto, the method can be performed by adding the peptide to a culture medium for culturing human stem cells. The method may be preferably a method that is performed *in vitro.*

Here, the human stem cells include pluripotent stem cells such as embryonic stem cells (ES cells) and induced pluripotent stem cells (iPS cells), and multipotential stem cells such as mesenchymal stem cells.

In addition, the ability to regulate differentiation induction may be an ability to promote or inhibit differentiation induction, and varies depending on the type of human stem cells.

The above-mentioned composition for inhibiting BMP4 and/or BMP7 signal transduction and/or the differentiation induction regulator of human stem cells, and the composition having an ability to regulate differentiation induction of human stem cells may be a pharmaceutical composition or may be an active pharmaceutical ingredient for producing a pharmaceutical composition, as one embodiment.

Furthermore, in one embodiment, the present invention provides a culture medium supplement for culturing human stem cells and a culture medium for culturing human stem cells that includes the culture medium supplement, where the culture medium supplement for culturing human stem cells includes the peptide, the BMP4 and/or BMP7 signal transduction inhibitor, and/or the inhibitor of differentiation and induction of human stem cells. The culture medium supplement for culture may be in the form of a solution or a dried solid (for example, solid, powder, etc.). When the culture medium supplement is in the form of a solution, it may be used as a culture medium for culture as it is, or it may be diluted with a solvent and the above-mentioned supplement may be added thereto if necessary to be used as a culture medium. Examples of the solvent used for dilution include water, buffer solutions, physiological saline, and culture media used for various cells and tissue cultures, and these may be used alone or in combination of two or more types.

The culture medium for culture of the present invention is not particularly limited as long as it is a culture medium for culturing cells or tissues, but it can be prepared by appropriately using a culture medium normally used for culturing the target human stem cells as the base culture medium.

Noggin, Chordin, follistatin, and the like are known as secretory proteins that act as BMP inhibitors and have an ability to regulate differentiation induction. However, since these are proteins, there are problems such as high culture costs and poor reproducibility when used for cell culture. The differentiation induction regulator of human stem cells that contains the peptide of the present invention has a lower production cost and contains fewer impurities proteins because of its easy purification, compared to the above proteins. Therefore, by using the culture medium of the present invention, it is possible to reduce culture costs and perform experiments of human stem cell differentiation induction with high reproducibility compared to conventional ones.

The composition that includes the peptide of the present invention may appropriately include a known carrier (for example, water, physiological saline, or excipient) other than the peptide of the present invention.

An agent that contains the peptide of the present invention includes an effective amount of the peptide of the present invention as an active ingredient. The agent that contains the peptide of the present invention can be produced by mixing, for example, the peptide of the present invention and a known carrier. Examples of the dosage form of such an agent include an injection and an oral agent. When the agent of the present invention is administered to a mammal (for example, human, mouse, rat, guinea pig, rabbit, dog, horse, monkey, pig, sheep, and the like), particularly to a human, the dosage thereof varies depending on symptoms, age of a patient, sex, weight, sensitivity difference, an administration method, administration intervals, a type of active ingredient, and a type of preparation, and is not particularly limited. The dosage may be, for example, 30 µg to 1,000 mg, 100 µg to 500 mg, or 100 µg to 100 mg administered once or in several doses. In the case of administration by an injection, the dosage may be, for example, 1 µg/kg to 3,000 µg/kg, or 3 µg/kg to 1,000 µg/kg administered once or in several doses depending on the patient's weight.

### EXAMPLES

The present invention is described in more detail below with reference to the Examples. However, the present invention is not limited to the following Examples. A person skilled in the art may easily modify or change the present invention based on the description of the present specification, and these modifications and changes are included in the technical scope of the present invention.

### Chemical synthesis

All raw materials, building blocks, reagents, acids, bases, solid-phase resins, and solvents used in the chemical syntheses of the following Examples were used as they were commercially available, or they can be synthesized by those skilled in the art using organic chemical techniques. An amino acid including a protecting group was used as it was commercially available unless otherwise noted.

The elongation of a peptide chain in the solid-phase resin was performed by using the resins described in the Examples as starting materials and using commonly used peptide coupling reaction conditions and Fmoc removal reaction conditions. The reactions were performed using Syro I or Syro II available from Biotage, or Liberty Blue, Liberty Blue HT12, or Liberty Prime available from CEM, which is an automated peptide synthesizer, according to the manufacturer's manual.

The resin used was Fmoc-D-Glu(tBu)-Wang resin, NovaPEG Rink Amide resin, or Seiber Amide resin, and the amount used ranged from 4 mg to 2 g for each peptide.

The amount of the reagent cocktail used for the deprotection of the side chain and the cleavage of the side chain from the solid-phase resin is 0.2 mL to 50 mL for each peptide, and solutions of the following compositions were used.
Composition A: TFA/H₂O/TIS/DODT (92.5/2.5/2.5/2.5)
Composition B: TFA/H₂O/TIS/DODT (90/2.5/2.5/5)

Common amino acids that were used are listed below, and the side chain protecting groups are indicated in parentheses:
Fmoc-Ala-OH;
Fmoc-Arg(Pbf)-OH;
Fmoc-Asn(Trt)-OH;
Fmoc-Asp(OMpe)-OH;
Fmoc-Asp(OtBu)-OH;
Fmoc-Cys(Trt)-OH;
Fmoc-Gln(Trt)-OH;
Fmoc-Glu(OtBu)-OH;
Fmoc-Gly-OH;
Fmoc-Ile-OH;
Fmoc-His(Boc)-OH;
Fmoc-His(Trt)-OH;
Fmoc-Leu-OH;
Fmoc-Lys(Boc)-OH;
Fmoc-Phe-OH;
Fmoc-Pro-OH;
Fmoc-Ser(tBu)-OH;
Fmoc-Ser(Trt)-OH;
Fmoc-Thr(tBu)-OH;
Fmoc-Thr(Trt)-OH;
Fmoc-Tyr(tBu)-OH;
Fmoc-Val-OH

As a method for purifying the obtained crude purified peptide, a reverse phase preparative HPLC with AutoPurification System-SQD2 single quadruple mass spectrometer available from Waters was used, and elution was performed while m/z ions derived from the target were monitored. It was confirmed that the mass spectrum obtained in ESI-positive scan mode and the mass spectrum including multivalent ions calculated from the molecular formula of the target correspond within the error range of the mass analyzer used. Note that the purification conditions including the column used were shown in each Example.

The structure of the chemically synthesized peptides was determined by confirming, by ESI-MS (+) in the mass spectrometry, the amino acids used according to the target sequence and the molecular weight calculated by taking the building blocks used according to necessity into account. Note that "ESI-MS (+)" refers to electrospray ionization mass spectrometry performed in positive ion mode. The detected mass was reported in the unit of "m/z". Compounds with molecular weights greater than approximately 1,000 were frequently detected as multivalent ions.

Unless otherwise specified, the following basic analytical equipment and basic conditions were used for the mass spectrometry of the peptides synthesized in the following Examples. The analysis was performed by using gradient B (%) under either X/Y/Z conditions.

Device: Shimadzu LC/MS system (LC-20ADXR, CTO-20AC, SPD-M20A, SIL-20AXR, CBM-20A and LCMS-2020)
Column temperature: 60°C
Mobile phase A: 0.025% TFA in H₂O
Mobile phase B: 0.025% TFA in MeCN
Flow rate: 0.5 mL/min
Wavelength: 225 nm PDA
Gradient B (%): X: 20 to 60%/7.15 min, 60 to 95%/0.3 min, 95 to 95%/1.55 min;
Y: 40 to 80%/7.15 min, 80 to 95%/0.3 min, 95 to 95%/1.55 min;
Z: 5 to 45%/7.15 min, 45 to 95%/0.3 min, 95 to 95%/1.55 min

### [Example 1]

### Synthesis of BMP4 signal transduction inhibition peptide No. 5 (SEQ ID NO: 5)

In the present Example, BMP4 signal transduction inhibition peptide No. 5(SEQ ID NO: 5) having the following structure was synthesized.

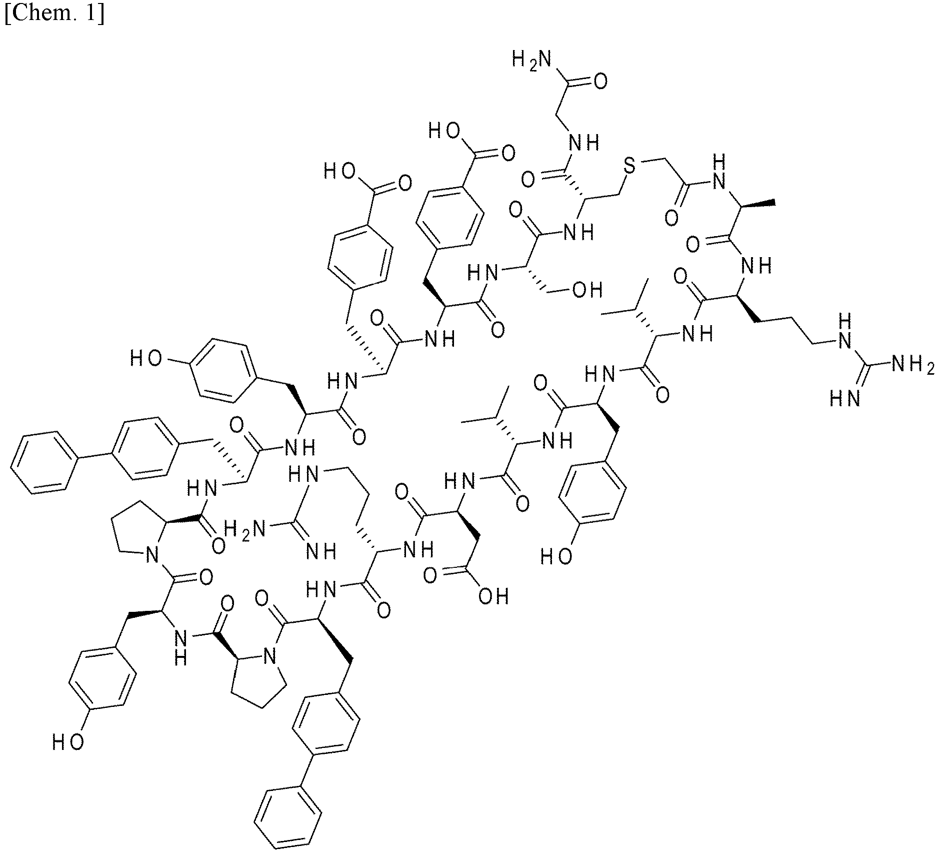

Sieber amide resin (Merck, 0.49 mmol/g) was used to start removing the Fmoc group by a general method, to synthesize a peptide as a target. At that time, Syro II available from Biotage was used as a solid phase synthesizer to perform synthesis according to the manufacturer's manual. For introducing each residue, Fmoc-AA/HATU/DIEA (4.2 equivalents/3.9 equivalents/8.4 equivalents) per 1 equivalent of a resin was used, followed by reaction two times in DMF at 75°C for 10 minutes. Note that the reactions for the second residue, the seventh residue, and the 17th residue were performed two times at 50°C for 30 minutes.

To remove the Fmoc group, the reaction with a 20% piperidine solution in DMF was performed for 5 minutes at room temperature, the solution was removed, and then the reaction with a 20% piperidine solution in DMF was performed at room temperature for 15 minutes again.

For introduction of a chloroacetyl group, the Fmoc group of α-amino group was removed from the solid phase resin that retained the Fmoc-protected peptide obtained in the previous step by the above-described method, and 0.3 M DMF solution of chloroacetic acid (4.2 equivalents), 0.28 M DMF solution of HCTU (3.92 equivalents), and 1.05 M DMF solution of DIPEA (8.4 equivalents) were added to the solid phase resin, followed by repeating shaking two times at room temperature for 30 minutes.

For deprotection of a side chain and cleavage from the solid phase resin, the resin obtained after the step of introducing the chloroacetyl group was washed with DMF five times and methylene chloride three times, followed by drying under reduced pressure. Then, to a reaction vessel charged with the solid phase resin, a reactant cocktail-A (mixture of TFA/H₂O/TIS/DODT with a volume ratio of 92.5:2.5:2.5:2.5) was added, and was shook at room temperature for 60 minutes. The reaction liquid was filtered and collected with a frit. When this filtrate was added to cooled excess diisopropyl ether, precipitations were formed, and this mixture was centrifuged (8,500 rpm, 30 seconds), and the solution was subjected to decantation. The obtained solid was washed with a cooled mixed solvent of diethyl ether/hexane (1/1) again and was then dried under reduced pressure. The obtained solid was used for the subsequent cyclization reaction.

For the cyclization reaction of the peptide, after the peptide was dissolved in DMSO so that the final concentration of the peptide would be 2.5 mM based on the number of moles of the solid phase resin, triethylamine (0.01mL) was added thereto, followed by shaking at room temperature overnight. The obtained reaction solution was concentrated under reduced pressure, and DMSO (0.1 mL) was added to the obtained residues.

The obtained above solution was subjected to solid-phase extraction as described below. (1) Gilson ASPEC (registered trademark) C18 500mg 3 mL (Gilson) column was washed with an extraction liquid A (0.1% TFA in 95%MeCN/H₂O, 3 mL). (2) The column was equilibrated with an extraction liquid B (0.1% TFA in 5%MeCN/H₂O, 3 mL). (3) The reaction solution (0.1 mL) was loaded into the column. (4) The column was washed with the extraction liquid B (4 mL). (5) Extraction using the extraction liquid A (4 mL) was performed. The obtained extraction liquid was concentrated under reduced pressure.

One purity of the main peak of the target, which was calculated from an area ratio of LC/MS (UV wavelength 220 nm) chromatogram under the following analysis conditions, was 43.4%.
Analysis conditions: retention time =1.39 min;
Column: Kinetex (registered trademark) EVO C18 1.7 µm 2.1 × 50 mm;
Mobile phase: A = 0.025% TFA in H₂O, B = 0.025% TFA in MeCN;
Temperature: 60°C;
Gradient (%B conc):5 to 95% for 2.10 minutes, then 95 to 95% for 0.75 minutes;
Flow rate: 0.6 mL/min;
ESI-MS (+) observation value m/z = 1258.0 (M+2H)²⁺.

### [Example 2]

### Synthesis of BMP4 signal transduction inhibition peptide No. 177 (SEQ ID NO: 177)

In the present Example, BMP4 signal transduction inhibition peptide No. 177 (SEQ ID NO: 177) having the following structure was synthesized.

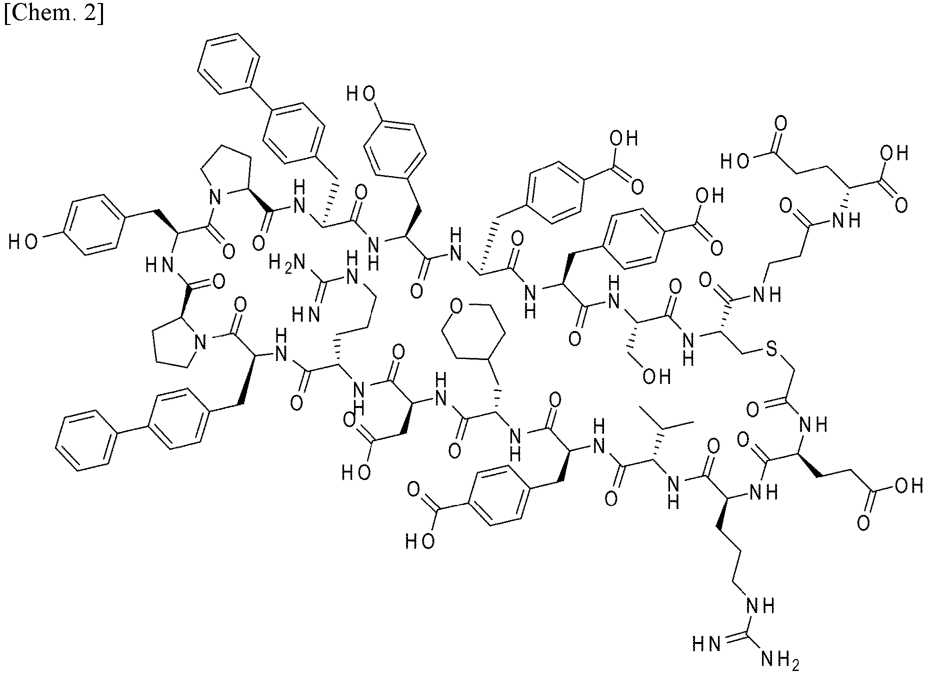

Fmoc-D-Glu (tBu)-Wang resin (WATANABE CHEMICAL INDUSTRIES, LTD., 0.62 mmol/g) was used to start removing the Fmoc group by a general method, to synthesize a peptide as a target. Syro I available from Biotage was used as a solid phase synthesizer to perform synthesis according to the manufacturer's manual. For introducing each residue, Fmoc-AA/HATU/DIPEA (4.2 equivalents/4 equivalents/8.4 equivalents) per 1 equivalent of a resin was used, followed by reaction two times in DMF at 75°C for 20 minutes. Note that the reactions for the second residue and the seventh residue were performed two times at room temperature for 30 minutes. The reaction for the 17th residue was performed once at room temperature for 30 minutes. The reaction for the 18th residue was performed once at 75°C for 20 minutes.

To remove the Fmoc group, such conditions, in which the reaction with a 20% piperidine solution in DMF was performed for 5 minutes at room temperature, the solution was removed, and then the reaction with a 20% piperidine solution in DMF was performed at room temperature for 15 minutes again, were used.

For introduction of a chloroacetyl group, after the Fmoc group of α-amino group was removed from the solid phase resin that retained the Fmoc-protected peptide obtained by the previous step, by the above-described method, a DCM/DMF solution of ClAcOSu obtained from chloroacetic acid (10 equivalents), DIPCI (10 equivalents), and HOSu (10 equivalents) was added to the solid phase resin, and was shook at room temperature for 60 minutes.

For deprotection of a side chain and cleavage from the solid phase resin, the resin obtained after the step of introducing the chloroacetyl group was washed with DMF five times and methylene chloride three times, followed by drying under reduced pressure. Then, to a reaction vessel charged with the solid phase resin, a reactant cocktail-A (mixture of TFA/H₂O/TIS/DODT with a volume ratio of 92.5:2.5:2.5:2.5) was added, and was shook at room temperature for 90 minutes. The reaction liquid was filtered and collected with a frit. The solid-phase resin that remained in the reaction vessel was shook again with a cocktail for cleavage, and solution components were collected using a frit and were mixed with the above-described filtrate. The filtrate was added to a mixture solvent of diethyl ether/hexane (1/1), to thereby cause precipitation. Then this mixture was centrifuged and the solution was subjected to decantation. The obtained solid was washed with diethyl ether again, and was dried under reduced pressure. The obtained solid was used for the next cyclization reaction.

For the cyclization reaction of the peptide, after the peptide was dissolved in MeCN/H₂O (1/1) so that the final concentration of the peptide would be 5 mM based on the number of moles of the solid phase resin, triethylamine (10 equivalents) was added thereto, followed by shaking at room temperature for 15 hours. After acetic acid was added to the obtained reaction solution, the resultant was concentrated under reduced pressure using Genevac EZ-II elite.

The obtained crude product was purified using the following conditions.
Column: Waters XBridge (registered trademark) C18, 30 × 150 mm;
Mobile phase: A = 0.1% TFA in H₂O, B = 0.1% TFA in MeCN; temperature: 50°C; gradient (%B conc.):9 to 34% for 3 minutes, then 34 to 39% for 8 minutes, then 39 to 60% for one minute; flow rate: 45 mL/minute
The purity of the target, which was calculated from an area ratio of LC/MS (UV wavelength 225 nm) chromatogram under the following analysis conditions, was 93.5%.
Analysis conditions: retention time = 4.49 min;
Column: Kinetex (registered trademark) EVO C18 2.6 µm 2.1 × 150 mm, 100 Å;
Mobile phase: A = 0.025% TFA in H₂O, B = 0.025% TFA in MeCN;
Temperature: 60°C; gradient (%B conc): 20 to 60% for 7.15 minutes, then 60 to 95% for 0.30 minutes, then 95 to 95% for 1.55 minutes;
Flow rate: 0.5 mL/min
ESI-MS (+) observation value m/z = 1400.9 (M+2H)²⁺.

### [Example 3]

### Synthesis of BMP4 signal transduction inhibition peptide No. 84 (SEQ ID NO: 84)

In the present Example, BMP4 signal transduction inhibition peptide No. 84 (SEQ ID NO: 84) having the following structure was synthesized.

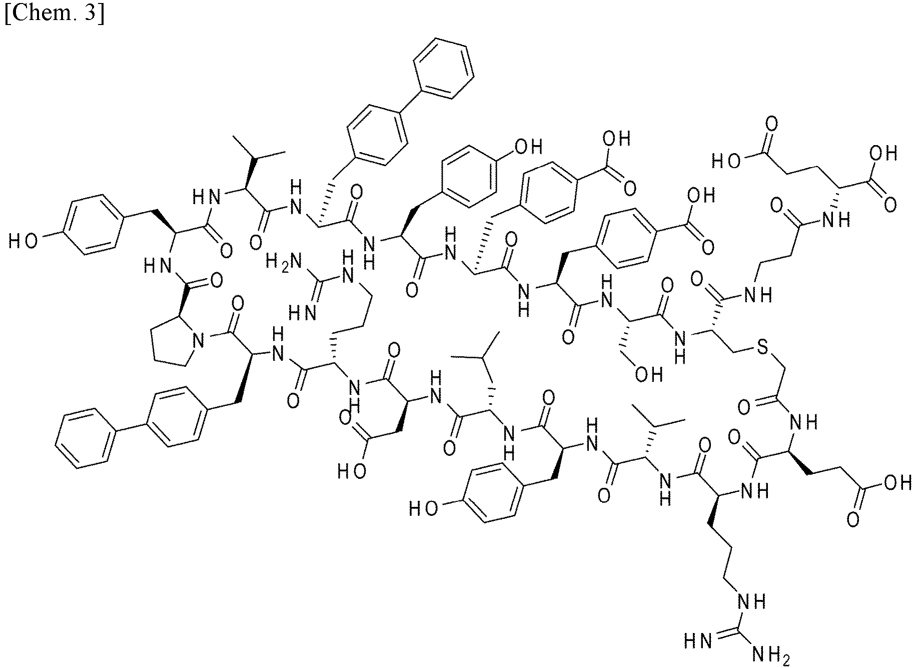

Fmoc-D-Glu (OtBu)-Wang resin (WATANABE CHEMICAL INDUSTRIES, LTD., 0.79 mmol/g) was used to start removing the Fmoc group by a general method, to synthesize a peptide as a target. Syro I available from Biotage was used as a solid phase synthesizer to perform synthesis according to the manufacturer's manual. For introducing each residue, Fmoc-AA/HATU/DIPEA (4.2 equivalents/4 equivalents/8.4 equivalents) per 1 equivalent of a resin was used, followed by reaction two times in DMF at 75°C for 20 minutes. Note that the reaction for the second residue was performed two times at 75°C for 30 minutes. The reaction for the seventh residue was performed two times at 50°C for 30 minutes. The reaction for the 17th residue was performed two times at room temperature for 30 minutes.

To remove the Fmoc group, such conditions, in which the reaction with a 20% piperidine solution in DMF was performed for 5 minutes at room temperature, the solution was removed, and then the reaction with a 20% piperidine solution in DMF was performed at room temperature for 15 minutes again, were used.

For introduction of a chloroacetyl group, an NMP/DCM solution of ClAcOSu obtained from ClAcOH/DIPCI/HOSu (5.3 equivalents/5.25 equivalents/5.3 equivalents) relative to 1 equivalent of the solid phase resin that retained the Fmoc-protected peptide obtained in the previous step was added to the solid phase resin, and was shook at room temperature for 90 minutes.

For deprotection of a side chain and cleavage from the solid phase resin, the resin obtained after the step of introducing the chloroacetyl group was washed with DMF five times and methylene chloride three times, followed by drying under reduced pressure. Then, to a reaction vessel charged with the solid phase resin, a reactant cocktail-A (mixture of TFA/H₂O/TIS/DODT with a volume ratio of 92.5:2.5:2.5:2.5) was added, and was shook at room temperature for 90 minutes. The reaction liquid was filtered and collected with a frit. The solid-phase resin that remained in the reaction vessel was shook again with a cocktail for cleavage, and solution components were collected using a frit and were mixed with the above-described filtrate. When this filtrate was added to cooled excess diisopropyl ether, precipitations were formed, and this mixture was centrifuged to remove the supernatant. The obtained solid was washed with cooled diethyl ether again, and was then dried under reduced pressure. The obtained solid was used for the subsequent cyclization reaction.

For the cyclization reaction of the peptide, after the peptide was dissolved in DMSO/H₂O (9/1) so that the final concentration of the peptide would be 2.5 mM based on the number of moles of the solid phase resin, triethylamine (10 equivalents) was added thereto, followed by shaking at room temperature for 2 hours. After acetic acid was added to the obtained reaction solution, the resultant was concentrated under reduced pressure using Genevac EZ-II elite.

The obtained crude product was purified under the following conditions.
Column: Jeanious One-Column, 20 × 150 mm (available from ChromaJean);
Mobile phase: A = 0.1% TFA in H₂O, B = 0.1% TFA in MeCN; temperature: room temperature; gradient (%B conc): 25.9 to 45.9% for 9 minutes, then 45.9 to 90% for 0.01 minutes;
Flow rate: 20 mL/min
The purity of the target, which was calculated from an area ratio of LC/MS (UV wavelength 225 nm) chromatogram under the following analysis conditions, was 82.8%.
Analysis conditions: retention time = 4.14 min; Column: Kinetex (registered trademark) EVO C18 2.6 µm 2.1 × 150 mm, 100Å; mobile phase: A = 0.025% TFA in H₂O, B = 0.025% TFA in MeCN; temperature: 60°C; gradient (%B conc): 20 to 60% for 7.15 minutes, then 60 to 95% for 0.30 minutes, then 95 to 95% for 1.55 minutes; flow rate: 0.5 mL/min
   ESI-MS (+) observation value m/z = 1359.9 (M+2H)2+.

### [Example 4]

### Synthesis of BMP4 signal transduction inhibition peptide No. 87 (SEQ ID NO: 87)

In the present Example, BMP4 signal transduction inhibition peptide No. 87 (SEQ ID NO: 87) having the following structure was synthesized.

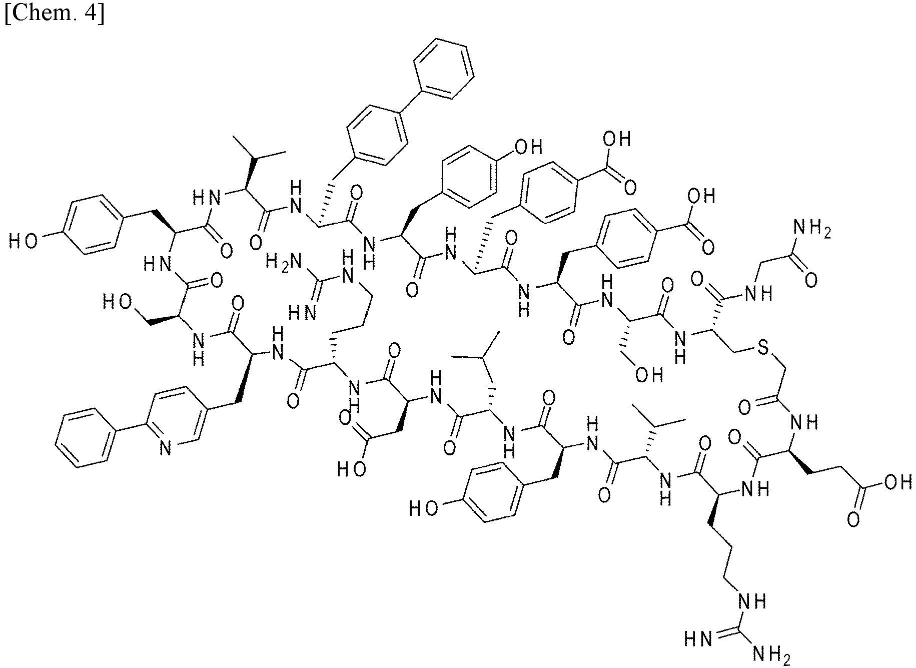

Sieber Amide resin (available from Merck, 0.48 mmol/g) was used to start removing the Fmoc group by the above-described general method, to synthesize a peptide as a target. Syro I available from Biotage was used as a solid phase synthesizer to perform synthesis according to the manufacturer's manual. For introducing each residue, Fmoc-AA/HATU/DIPEA (4.2 equivalents/4 equivalents/8.4 equivalents) per 1 equivalent of a resin was used, followed by reaction two times in DMF at 75°C for 20 minutes. Note that the reaction for the second residue was performed two times at 75°C for 30 minutes. The reaction for the seventh residue was performed two times at 50°C for 30 minutes. The reaction for the 17th residue was performed two times at room temperature for 30 minutes.

To remove the Fmoc group, such conditions, in which the reaction with a 20% piperidine solution in DMF was performed for 5 minutes at room temperature, the solution was removed, and then the reaction with a 20% piperidine solution in DMF was performed at room temperature for 15 minutes again, were used.

For introduction of a chloroacetyl group, an NMP/DCM solution of ClAcOSu obtained from ClAcOH/DIPCI/HOSu (5.3 equivalents/5.25 equivalents/5.3 equivalents) relative to 1 equivalent of the solid phase resin that retained the Fmoc-protected peptide obtained in the previous step was added to the solid phase resin, and was shook at room temperature for 90 minutes.

For deprotection of a side chain and cleavage from the solid phase resin, the resin obtained after the step of introducing the chloroacetyl group was washed with DMF five times and methylene chloride three times, followed by drying under reduced pressure. Then, to a reaction vessel charged with the solid phase resin, a reactant cocktail-A (mixture of TFA/H₂O/TIS/DODT with a volume ratio of 92.5:2.5:2.5:2.5) was added, and was shook at room temperature for 90 minutes. The reaction liquid was filtered and collected with a frit. The solid-phase resin that remained in the reaction vessel was shook again with a cocktail for cleavage, and solution components were collected using a frit and were mixed with the above-described filtrate. When this filtrate was added to cooled excess diisopropyl ether, precipitations were formed, and this mixture was centrifuged to remove the supernatant. The obtained solid was washed with cooled diethyl ether again, and was then dried under reduced pressure. The obtained solid was used for the subsequent cyclization reaction.

For the cyclization reaction of the peptide, after the peptide was dissolved in DMSO/H₂O (9/1) so that the final concentration of the peptide would be 2.5 mM based on the number of moles of the solid phase resin, triethylamine (10 equivalents) was added thereto, followed by shaking at room temperature for 2 hours. After acetic acid was added to the obtained reaction solution, the resultant was concentrated under reduced pressure using Genevac EZ-II elite.

The obtained crude product was purified using the following conditions.
Column: Jeanious One-Column, 20 × 150 mm (available from ChromaJean);
Mobile phase: A = 0.1% TFA in H₂O, B = 0.1% TFA in MeCN; temperature: room temperature; gradient (%B conc): 18.7 to 38.7% for 9 minutes, then 38.7 to 90% for 0.01 minutes; flow rate: 20 mL/min
The purity of the target, which was calculated from an area ratio of LC/MS (UV wavelength 225 nm) chromatogram under the following analysis conditions, was 90.3%.
Analysis conditions: retention time = 3.15 minutes; Column: Kinetex (registered trademark) EVO C18 2.6 µm 2.1 × 150 mm, 100Å; mobile phase: A = 0.025% TFA in H₂O, B = 0.025% TFA in MeCN; temperature: 60°C; gradient (%B conc):20 to 60% for 7.15 minutes, then 60 to 95% for 0.30 minutes, then 95 to 95% for 1.55 minutes; flow rate: 0.5 mL/min
ESI-MS (+) observation value m/z = 1283.6 (M+2H)²⁺.

### [Example 5]

### Synthesis of BMP7 signal transduction inhibition peptide

In the present Example, BMP7 signal transduction inhibition peptide (ClAc-F-G-MeF-G-Bph-G-D-D-Cha-A-MeF-L-H-C-G-NH2, SEQ ID NO: 371, -NH2 is an addition sequence) having the following structure was synthesized.

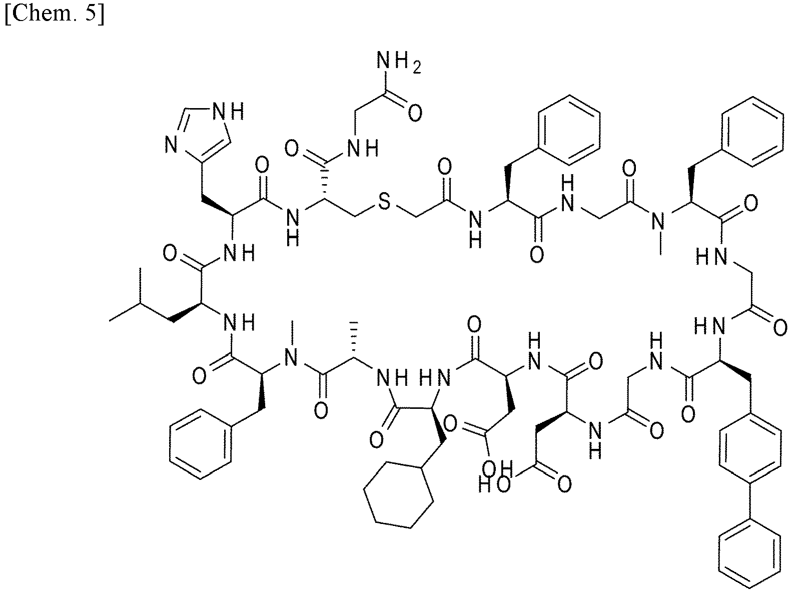

Sieber amide resin (WATANABE CHEMICAL INDUSTRIES, LTD., 0.57 mmol/g) was used to start removing the Fmoc group by a general method, to synthesize a peptide as a target. At that time, Syro II available from Biotage was used as a solid phase synthesizer to perform synthesis according to the manufacturer's manual. For introducing each residue, Fmoc-AA/HATU/DIEA (4.2 equivalents/3.9 equivalents/8.4 equivalents) per 1 equivalent of a resin was used, followed by reaction two times in DMF at 75°C for 20 minutes. The reaction for the 11th residue and the reaction for 13th residue were performed two times at 50°C for 30 minutes. The reaction for the 14th residue was performed two times at 25°C for 20 minutes.

To remove the Fmoc group, the reaction with a 20% piperidine solution in DMF was performed for 5 minutes at room temperature, the solution was removed, and then the reaction with a 20% piperidine solution in DMF was performed at room temperature for 15 minutes again.

For introduction of a chloroacetyl group, after the Fmoc group of α-amino group was removed from the solid phase resin that retained the Fmoc-protected peptide obtained in the previous step by the above-described method, 0.3 M DMF solution of chloroacetic acid (4.2 equivalents), 0.28 M DMF solution of HCTU (3.9 equivalents), and 1.05 M DMF solution of DIEA (8.4 equivalents) were added to the solid phase resin, followed by shaking two times at room temperature for 30 minutes.

For deprotection of a side chain and cleavage from the solid phase resin, the resin obtained after the step of introducing the chloroacetyl group was washed with DMF three times and methylene chloride three times, followed by drying under reduced pressure. Then, to a reaction vessel charged with the solid phase resin, a reactant cocktail-A (mixture of TFA/H₂O/TIS/DODT with a volume ratio of 92.5:2.5:2.5:2.5) was added, and was shook at room temperature for 60 minutes. The reaction liquid was filtered and collected with a frit. When this filtrate was added to cooled excess diisopropyl ether, precipitations were formed, and this mixture was centrifuged (8,500 rpm, 30 seconds) and the solution was subjected to decantation. The obtained solid was washed with a cooled mixted solvent of diethyl ether/hexane (1/1) again. This solid was centrifuged again (8,500 rpm, 30 seconds) and the solution was subjected to decantation, followed by drying under reduced pressure. The obtained solid was used for the subsequent cyclization reaction.

For the cyclization reaction of the peptide, after the peptide was dissolved in 30% water-containing DMSO so that the final concentration of the peptide would be 2.5 mM based on the number of moles of the solid phase resin, triethylamine (10 equivalents) was added thereto, followed by shaking at room temperature overnight. The obtained reaction solution was concentrated under reduced pressure using Genevac EZ-2 Elite, DMSO (0.1 mL) was added to the obtained residue.

The above solution obtained was subjected to solid phase extraction using a column available from GILSON (column: Gilson ASPEC C18 500 mg 3 mL): (1) The column was washed with an extraction liquid A (0.1% TFA in 95% MeCN/H₂O, 3 mL). (2) The column was equilibrated with an extraction liquid B (0.1% TFA in 5%MeCN/H₂O, 3 mL). (3) The above reaction solution (0.1 mL) was loaded into the column. (4) The column was washed with the extraction liquid B (4 mL). (5) Extraction was performed with the extraction liquid A (4 mL). The obtained extraction liquid was concentrated under reduced pressure using EZ-2 Elite.

One purity of the main peak of the target, which was calculated from an area ratio of LC/MS (UV wavelength 220 nm) chromatogram of the following analysis conditions, was 60.1%.

Analysis conditions: retention time = 1.58 min; Column: Kinetex EVO C18 1.7 µm 2.1 × 50 mm; mobile phase: A = 0.025% TFA in H₂O, B = 0.025% TFA in MeCN; temperature: 60°C; gradient (%B conc):5 to 95% for 2.10 minutes, then 95 to 95% for 0.75 minutes; flow rate: 0.6 mL/min
ESI-MS (+) observation value m/z = 894.2 (M+2H)²⁺.

### [Example 6]

### Synthesis of BMP7 signal transduction inhibition peptide

In the present Example, BMP7 signal transduction inhibition peptide (ClAc-F4F-G-MeF-G-3Py6Ph-G-D-E-Cha-Hgl-MeF4Me-L-P4Sh-C-bA-de-OH, SEQ ID NO: 417, bA-de-OH is an addition sequence) having the following structure was synthesized.

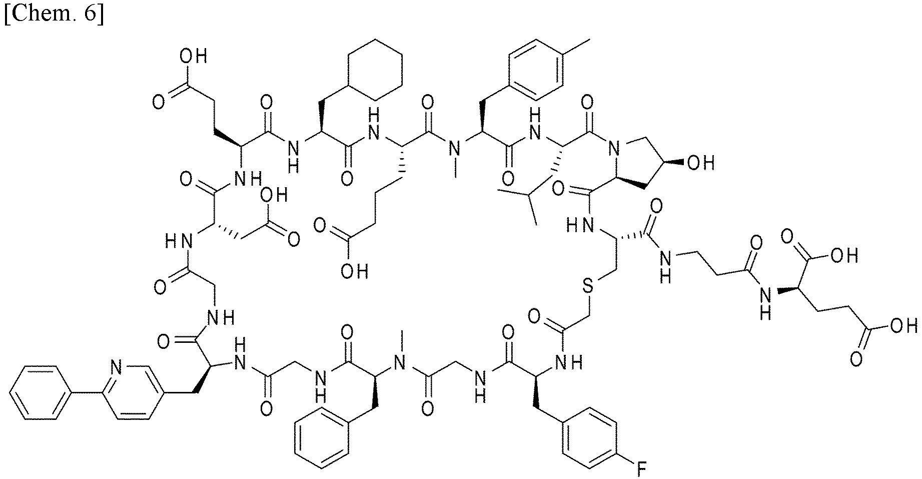

Fmoc-d-Glu(tBu)-Wang resin (WATANABE CHEMICAL INDUSTRIES, LTD., 0.62 mmol/g) was used to start removing the Fmoc group by a general method, to synthesize a peptide as a target. Liberty Prime available from CEM was used as a solid phase synthesizer to perform synthesis according to the manufacturer's manual. For introduction of each residue, the Fmoc-AA/DIC/Oxyma pure (5.25 equivalents/10 equivalents/5 equivalents) relative to 1 equivalent of a resin was used to perform reaction in DMF one time at 105°C for two minutes. Note that Fmoc-AA dissolved in NMP was used for the fifth residue. The reaction for the second residue and the reaction for the tenth residue were performed two times at 90°C for 10 minutes. The reaction for the 14th residue was performed one time at 50°C for 15 minutes.

To remove the Fmoc group, such a condition that a DMF solution of 4% pyrrolidine was allowed to react at 110°C for a minute was used as a standard. Note that the first residue, the third residue, the fourth residue, the fifth residue, the sixth residue, and seventh residue were allowed to react with a DMF solution of 10% pyrrolidine at 50°C for 90 seconds. For the second residue and the tenth residue, a condition, in which reaction with a DMF solution of 10% pyrrolidine was performed for one minute, and the solution was removed, followed by reaction with a DMF solution of 10% pyrrolidine at room temperature for one minute, was used.

The chloroacetyl group was introduced by adding a DCM/DMF solution of ClAcOSu (10 equivalents) to the solid phase resin, followed by shaking at room temperature for 60 minutes.

For deprotection of a side chain and cleavage from the solid phase resin, the resin obtained after the step of introducing the chloroacetyl group was washed with DMF and methylene chloride and then diethyl ether, followed by drying under reduced pressure. Then, to a reaction vessel charged with the solid phase resin, a reactant cocktail-A (mixture of TFA/H₂O/TIS/DODT with a volume ratio of 92.5:2.5:2.5:2.5) was added, and was shook at room temperature for 75 minutes. The reaction liquid was filtered and collected with a frit.

When the obtained filtrate was added to a cooled mixture solvent of excess diethyl ether/hexane (1/1), precipitations were formed, and this mixture was centrifuged, and the solution was subjected to decantation. The obtained solid was washed with diethyl ether again and was dried under reduced pressure. The obtained solid was used for the subsequent cyclization reaction.

For the cyclization reaction of the peptide, after the peptide was dissolved in DMSO so that the final concentration of the peptide would be 5 mM based on the number of moles of the solid phase resin, triethylamine (10 equivalents) was added thereto, followed by shaking at room temperature for 20 hours. After acetic acid was added to the obtained reaction solution, the resultant was concentrated under reduced pressure using Genevac EZ-II elite.

The obtained crude product was purified using the following conditions (Column: Waters XBridge (registered trademark) C18, 19 × 150 mm; mobile phase: A= 0.1% TFA in H₂O, B = 0.1% TFA in MeCN; temperature: 50°C; gradient (%B conc.): 12 to 37% for 3 minutes, then 37 to 42% for 8 minutes, and then 42 to 60% for a minute; flow rate: 17 mL/min.

The purity of the target, which was calculated from an area ratio of LC/MS (UV wavelength 225 nm) chromatogram under the following analysis conditions, was 97.93%.

Analysis conditions: retention time = 5.48 min; Column: Kinetex EVO C18 2.6 µm 2.1 × 150 mm, 100Å; mobile phase: A = 0.025% TFA in H₂O, B = 0.025% TFA in MeCN; temperature: 60°C; gradient (%B conc): 20 to 60% for 7.15 minutes, then 60 to 95% for 0.30 minutes, then 95 to 95% for 1.55 minutes; flow rate: 0.5 mL/min

ESI-MS (+) observation value m/z = 1013.5 (M+2H)²⁺.

### [Example 7]

### Synthesis of BMP7 signal transduction inhibition peptide

In the present Example, BMP7 signal transduction inhibition peptide (ClAc-F-G-MeF-G-3Py6Ph-G-D-E-Cha-Hgl-MeF-L-P4Sh-C-bA-de-OH, SEQ ID NO: 415, bA-de-OH was an addition sequence) having the following structure was synthesized.

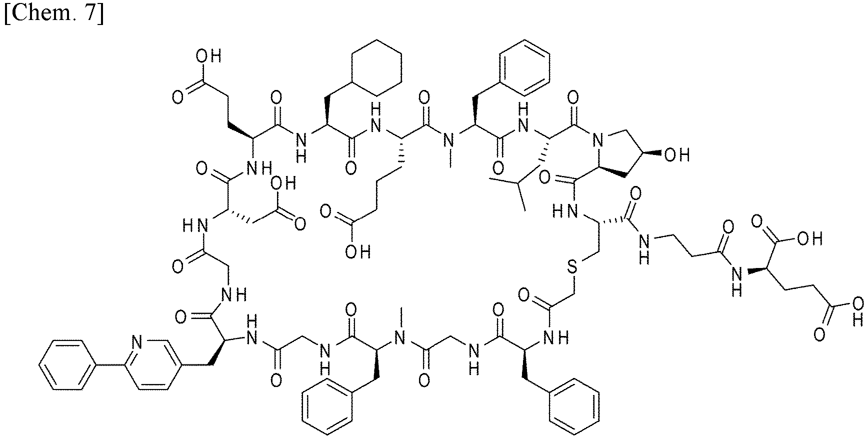

Fmoc-d-Glu(tBu)-Wang resin (WATANABE CHEMICAL INDUSTRIES, LTD., 0.62 mmol/g) was used to start removing the Fmoc group by a general method, to synthesize a peptide as a target. Liberty Prime available from CEM was used as a solid phase synthesizer to perform synthesis according to the manufacturer's manual. For introduction of each residue, the Fmoc-AA/DIC/Oxyma pure (5.25 equivalents/10 equivalents/5 equivalents) relative to 1 equivalent of a resin was used to perform reaction in DMF one time at 105°C for two minutes. Note that Fmoc-AA dissolved in NMP was used for the fifth residue. The reaction for the second residue and the reaction for the tenth residue were performed two times at 90°C for 10 minutes. The reaction for the 14th residue was performed one time at 50°C for 15 minutes. The reaction for the 15th residue was performed one time at 105°C for one minute.

To remove the Fmoc group, such a condition that a DMF solution of 4% pyrrolidine was allowed to react at 110°C for one minute was used as a standard. Note that the first residue, the third residue, the fourth residue, the fifth residue, sixth residue, and the seventh residue were allowed to react with a DMF solution of 10% pyrrolidine at 50°C for 90 seconds. For the second residue and the tenth residue, a condition, in which reaction with a DMF solution of 10% pyrrolidine was performed for one minute, and the solution was removed, followed by reaction with a DMF solution of 10% pyrrolidine at room temperature for one minute, was used.

The chloroacetyl group was introduced by adding a DCM/DMF solution of ClAcOSu (10 equivalents) to the solid phase resin, followed by shaking at room temperature for 60 minutes. For deprotection of a side chain and cleavage from the solid phase resin, the resin obtained after the step of introducing the chloroacetyl group was washed with DMF and methylene chloride and then diethyl ether, followed by drying under reduced pressure. Then, to a reaction vessel charged with the solid phase resin, a reactant cocktail-A (mixture of TFA/H₂O/TIS/DODT with a volume ratio of 92.5:2.5:2.5:2.5) was added, and was shook at room temperature for 75 minutes. The reaction liquid was filtered and collected with a frit.

When the obtained filtrate was added to a cooled mixture solvent of excess diethyl ether/hexane (1/1), precipitations were formed, and this mixture was centrifuged, and the solution was subjected to decantation. The obtained solid was washed with diethyl ether again and was dried under reduced pressure. The obtained solid was used for the subsequent cyclization reaction.

For the cyclization reaction of the peptide, after the peptide was dissolved in DMSO so that the final concentration of the peptide would be 5 mM based on the number of moles of the solid phase resin, triethylamine (10 equivalents) was added thereto, followed by shaking at room temperature for 20 hours. After acetic acid was added to the obtained reaction solution, the resultant was concentrated under reduced pressure using Genevac EZ-II elite.

The obtained crude product was purified using the following conditions (Column: Jeanious One-Column 20 mm I. D. × 150 mmL; mobile phase: A = 0.1% TFA in H₂O, B = 0.1% TFA in MeCN; temperature: 60°C; gradient (%B conc.): 27.6 to 47.6% for 9 minutes; flow rate: 20 mL/min.

The purity of the target, which was calculated from an area ratio of LC/MS (UV wavelength 225 nm) chromatogram under the following analysis conditions, was 94.88%.

Analysis conditions: retention time = 5.40 minutes; Column: Kinetex EVO C18 2.6 µm 2.1 × 150 mm, 100Å; mobile phase: A = 0.025% TFA in H₂O, B = 0.025% TFA in MeCN; temperature: 60°C; gradient (%B conc):20 to 60% for 7.15 minutes, then 60 to 95% for 0.30 minutes, then 95 to 95% for 1.55 minutes; flow rate: 0.5 mL/min

ESI-MS (+) observation value m/z = 997.5 (M+2H)²⁺.

### [Example 8]

### Synthesis of BMP4 signal transduction inhibition peptide

In the present Example, BMP4 signal transduction inhibition peptide (ClAc-E-R-V-F4COO-V-KCOpipzaa-R-3Py6Ph-KCOpipzaa-Y-Hyp-Bph-Y-F4COO-F4COO-S-C-G-NH2, SEQ ID NO: 277) having the following structure was synthesized.

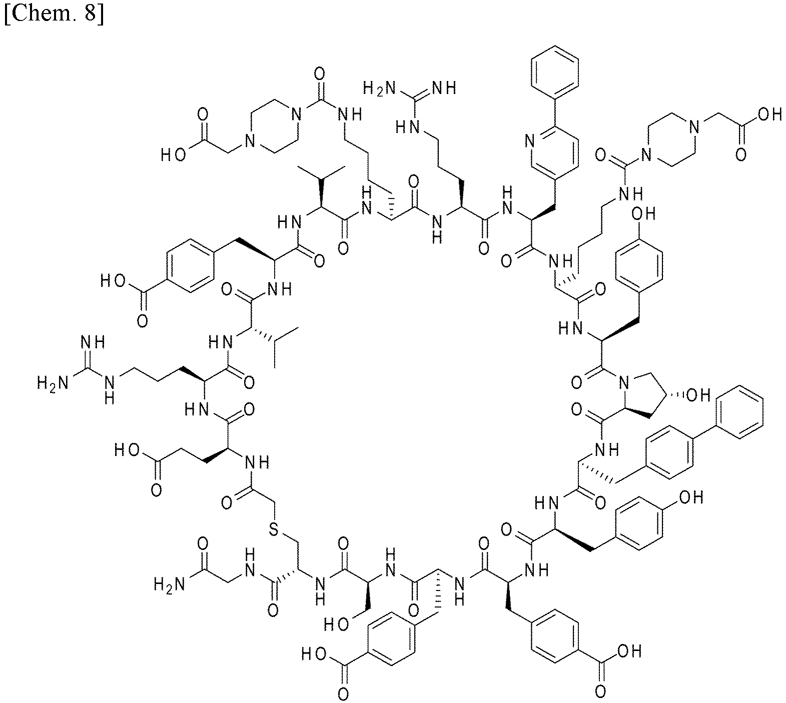

Sieber amide resin (WATANABE CHEMICAL INDUSTRIES, LTD., 0.57 mmol/g) was used to start removing the Fmoc group by a general method, to synthesize a peptide as a target. Liberty Prime available from CEM was used as a solid phase synthesizer to perform synthesis according to the manufacturer's manual. For introduction of each residue, the Fmoc-AA/DIC/Oxyma pure (4.2 equivalents/16 equivalents/6 equivalents) relative to 1 equivalent of a resin was used to perform reaction in DMF one time at 105°C for 90 seconds. Note that the eighth residue, Fmoc-AA dissolved in NMP was used. The reaction for the second residue and the reaction for the seventh residue were performed two times at 50°C for 15 minutes. The reaction for the 17th residue was performed one time at 50°C for 15 minutes.

To remove the Fmoc group, a condition, in which reaction with a DMF solution of 4% pyrrolidine was performed at 110°C for one minute, was used.

A chloroacetyl group was introduced by adding a DMF solution of ClAcOSu (5 equivalents) to the solid phase resin, followed by shaking at room temperature for 60 minutes.

For deprotection of a side chain and cleavage from the solid phase resin, the resin obtained after the step of introducing the chloroacetyl group was washed with DMF and methylene chloride and then diethyl ether, followed by drying under reduced pressure. Then, to a reaction vessel charged with the solid phase resin, a reactant cocktail-A (mixture of TFA/H₂O/TIS/DODT with a volume ratio of 92.5:2.5:2.5:2.5) was added, and was shook at room temperature for 90 minutes. The reaction liquid was filtered and collected with a frit.

When the obtained filtrate was added to a cooled mixture solvent of excess diisopropyl ether/hexane (1/1), precipitations were formed. This mixture was centrifuged, and the solution was subjected to decantation. The obtained solid was washed with diethyl ether again and was dried under reduced pressure. The obtained solid was used for the subsequent cyclization reaction.

For the cyclization reaction of the peptide, after the peptide was dissolved in DMSO so that the final concentration of the peptide would be 2.5 mM based on the number of moles of the solid phase resin, triethylamine (10 equivalents) was added thereto, followed by shaking at room temperature for 8 hours. After acetic acid was added to the obtained reaction solution, the resultant was concentrated under reduced pressure using Genevac EZ-II elite.

The obtained crude product was purified using the following conditions (Column: Waters XBridge (registered trademark) C18, 30 × 150 mm; mobile phase: A = 0.1% TFA in H₂O, B = 0.1% TFA in MeCN; temperature: 50°C; gradient (%B conc.): 5 to 22% for 3 minutes, then 22 to 27% for 8 minutes, then 27 to 60% for one minute; flow rate: 45 mL/minute.

The purity of the target, which was calculated from an area ratio of LC/MS (UV wavelength 225 nm) chromatogram under the following analysis conditions, was 93.29%.

Analysis conditions: retention time = 5.36 min; Column: Kinetex EVO C18 2.6 µm 2.1 × 150 mm, 100Å; mobile phase: A = 0.025% TFA in H₂O, B = 0.025% TFA in MeCN; temperature: 60°C; gradient (%B conc): 5 to 45% for 7.15 minutes, then 45 to 95% for 0.30 minutes, then 95 to 95% for 1.55 minutes; flow rate: 0.5 mL/min

ESI-MS (+) observation value m/z = 1001.3(M+3H)³⁺.

### [Example 9]

### Synthesis of BMP4 signal transduction inhibition peptide

In the present Example, BMP4 signal transduction inhibition peptide (ClAc-E-R-V-F4COO-V-KCOpipzaa-R-3Py6Ph-KCOpipzaa-Y-Hyp- Bph3F-Y-F4COO-F4COO-S-C-G-NH2, SEQ ID NO: 311) having the following structure was synthesized.

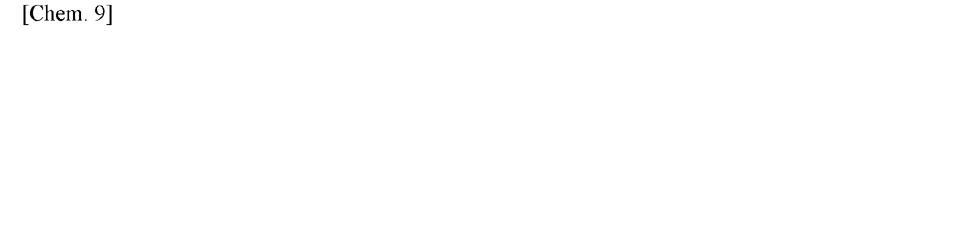

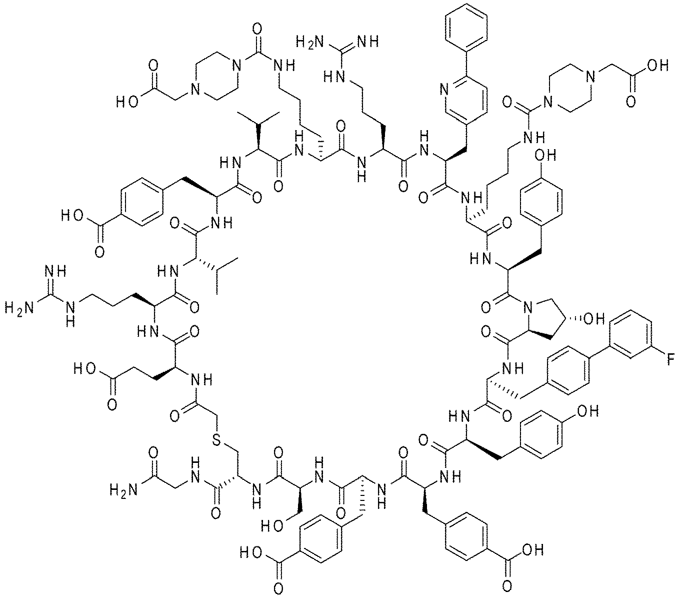

Sieber amide resin was used to start removing the Fmoc group by a general method, to synthesize a peptide as a target. Liberty Prime available from CEM was used as a solid phase synthesizer to perform synthesis according to the manufacturer's manual. For introduction of each residue, the Fmoc-AA/DIC/Oxyma pure (4.2 equivalents/16 equivalents/6 equivalents) relative to 1 equivalent of a resin was used to perform reaction in DMF one time at 105°C for 90 seconds. Note that, for the eighth residue, Fmoc-AA dissolved in NMP was used. The reaction for the second residue and the reaction for the seventh residue were performed two times at 50°C for 15 minutes.

To remove the Fmoc group, a condition, in which reaction with a DMF solution of 4% pyrrolidine was performed at 110°C for one minute, was used.

A chloroacetyl group was introduced by adding a DMF solution of ClAcOH/HATU/DIEA (5 equivalents/5 equivalents/10 equivalents) to the solid phase resin, followed by shaking at room temperature for 30 minutes.

For deprotection of a side chain and cleavage from the solid phase resin, the resin obtained after the step of introducing the chloroacetyl group was washed with DMF and methylene chloride and then diethyl ether, followed by drying under reduced pressure. Then, to a reaction vessel charged with the solid phase resin, a reactant cocktail-A (mixture of TFA/H₂O/TIS/DODT with a volume ratio of 92.5:2.5:2.5:2.5) was added, and was shook at room temperature for 60 minutes. The reaction liquid was filtered and collected with a frit.

When the obtained filtrate was added to a cooled mixture solvent of excess diethyl ether/hexane (1/1), precipitations were formed. This mixture was centrifuged, and the solution was subjected to decantation. The obtained solid was washed with diethyl ether again and was dried under reduced pressure. The obtained solid was used for the subsequent cyclization reaction.

For the cyclization reaction of the peptide, after the peptide was dissolved in MeCN/H₂O (1/1) so that the final concentration of the peptide would be 2 mM based on the number of moles of the solid phase resin, triethylamine (20 equivalents) was added thereto, followed by shaking at room temperature overnight. After acetic acid (30 equivalents) was added to the obtained reaction solution, concentration under reduced pressure was performed using Genevac HT-12.

The obtained crude product was purified using the following conditions (Column: Waters XBridge (registered trademark) C18, 30 × 150 mm; mobile phase: A= 0.1% TFA in H₂O, B = 0.1% TFA in MeCN; temperature: 50°C; gradient (%B conc.): 5 to 23% for 3 minutes, then 23 to 28% for 8 minutes, and then 28 to 60% for one minute; flow rate: 45 mL/minute.

The purity of the target, which was calculated from an area ratio of LC/MS (UV wavelength 225 nm) chromatogram under the following analysis conditions, was 95.52%.

Analysis conditions: retention time = 5.04 minutes; Column: Kinetex EVO C18 2.6 µm 2.1 × 150 mm, 100Å; mobile phase: A = 0.025% TFA in H₂O, B = 0.025% TFA in MeCN; temperature: 60°C; gradient (%B conc):5 to 45% for 7.15 minutes, then 45 to 95% for 0.30 minutes, then 95 to 95% for 1.55 minutes; flow rate: 0.5 mL/min
ESI-MS (+) observation value m/z = 1007.7(M+3H)³⁺.

### [Example 10]

### Synthesis of various peptides

In the present Example, various cyclic peptides were chemically synthesized in the same manner as in Example 1 or 2. The sequences of the synthesized peptides are shown in Table 1, Table 5, Table 6, and Table 7. Note that the peptides synthesized and purified in the same manner as in Example 1 were shown as 1, and the peptides synthesized and purified in the same manner as in Example 2 were shown as 2 in the row of Synthesis in Table 1, Table 6, and Table 7. In addition, in Table 1, the peptides having an amino acid sequence other than SEQ ID NO: 46 have a cyclic structure in which the first amino acid chloroacetylated and the 17th amino acid Cys are bound, and further have a structure in which Gly or bA, and PEG are bound to a Cys terminal thereof. Note that SEQ ID NO: 46 has amino acid sequences set forth in SEQ ID NOs: 47 to 50, but does not have an additional structure such as glycine (G) at C terminal. The peptides in Table 6 are those obtained by substituting an amino acid residue at 5' terminal with a chloroacetyl (ClAc) group. The synthesized peptide was analyzed using basic analysis apparatuses under the basic analysis conditions or the analysis conditions described in Examples 1 to 9, and the structure was confirmed by ESI-MS (+) in mass spectrometry. The obtained ESI-MS (+) observation values (ESI(m/z)) and valences ([M+XH]X+) in that case are shown in Table 1.

### Table 1

**[Table 1-1]**

| SEQ ID No. | Peptide Sequence | | | | | | | | | |
|---|---|---|---|---|---|---|---|---|---|---|
| | 1 | 2 | 3 | 4 | 5 | 6 | 7 | 8 | 9 | 10 |
| 5 | A | R | V | Y | V | D | R | Bph | P | Y |
| 6 | A | R | V | Y | V | K | R | Bph | P | Y |
| 7 | A | R | V | Y | V | R | R | Bph | P | Y |
| 8 | A | R | V | Y | V | G | R | Bph | P | Y |
| 9 | A | R | V | Y | V | S | R | Bph | P | Y |
| 10 | A | R | V | Y | V | H | R | Bph | P | Y |
| 11 | A | R | V | Y | V | D | R | Bph | K | Y |
| 12 | A | R | V | Y | V | D | R | Bph | H | Y |
| 13 | A | R | V | Y | V | D | R | Bph | P | Y |
| 14 | A | R | V | Y | V | D | R | Bph | P | Y |
| 15 | A | R | V | Y | V | D | R | Bph | P | Y |
| 16 | A | R | V | Y | V | D | R | Bph | P | Y |
| 17 | A | R | V | Y | V | D | R | Bph | G | Y |
| 18 | A | R | V | Y | V | D | R | Bph | S | Y |
| 19 | A | R | V | Y | V | D | R | Bph | V | Y |
| 20 | A | R | V | Y | V | D | R | Bph | Y | Y |
| 21 | A | R | V | Y | V | D | R | Bph | P | Y |
| 22 | A | R | V | Y | V | K | R | Bph | KCOpipzaa | Y |
| 23 | A | KCOpipzaa | V | Y | V | K | R | Bph | P | Y |
| 24 | A | KCOpipzaa | V | Y | V | KCOpipzaa | R | Bph | P | Y |
| 25 | A | K | V | Y | V | D | R | Bph | K | Y |
| 26 | A | R | V | 4Py | V | D | R | Bph | P | Y |
| 27 | A | R | V | Y | V | D | R | Bph | P | 4Py |
| 28 | A | R | V | Y | V | D | R | Bph | P | Y |
| 29 | A | R | V | Y | V | D | R | 3Py6Ph | P | Y |
| 30 | A | R | V | Y | V | D | R | Bph | P | Y |
| 31 | A | R | V | Y | V | D | R | 3Py6Ph | P | Y |
| 32 | A | R | V | Y | V | D | R | 3Py6Ph | P | Y |
| 33 | A | R | V | Y | V | D | R | 3Py6Ph | P | Y |
| 34 | A | K | V | Y | V | D | H | Bph | P | Y |
| 35 | A | K | V | Y | V | D | K | Bph | P | Y |
| 36 | A | K | V | Y | V | KCOpipzaa | K | Bph | P | Y |
| 37 | A | KAc | V | Y | V | D | K | Bph | P | Y |
| 38 | A | KCOpipzaa | V | Y | V | D | K | Bph | P | Y |
| 39 | A | Q | V | Y | V | D | K | Bph | P | Y |
| 40 | A | S | V | Y | V | D | K | Bph | P | Y |

**[Table 1-2]**

| SEQ ID No. | Peptide Sequence (Continued) | | | | | | | | | |
|---|---|---|---|---|---|---|---|---|---|---|
| | 11 | 12 | 13 | 14 | 15 | 16 | 17 | | | |
| 5 | P | Bph | Y | F4COO | F4COO | S | C | G | -NH2 | |
| 6 | P | Bph | Y | F4COO | F4COO | S | C | G | -NH2 | |
| 7 | P | Bph | Y | F4COO | F4COO | S | C | G | -NH2 | |
| 8 | P | Bph | Y | F4COO | F4COO | S | C | G | -NH2 | |
| 9 | P | Bph | Y | F4COO | F4COO | S | C | G | -NH2 | |
| 10 | P | Bph | Y | F4COO | F4COO | S | C | G | -NH2 | |
| 11 | P | Bph | Y | F4COO | F4COO | S | C | G | -NH2 | |
| 12 | P | Bph | Y | F4COO | F4COO | S | C | G | -NH2 | |
| 13 | A | Bph | Y | F4COO | F4COO | S | C | G | -NH2 | |
| 14 | P | Bph | Y | F4COO | Y | S | C | G | -NH2 | |
| 15 | P | Bph | Y | F4COO | 4Py | S | C | G | -NH2 | |
| 16 | P | Bph | Y | F4COO | F4COO | K | C | G | -NH2 | |
| 17 | P | Bph | Y | F4COO | F4COO | S | C | G | -NH2 | |
| 18 | P | Bph | Y | F4COO | F4COO | S | C | G | -NH2 | |
| 19 | P | Bph | Y | F4COO | F4COO | S | C | G | -NH2 | |
| 20 | P | Bph | Y | F4COO | F4COO | S | C | G | -NH2 | |
| 21 | V | Bph | Y | F4COO | F4COO | S | C | G | -NH2 | |
| 22 | P | Bph | Y | F4COO | F4COO | S | C | G | -NH2 | |
| 23 | P | Bph | Y | F4COO | F4COO | S | C | G | -NH2 | |
| 24 | P | Bph | Y | F4COO | F4COO | S | C | G | -NH2 | |
| 25 | P | Bph | Y | F4COO | F4COO | S | C | G | -NH2 | |
| 26 | P | Bph | Y | F4COO | F4COO | S | C | G | -NH2 | |
| 27 | P | Bph | Y | F4COO | F4COO | S | C | G | -NH2 | |
| 28 | P | Bph | 4P y | F4COO | F4COO | S | C | G | -NH2 | |
| 29 | P | Bph | Y | F4COO | F4COO | S | C | G | -NH2 | |
| 30 | P | 3Py6Ph | Y | F4COO | F4COO | S | C | G | -NH2 | |
| 31 | P | Bph | 4Py | F4COO | F4COO | S | C | G | -NH2 | |
| 32 | P | 3Py6Ph | Y | F4COO | F4COO | S | C | G | -NH2 | |
| 33 | P | 3Py6Ph | 4P y | F4COO | F4COO | S | C | G | -NH2 | |
| 34 | P | Bph | Y | F4COO | F4COO | S | C | G | -NH2 | |
| 35 | P | Bph | Y | F4COO | F4COO | S | C | G | -NH2 | |
| 36 | P | Bph | Y | F4COO | F4COO | S | C | G | -NH2 | |
| 37 | P | Bph | Y | F4COO | F4COO | S | C | G | -NH2 | |
| 38 | P | Bph | Y | F4COO | F4COO | S | C | G | -NH2 | |
| 39 | P | Bph | Y | F4COO | F4COO | S | C | G | -NH2 | |
| 40 | P | Bph | Y | F4COO | F4COO | S | C | G | -NH2 | |

**[Table 1-3]**

| SEQ ID No. | (Continued) | | | | | |
|---|---|---|---|---|---|---|
| | Ret time | ESI (m/z) | [M+XH]X+ | aLISA BMP4 | aLISA BMP7 | Synthesis |
| 5 | 4.288 | 1257.63 | 2 | A | C | 2 |
| 6 | 3.6 | 1264.26 | 2 | A | C | 2 |
| 7 | 3.62 | 1278.22 | 2 | A | C | 2 |
| 8 | 4.356 | 1228.64 | 2 | A | C | 2 |
| 9 | 4.316 | 1243.68 | 2 | A | C | 2 |
| 10 | 3.652 | 1268.7 | 2 | A | C | 2 |
| 11 | 3.388 | 1273.24 | 2 | A | C | 2 |
| 12 | 3.476 | 1277.67 | 2 | A | C | 2 |
| 13 | 4.188 | 1244.68 | 2 | A | C | 2 |
| 14 | 4.204 | 1243.87 | 2 | B | C | 2 |
| 15 | 3.576 | 824.63 | 3 | A | B | 2 |
| 16 | 3.556 | 852.65 | 3 | A | C | 2 |
| 17 | 3.924 | 1237.81 | 2 | A | C | 2 |
| 18 | 4.024 | 1252.72 | 2 | A | C | 2 |
| 19 | 4.484 | 1258.74 | 2 | A | C | 2 |
| 20 | 4.336 | 1290.71 | 2 | A | C | 2 |
| 21 | 4.376 | 1258.73 | 2 | A | C | 2 |
| 22 | 3.092 | 1364.86 | 2 | A | C | 2 |
| 23 | 3.676 | 1335.32 | 2 | A | C | 2 |
| 24 | 3.952 | 1420.41 | 2 | A | C | 2 |
| 25 | 3.612 | 1259.22 | 2 | A | C | 2 |
| 26 | 3.696 | 1250.19 | 2 | A | C | 2 |
| 27 | 3.272 | 1250.17 | 2 | A | D | 2 |
| 28 | 3.584 | 1250.16 | 2 | A | C | 2 |
| 29 | 3.46 | 1258.19 | 2 | A | ND | 2 |
| 30 | 3.404 | 1258.19 | 2 | A | ND | 2 |
| 31 | 2.924 | 834.15 | 3 | A | ND | 2 |
| 32 | 5.48 | 1258.67 | 2 | A | ND | 2 |
| 33 | 5.064 | 1251.17 | 2 | C | ND | 2 |
| 34 | 4.208 | 1234.15 | 2 | A | C | 2 |
| 35 | 4.096 | 1229.7 | 2 | A | ND | 2 |
| 36 | 3.836 | 1321.29 | 2 | A | ND | 2 |
| 37 | 4.808 | 1250.63 | 2 | A | ND | 2 |
| 38 | 4.396 | 1314.77 | 2 | A | ND | 2 |
| 39 | 4.748 | 1229.63 | 2 | C | ND | 2 |
| 40 | 4.828 | 1209.17 | 2 | D | ND | 2 |

**[Table 1-4]**

| SEQ ID No. | Peptide Sequence | | | | | | | | | |
|---|---|---|---|---|---|---|---|---|---|---|
| | 1 | 2 | 3 | 4 | 5 | 6 | 7 | 8 | 9 | 10 |
| 41 | A | Tic | V | Y | V | D | K | Bph | P | Y |
| 42 | A | K | V | Y | V | D | KAc | Bph | P | Y |
| 43 | A | K | V | Y | V | D | 4Py | Bph | P | Y |
| 44 | A | K | V | Y | V | D | S | Bph | P | Y |
| 45 | A | KCOpipzaa | V | Y | V | KCOpipzaa | K | Bph | P | Y |
| 46 | A | R | V | Y | V | D | R | Bph | P | Y |
| 47 | A | R | V | Y | V | D | R | Bph | P | Y |
| 48 | A | R | V | Y | V | D | R | Bph | P | Y |
| 49 | A | R | V | Y | V | D | R | Bph | P | Y |
| 50 | A | R | V | Y | V | D | R | Bph | P | Y |
| 51 | A | KCOpipzaa | V | Y | V | D | R | Bph | P | Y |
| 52 | A | R | V | Y | V | D | R | Bph | P | Y |
| 53 | E | R | V | Y | V | D | R | Bph | P | Y |
| 54 | Q | R | V | Y | V | D | R | Bph | P | Y |
| 55 | A | Cit | V | Y | V | D | R | Bph | P | Y |
| 56 | A | R | V | F4F | V | D | R | Bph | P | Y |
| 57 | A | R | V | Y | V | D | KCOpipzaa | Bph | P | Y |
| 58 | A | R | V | Y | V | D | Cit | Bph | P | Y |
| 59 | A | KCOpipzaa | V | Y | V | D | R | Bph | P | Y |
| 60 | A | KCOpipzaa | V | Y | V | D | R | Bph | P | Y |
| 61 | A | KCOpipzaa | V | Y | V | D | KCOpipzaa | Bph | P | Y |
| 62 | A | KCOpipzaa | V | Y | V | D | KCOpipzaa | Bph | P | Y |
| 63 | E | KCOpipzaa | V | Y | V | D | R | Bph | P | Y |
| 64 | E | KCOpipzaa | V | Y | V | D | KCOpipzaa | Bph | P | Y |
| 65 | E | KCOpipzaa | V | Y | V | D | R | Bph | P | Y |
| 66 | E | KCOpipzaa | V | Y | V | D | R | Bph | P | Y |
| 67 | E | KCOpipzaa | V | Y | V | D | KCOpipzaa | Bph | P | Y |
| 68 | E | KCOpipzaa | V | Y | V | D | KCOpipzaa | Bph | P | Y |
| 69 | A | K | V | Y | V | D | R | Bph | P | Y |
| 70 | A | R | V | Y | V | D | K | Bph | P | Y |
| 71 | E | R | V | Y | V | D | R | Bph | P | Y |
| 72 | E | R | V | Y | V | D | R | Bph | P | Y |
| 73 | E | KCOpipzaa | V | Y | V | D | R | Bph | P | Y |
| 74 | E | R | V | Y | V | KCOpipzaa | R | Bph | P | Y |
| 75 | E | R | V | Y | V | D | KCOpipzaa | Bph | P | Y |
| 76 | E | KCOpipzaa | V | Y | V | KCOpipzaa | KCOpipzaa | Bph | P | Y |
| 77 | E | R | V | Y | V | D | R | Bph | KCOpipzaa | Y |
| 78 | E | R | V | Y | V | D | R | Bph | KCOpipzaa | Y |

**[Table 1-5]**

| SEQ ID No. | Peptide Sequence(Continued) | | | | | | | | | |
|---|---|---|---|---|---|---|---|---|---|---|
| | 11 | 12 | 13 | 14 | 15 | 16 | 17 | | | |
| 41 | P | Bph | Y | F4COO | F4COO | S | C | G | -NH2 | |
| 42 | P | Bph | Y | F4COO | F4COO | S | C | G | -NH2 | |
| 43 | P | Bph | Y | F4COO | F4COO | S | C | G | -NH2 | |
| 44 | P | Bph | Y | F4COO | F4COO | S | C | G | -NH2 | |
| 45 | P | Bph | Y | F4COO | F4COO | S | C | G | -NH2 | |
| 46 | P | Bph | Y | F4COO | F4COO | S | C | -NH2 | | |
| 47 | P | Bph | Y | F4COO | F4COO | S | C | bA | de | -OH |
| 48 | P | Bph | Y | F4COO | F4COO | S | C | bA | K | -NH2 |
| 49 | P | Bph | Y | F4COO | F4COO | S | C | bA | PEG12Me | |
| 50 | P | Bph | Y | F4COO | F4COO | S | C | bA | K(OCOmPEG10000) | -NH2 |
| 51 | P | Bph | Y | F4COO | F4COO | S | C | G | -NH2 | |
| 52 | P | Bph | F4F | F4COO | F4COO | S | C | G | -NH2 | |
| 53 | P | Bph | Y | F4COO | F4COO | S | C | G | -NH2 | |
| 54 | P | Bph | Y | F4COO | F4COO | S | C | G | -NH2 | |
| 55 | P | Bph | Y | F4COO | F4COO | S | C | G | -NH2 | |
| 56 | P | Bph | Y | F4COO | F4COO | S | C | G | -NH2 | |
| 57 | P | Bph | Y | F4COO | F4COO | S | C | G | -NH2 | |
| 58 | P | Bph | Y | F4COO | F4COO | S | C | G | -NH2 | |
| 59 | P | Bph | F4F | F4COO | F4COO | S | C | G | -NH2 | |
| 60 | P | Bph | 4Py | F4COO | F4COO | S | C | G | -NH2 | |
| 61 | P | Bph | F4F | F4COO | F4COO | S | C | G | -NH2 | |
| 62 | P | Bph | 4Py | F4COO | F4COO | S | C | G | -NH2 | |
| 63 | P | Bph | Y | F4COO | F4COO | S | C | G | -NH2 | |
| 64 | P | Bph | Y | F4COO | F4COO | S | C | G | -NH2 | |
| 65 | P | Bph | F4F | F4COO | F4COO | S | C | G | -NH2 | |
| 66 | P | Bph | 4Py | F4COO | F4COO | S | C | G | -NH2 | |
| 67 | P | Bph | F4F | F4COO | F4COO | S | C | G | -NH2 | |
| 68 | P | Bph | 4Py | F4COO | F4COO | S | C | G | -NH2 | |
| 69 | P | Bph | Y | F4COO | F4COO | S | C | G | -NH2 | |
| 70 | P | Bph | Y | F4COO | F4COO | S | C | G | -NH2 | |
| 71 | P | Bph | F4F | F4COO | F4COO | S | C | G | -NH2 | |
| 72 | P | Bph | F4F | F4COO | 4Py | S | C | G | -NH2 | |
| 73 | P | Bph | F4F | F4COO | 4Py | S | C | G | -NH2 | |
| 74 | P | Bph | F4F | F4COO | 4Py | S | C | G | -NH2 | |
| 75 | P | Bph | F4F | F4COO | 4Py | S | C | G | -NH2 | |
| 76 | P | Bph | F4F | F4COO | 4Py | S | C | G | -NH2 | |
| 77 | P | Bph | F4F | F4COO | F4COO | S | C | G | -NH2 | |
| 78 | P | Bph | F4F | F4COO | 4Py | S | C | G | -NH2 | |

**[Table 1-6]**

| SEQ ID No. | (Continued) | | | | | |
|---|---|---|---|---|---|---|
| | Ret time | ESI (m/z) | [M+XH]X+ | aLISA BMP4 | aLISA BMP7 | Synthesis |
| 41 | 5.296 | 1245.17 | 2 | C | ND | 2 |
| 42 | 4.988 | 1250.72 | 2 | A | ND | 2 |
| 43 | 4.248 | 1239.79 | 2 | C | ND | 2 |
| 44 | 4.928 | 1209.26 | 2 | A | ND | 2 |
| 45 | 4.02 | 1406.33 | 2 | A | ND | 2 |
| 46 | 4.204 | 1229.18 | 2 | A | ND | 2 |
| 47 | 4.172 | 1329.8 | 2 | A | ND | 2 |
| 48 | 3.64 | 886.17 | 3 | C | ND | 2 |
| 49 | 4.652 | 1024.37 | 3 | C | ND | 2 |
| 50 | 3.475 | 681.69 | - | D | B | 2 |
| 51 | 4.42 | 1328.83 | 2 | A | ND | 2 |
| 52 | 4.576 | 1258.73 | 2 | A | ND | 2 |
| 53 | 4.152 | 1286.76 | 2 | A | ND | 2 |
| 54 | 4.124 | 1286.28 | 2 | A | C | 2 |
| 55 | 4.792 | 1258.26 | 2 | A | C | 2 |
| 56 | 4.448 | 1258.74 | 2 | A | ND | 2 |
| 57 | 4.528 | 1328.81 | 2 | C | C | 2 |
| 58 | 4.888 | 1258.22 | 2 | A | C | 2 |
| 59 | 4.76 | 1329.85 | 2 | A | C | 2 |
| 60 | 3.832 | 1321.3 | 2 | A | ND | 2 |
| 61 | 4.984 | 1400.83 | 2 | C | C | 2 |
| 62 | 4.056 | 1392.35 | 2 | D | ND | 2 |
| 63 | 4.34 | 1357.8 | 2 | A | C | 2 |
| 64 | 4.576 | 1428.86 | 2 | C | D | 2 |
| 65 | 4.66 | 1358.78 | 2 | A | C | 2 |
| 66 | 3.764 | 1350.29 | 2 | C | ND | 2 |
| 67 | 4.888 | 1429.89 | 2 | C | C | 2 |
| 68 | 3.984 | 1421.39 | 2 | D | ND | 2 |
| 69 | 4.228 | 1243.73 | 2 | A | C | 2 |
| 70 | 4.224 | 1243.72 | 2 | A | C | 2 |
| 71 | 4.396 | 1287.85 | 2 | A | C | 2 |
| 72 | 3.852 | 844.51 | 3 | A | C | 2 |
| 73 | 4.012 | 891.91 | 3 | A | B | 2 |
| 74 | 3.508 | 905.59 | 3 | A | C | 2 |
| 75 | 4.092 | 891.93 | 3 | C | C | 2 |
| 76 | 3.836 | 1000.36 | 3 | C | C | 2 |
| 77 | 3.82 | 925.95 | 3 | A | C | 2 |
| 78 | 3.388 | 911.6 | 3 | A | C | 2 |

**[Table 1-7]**

| SEQ ID No. | Peptide Sequence | | | | | | | | | |
|---|---|---|---|---|---|---|---|---|---|---|
| | 1 | 2 | 3 | 4 | 5 | 6 | 7 | 8 | 9 | 10 |
| 79 | E | KCOpipzaa | V | Y | V | D | R | Bph | KCOpipzaa | Y |
| 80 | E | KCOpipzaa | V | Y | V | D | R | Bph | KCOpipzaa | Y |
| 81 | E | KCOpipzaa | V | Y | V | D | R | Bph | MeKCOpipzaa | Y |
| 82 | A | KCOpipzaa | V | Y | V | KCOpipzaa | R | Bph | P | Y |
| 83 | E | KCOpipzaa | V | Y | V | KCOpipzaa | R | Bph | P | Y |
| 84 | E | R | V | Y | V | D | R | Bph | P | Y |
| 85 | E | R | V | Y | V | D | R | Bph | S | Y |
| 86 | E | R | V | Y | V | D | R | 3Py6Ph | S | Y |
| 87 | E | R | V | Y | V | D | R | 3Py6Ph | S | Y |
| 88 | E | R | V | Y | V | D | R | Bph | P | Y |
| 89 | E | K | V | Y | V | D | K | Bph | P | Y |
| 90 | E | K | V | Y | V | D | K | Bph | P | Y |
| 91 | E | K | V | Y | V | D | K | Bph | Y | Y |
| 92 | E | K | V | Y | V | D | K | Bph | Y | Y |
| 93 | E | K | V | Y | V | D | R | Bph | P | Y |
| 94 | A | KCOpipzaa | V | Y | V | KCOpipzaa | R | Bph | P | Y |
| 95 | E | R | V | Y | V | D | R | Bph | S | Y |
| 96 | E | R | V | Y | V | D | R | 3Py6Ph | S | Y |
| 97 | E | R | V | Y | V | KCOpipzaa | R | Bph | S | Y |
| 98 | E | R | V | Y | V | KCOpipzaa | R | 3Py6Ph | S | Y |
| 99 | E | KCOpipzaa | V | Y | V | D | R | Bph | S | Y |
| 100 | E | KCOpipzaa | V | Y | V | KCOpipzaa | R | Bph | S | Y |
| 101 | E | KCOpipzaa | V | Y | V | KCOpipzaa | R | 3Py6Ph | S | Y |
| 102 | E | KCOpipzaa | V | Y | V | KCOpipzaa | R | Bph | S | Y |
| 103 | E | R | V | F4COO | V | KCOpipzaa | R | Bph | S | Y |
| 104 | E | R | V | Y | V | D | R | Bph | P | Y |
| 105 | E | R | V | Y | V | D | R | Bph | P | Y |
| 106 | E | R | V | Y | V | D | R | Bph | P | Y |
| 107 | E | R | V | Y | V | D | R | Bph | P | Y |
| 108 | E | R | V | Y | V | D | R | Bph | P | Y |
| 109 | E | R | V | Y | V | D | R | Bph | P | Y |
| 110 | E | R | V | Y | V | D | R | Bph | P | Y |
| 111 | E | R | V | Y | V | D | R | Bph | P | Y |
| 112 | E | R | V | Y | V | D | R | Bph | P | Y |
| 113 | E | R | V | Y | V | D | R | Bph | P | Y |
| 114 | E | KCOpipzaa | V | Y | V | KCOpipzaa | R | Bph | P | Y |
| 115 | E | KCOpipzaa | V | Y | V | KCOpipzaa | R | Boh | P | Y |
| 116 | E | KCOpipzaa | V | Y | V | KCOpipzaa | R | Bph | P | Y |

**[Table 1-8]**

| SEQ ID No. | Peptide Sequence(Continued) | | | | | | | | | |
|---|---|---|---|---|---|---|---|---|---|---|
| | 11 | 12 | 13 | 14 | 15 | 16 | 17 | | | |
| 79 | P | Bph | F4F | F4COO | F4COO | S | C | G | -NH2 | |
| 80 | P | Bph | F4F | F4COO | 4Py | S | C | G | -NH2 | |
| 81 | P | Bph | F4F | F4COO | 4Py | S | C | G | -NH2 | |
| 82 | V | Bph | Y | F4COO | F4COO | S | C | G | -NH2 | |
| 83 | V | Bph | Y | F4COO | F4COO | S | C | G | -NH2 | |
| 84 | V | Bph | Y | F4COO | F4COO | S | C | bA | de | -OH |
| 85 | V | Bph | Y | F4COO | F4COO | S | C | bA | de | -OH |
| 86 | V | Bph | Y | F4COO | F4COO | S | C | bA | de | -OH |
| 87 | V | Bph | Y | F4COO | F4COO | S | C | G | -NH2 | |
| 88 | V | Bph | F4F | F4COO | F4COO | S | C | bA | de | -OH |
| 89 | V | Bph | F4F | F4COO | F4COO | S | C | bA | de | -OH |
| 90 | V | Bph | F4F | F4COO | 4Py | S | C | bA | de | -OH |
| 91 | V | Bph | F4F | F4COO | 4Py | S | C | bA | de | -OH |
| 92 | V | Bph | F4F | F4COO | 4Py | S | C | G | -NH2 | |
| 93 | V | Bph | F4F | F4COO | F4COO | S | C | bA | de | -OH |
| 94 | V | Bph | Y | F4COO | F4COO | S | C | bA | de | -OH |
| 95 | P | Bph | Y | F4COO | F4COO | S | C | bA | de | -OH |
| 96 | P | Bph | Y | F4COO | F4COO | S | C | bA | de | -OH |
| 97 | P | Bph | Y | F4COO | F4COO | S | C | bA | de | -OH |
| 98 | P | Bph | Y | F4COO | F4COO | S | C | bA | de | -OH |
| 99 | P | Bph | Y | F4COO | F4COO | S | C | bA | de | -OH |
| 100 | P | Bph | Y | F4COO | F4COO | S | C | bA | de | -OH |
| 101 | P | Bph | Y | F4COO | F4COO | S | C | bA | de | -OH |
| 102 | V | Bph | Y | F4COO | F4COO | S | C | bA | de | -OH |
| 103 | P | Bph | Y | F4COO | F4COO | S | C | bA | de | -OH |
| 104 | P | Bph | Y | F4COO | F4COO | S | C | bA | de | -OH |
| 105 | Aze | Bph | Y | F4COO | F4COO | S | C | bA | de | -OH |
| 106 | Hpr | Bph | Y | F4COO | F4COO | S | C | bA | de | -OH |
| 107 | Hyp | Bph | Y | F4COO | F4COO | S | C | bA | de | -OH |
| 108 | P4Sh | Bph | Y | F4COO | F4COO | S | C | bA | de | -OH |
| 109 | Pc4F | Bph | Y | F4COO | F4COO | S | C | bA | de | -OH |
| 110 | Pt4F | Bph | Y | F4COO | F4COO | S | C | bA | de | -OH |
| 111 | P4F2 | Bph | Y | F4COO | F4COO | S | C | bA | de | -OH |
| 112 | aMeP | Bph | Y | F4COO | F4COO | S | C | bA | de | -OH |
| 113 | MeV | Bph | Y | F4COO | F4COO | S | C | bA | de | -OH |
| 114 | P | Bph | Y | F4COO | F4COO | S | C | G | -NH2 | |
| 115 | P | Bph | Y | F4COO | F4COO | S | C | G | -OH | |
| 116 | P | Bph | Y | F4COO | F4COO | S | C | bA | -OH | |

**[Table 1-9]**

| SEQ ID No. | (Continued) | | | | | |
|---|---|---|---|---|---|---|
| | Ret time | ESI (m/z) | [M+XH]X+ | aLISA BMP4 | aLISA BMP7 | Synthesis |
| 79 | 3.968 | 973.32 | 3 | C | D | 2 |
| 80 | 3.524 | 958.96 | 3 | A | C | 2 |
| 81 | 3.54 | 963.66 | 3 | A | C | 2 |
| 82 | 4.052 | 947.94 | 3 | C | ND | 2 |
| 83 | 3.924 | 967.28 | 3 | A | ND | 2 |
| 84 | 4.14 | 1359.91 | 2 | A | C | 2 |
| 85 | 3.956 | 1354.87 | 2 | A | C | 2 |
| 86 | 3.232 | 903.94 | 3 | A | C | 2 |
| 87 | 3.148 | 856.05 | 3 | A | ND | 2 |
| 88 | 4.476 | 1360.94 | 2 | A | C | 2 |
| 89 | 4.396 | 1333.08 | 2 | C | B | 2 |
| 90 | 3.98 | 874.74 | 3 | C | B | 2 |
| 91 | 4.028 | 896.71 | 3 | C | C | 2 |
| 92 | 3.984 | 848.73 | 3 | C | C | 2 |
| 93 | 4.428 | 1347.05 | 2 | A | C | 2 |
| 94 | 4.036 | 995.95 | 3 | A | C | 2 |
| 95 | 3.888 | 1353.89 | 2 | A | C | 2 |
| 96 | 3.184 | 903.17 | 3 | A | C | 2 |
| 97 | 3.588 | 963.91 | 3 | A | B | 2 |
| 98 | 2.956 | 964.23 | 3 | A | C | 2 |
| 99 | 4.064 | 1424.96 | 2 | A | C | 2 |
| 100 | 3.776 | 1011.31 | 3 | A | C | 2 |
| 101 | 3.076 | 1011.63 | 3 | A | C | 2 |
| 102 | 3.828 | 1011.95 | 3 | C | C | 2 |
| 103 | 3.532 | 973.27 | 3 | A | A | 2 |
| 104 | 4.028 | 1359.16 | 2 | A | B | 2 |
| 105 | 4.084 | 1351.88 | 2 | C | C | 2 |
| 106 | 4.408 | 1365.91 | 2 | C | C | 2 |
| 107 | 3.86 | 1366.89 | 2 | A | C | 2 |
| 108 | 3.94 | 1366.92 | 2 | A | B | 2 |
| 109 | 4.088 | 1367.83 | 2 | C | C | 2 |
| 110 | 3.992 | 1367.88 | 2 | A | B | 2 |
| 111 | 4.216 | 1376.88 | 2 | A | C | 2 |
| 112 | 4.38 | 1365.92 | 2 | A | B | 2 |
| 113 | 4.3 | 1366.92 | 2 | C | C | 2 |
| 114 | 3.92 | 966.63 | 3 | A | C | 2 |
| 115 | 4.136 | 966.97 | 3 | A | C | 2 |
| 116 | 3.904 | 971.93 | 3 | A | C | 2 |

**[Table 1-10]**

| SEQ ID No. | Peptide Sequence | | | | | | | | | |
|---|---|---|---|---|---|---|---|---|---|---|
| | 1 | 2 | 3 | 4 | 5 | 6 | 7 | 8 | 9 | 10 |
| 117 | E | KCOpipzaa | V | Y | V | KCOpipzaa | R | Bph | P | Y |
| 118 | E | KCOpipzaa | V | Y | V | KCOpipzaa | R | Bph | P | Y |
| 119 | E | KCOpipzaa | V | Y | V | KCOpipzaa | R | 3Py6Ph | S | Y |
| 120 | E | KCOpipzaa | V | Y | V | KCOpipzaa | R | 3Py6Ph | S | Y |
| 121 | E | R | V | F4COO | V | D | R | Bph | P | Y |
| 122 | E | R | V | F4COO | V | KCOpipzaa | R | Bph | P | Y |
| 123 | E | R | V | F4COO | V | KCOpipzaa | R | Bph | P | Y |
| 124 | E | R | V | F4COO | V | KCOpipzaa | R | Bph | S | Y |
| 125 | E | Hgl | V | Y | V | D | R | Bph | P | Y |
| 126 | E | Hph | V | Y | V | D | R | Bph | P | Y |
| 127 | E | Hcit | V | Y | V | D | R | Bph | P | Y |
| 128 | E | R | V | H | V | D | R | Bph | P | Y |
| 129 | E | R | V | W7N | V | D | R | Bph | P | Y |
| 130 | E | R | V | Y | Y | D | R | Bph | P | Y |
| 131 | E | R | V | Y | D | D | R | Bph | P | Y |
| 132 | E | R | V | Y | V | S | R | Bph | P | Y |
| 133 | E | R | V | Y | V | D | 3Py6-NH2 | Bph | P | Y |
| 134 | E | R | V | Y | V | D | F4G | Bph | P | Y |
| 135 | E | R | V | Y | V | D | Hcit | Bph | P | Y |
| 136 | E | R | V | Y | V | D | Atp | Bph | P | Y |
| 137 | E | R | V | Y | V | D | R | Bph | Hcit | Y |
| 138 | E | R | V | Y | V | D | R | Bph | W7N | Y |
| 139 | E | R | V | Y | V | D | R | Bph | D | Y |
| 140 | E | R | V | Y | V | D | R | Bph | P | Hty |
| 141 | E | R | V | Y | V | D | R | Bph | P | Y |
| 142 | E | R | V | Y | V | D | R | Bph | P | Y |
| 143 | E | R | V | Y | V | D | R | Bph | P | Y |
| 144 | E | R | V | Y | V | D | R | Bph | P | Y |
| 145 | E | R | V | Y | V | D | R | Bph | P | Y |
| 146 | E | R | V | Y | V | D | R | Bph | P | Y |
| 147 | E | R | V | Y | V | D | R | Bph | P | Y |
| 148 | E | R | V | Y | V | D | R | Bph | P | Y |
| 149 | E | R | V | Y | V | D | R | Bph | P | Y |
| 150 | E | R | V | Y | V | D | R | Bph | P | Y |
| 151 | E | R | V | Y | V | D | R | Bph | P | Y |
| 152 | E | R | V | Y | V | D | R | Bph | P | Y |
| 153 | E | R | V | Y | V | D | R | Bph | P | Y |
| 154 | E | R | V | Y | V | D | R | Bph | P | Y |

**[Table 1-11]**

| SEQ ID No. | Peptide Sequence(Continued) | | | | | | | | | |
|---|---|---|---|---|---|---|---|---|---|---|
| | 11 | 12 | 13 | 14 | 15 | 16 | 17 | | | |
| 117 | P | Bph | Y | F4COO | F4COO | S | C | bA | de | -OH |
| 118 | P | Bph | Y | F4COO | F4COO | S | C | bA | de | -NH2 |
| 119 | V | Bph | Y | F4COO | F4COO | S | C | G | -NH2 | |
| 120 | P | Bph | Y | F4COO | F4COO | S | C | G | -NH2 | |
| 121 | P | Bph | Y | F4COO | F4COO | S | C | bA | de | -OH |
| 122 | P | Bph | Y | F4COO | F4COO | S | C | bA | de | -OH |
| 123 | V | Bph | Y | F4COO | F4COO | S | C | bA | de | -OH |
| 124 | V | Bph | Y | F4COO | F4COO | S | C | bA | de | -OH |
| 125 | V | Bph | Y | F4COO | F4COO | S | C | G | -NH2 | |
| 126 | V | Bph | Y | F4COO | F4COO | S | C | G | -NH2 | |
| 127 | V | Bph | Y | F4COO | F4COO | S | C | G | -NH2 | |
| 128 | V | Bph | Y | F4COO | F4COO | S | C | G | -NH2 | |
| 129 | V | Bph | Y | F4COO | F4COO | S | C | G | -NH2 | |
| 130 | V | Bph | Y | F4COO | F4COO | S | C | G | -NH2 | |
| 131 | V | Bph | Y | F4COO | F4COO | S | C | G | -NH2 | |
| 132 | V | Bph | Y | F4COO | F4COO | S | C | G | -NH2 | |
| 133 | V | Bph | Y | F4COO | F4COO | S | C | G | -NH2 | |
| 134 | V | Bph | Y | F4COO | F4COO | S | C | G | -NH2 | |
| 135 | V | Bph | Y | F4COO | F4COO | S | C | G | -NH2 | |
| 136 | V | Bph | Y | F4COO | F4COO | S | C | G | -NH2 | |
| 137 | V | Bph | Y | F4COO | F4COO | S | C | G | -NH2 | |
| 138 | V | Bph | Y | F4COO | F4COO | S | C | G | -NH2 | |
| 139 | V | Bph | Y | F4COO | F4COO | S | C | G | -NH2 | |
| 140 | V | Bph | Y | F4COO | F4COO | S | C | G | -NH2 | |
| 141 | F4COO | Bph | Y | F4COO | F4COO | S | C | G | -NH2 | |
| 142 | F3aa | Bph | Y | F4COO | F4COO | S | C | G | -NH2 | |
| 143 | Y | Bph | Y | F4COO | F4COO | S | C | G | -NH2 | |
| 144 | R | Bph | Y | F4COO | F4COO | S | C | G | -NH2 | |
| 145 | Hcit | Bph | Y | F4COO | F4COO | S | C | G | -NH2 | |
| 146 | Atp | Bph | Y | F4COO | F4COO | S | C | G | -NH2 | |
| 147 | Q | Bph | Y | F4COO | F4COO | S | C | G | -NH2 | |
| 148 | V | Bph | 3Pv6-NH2 | F4COO | F4COO | S | C | G | -NH2 | |
| 149 | V | Bph | Y | F4COO | Y | S | C | G | -NH2 | |
| 150 | V | Bph | Y | F4COO | 3Py6-NH2 | S | C | G | -NH2 | |
| 151 | V | Bph | Y | F4COO | W7N | S | C | G | -NH2 | |
| 152 | V | Bph | Y | F4COO | N | S | C | G | -NH2 | |
| 153 | V | Bph | Y | F4COO | F4COO | Hgl | C | G | -NH2 | |
| 154 | V | Bph | Y | F4COO | F4COO | E | C | G | -NH2 | |

**[Table 1-12]**

| SEQ ID No. | (Continued) | | | | | |
|---|---|---|---|---|---|---|
| | Ret time | ESI (m/z) | [M+XH]X+ | aLISA BMP4 | aLISA BMP7 | Synthesis |
| 117 | 3.952 | 1014.68 | 3 | C | C | 2 |
| 118 | 3.872 | 1014.34 | 3 | A | C | 2 |
| 119 | 3.168 | 1445.97 | 2 | C | ND | 2 |
| 120 | 3.056 | 963.62 | 3 | A | ND | 2 |
| 121 | 4.172 | 1372.87 | 2 | A | B | 2 |
| 122 | 3.724 | 976.57 | 3 | A | C | 2 |
| 123 | 3.78 | 977.28 | 3 | A | C | 2 |
| 124 | 3.632 | 973.93 | 3 | A | C | 2 |
| 125 | 1.5 | 1281.8 | 2 | E | E | 1 |
| 126 | 1.6 | 1290.3 | 2 | F | ND | 1 |
| 127 | 1.5 | 1295.9 | 2 | D | F | 1 |
| 128 | 1.4 | 1274.9 | 2 | D | E | 1 |
| 129 | 1.4 | 1300.5 | 2 | D | E | 1 |
| 130 | 1.4 | 1320 | 2 | E | E | 1 |
| 131 | 1.4 | 1296.2 | 2 | D | F | 1 |
| 132 | 1.4 | 1273.9 | 2 | E | ND | 1 |
| 133 | 1.4 | 1291.4 | 2 | E | E | 1 |
| 134 | 1.4 | 1312 | 2 | E | E | 1 |
| 135 | 1.5 | 1295.4 | 2 | E | E | 1 |
| 136 | 1.6 | 1287.4 | 2 | E | E | 1 |
| 137 | 1.4 | 1325.1 | 2 | D | E | 1 |
| 138 | 1.4 | 1332.8 | 2 | E | F | 1 |
| 139 | 1.4 | 1296.9 | 2 | D | E | 1 |
| 140 | 1.4 | 1295.2 | 2 | E | ND | 1 |
| 141 | 1.4 | 1334.6 | 2 | D | E | 1 |
| 142 | 1.4 | 1340.8 | 2 | D | E | 1 |
| 143 | 1.4 | 1320 | 2 | D | E | 1 |
| 144 | 1.3 | 1316.8 | 2 | D | E | 1 |
| 145 | 1.4 | 1324.1 | 2 | D | E | 1 |
| 146 | 1.4 | 1316.5 | 2 | D | E | 1 |
| 147 | 1.4 | 1302.4 | 2 | D | E | 1 |
| 148 | 1.3 | 1288.2 | 2 | D | E | 1 |
| 149 | 1.4 | 1273.9 | 2 | E | E | 1 |
| 150 | 1.3 | 1273.7 | 2 | E | E | 1 |
| 151 | 1.4 | 1286 | 2 | E | E | 1 |
| 152 | 1.4 | 1249.3 | 2 | F | E | 1 |
| 153 | 1.4 | 1315.8 | 2 | D | E | 1 |
| 154 | 1.4 | 1309.2 | 2 | D | E | 1 |

**[Table 1-13]**

| SEQ ID No. | Peptide Sequence | | | | | | | | | |
|---|---|---|---|---|---|---|---|---|---|---|
| | 1 | 2 | 3 | 4 | 5 | 6 | 7 | 8 | 9 | 10 |
| 155 | E | R | V | Y | V | D | R | Bph | P | Y |
| 156 | E | R | V | Y | V | D | R | Bph | P | Y |
| 157 | E | R | V | Y | V | D | R | Bph | P | Y |
| 158 | E | R | V | Y | V | D | R | Bph | P | Y |
| 159 | E | R | V | Y | V | D | R | Bph | P | Y |
| 160 | E | R | V | Y | Atp | D | R | Bph | P | Y |
| 161 | E | R | V | Y | Atp | D | R | Bph | S | Y |
| 162 | E | R | V | Y | V | D | R | Bph | S | Y |
| 163 | E | R | V | Y | V | D | R | Bph | S | Y |
| 164 | E | R | V | Y | V | D | R | Bph | S | Y |
| 165 | E | R | V | Y | Atp | D | R | Bph | S | Y |
| 166 | E | R | V | Y | Atp | D | R | Bph | S | Y |
| 167 | E | R | V | Y | Atp | D | R | Bph | S | Y |
| 168 | E | Hcit | V | Y | V | D | R | Bph | S | Y |
| 169 | E | Hcit | V | Y | Atp | D | R | Bph | S | Y |
| 170 | E | R | V | Y | Atp | KCOpipzaa | R | Bph | S | Y |
| 171 | E | R | V | Y | Atp | KCOpipzaa | R | Bph | S | Y |
| 172 | A | R | V | Y3COO | V | D | R | Bph | P | Y |
| 173 | A | R | V | Y3CONmm | V | D | R | Bph | P | Y |
| 174 | A | R | V | F4COO | V | D | R | Bph | P | Y |
| 175 | E | R | V | F4COO | Atp | D | R | Bph | P | Y |
| 176 | E | R | V | F4COO | Atp | D | R | Bph | P | Y |
| 177 | E | R | V | F4COO | Atp | D | R | Bph | P | Y |
| 178 | E | R | V | F4COO | Atp | D | R | Bph | P | Y |
| 179 | E | R | V | F4COO | Atp | KCOpipzaa | R | Bph | P | Y |
| 180 | E | R | V | F4COO | Atp | KCOpipzaa | R | Bph | P | Y |
| 181 | E | R | V | F4COO | Atp | KCOpipzaa | R | Bph | P | Y |
| 182 | E | R | V | Y | Atp | KCOpipzaa | R | Bph | P | Y |
| 183 | E | R | V | Y | Atp | KCOpipzaa | R | Bph | P | Y |
| 184 | E | R | V | Y | Atp | KCOpipzaa | R | Bph | P | Y |
| 185 | E | KCOpipzaa | V | F4COO | Atp | D | R | Bph | P | Y |
| 186 | E | KCOpipzaa | V | F4COO | Atp | D | R | Bph | P | Y |
| 187 | E | KCOpipzaa | V | F4COO | Atp | D | R | Bph | P | Y |

**[Table 1-14]**

| SEQ ID No. | Peptide Sequence(Continued) | | | | | | | | | |
|---|---|---|---|---|---|---|---|---|---|---|
| | 11 | 12 | 13 | 14 | 15 | 16 | 17 | | | |
| 155 | V | Bph | Y | F4COO | F4COO | V | C | G | -NH2 | |
| 156 | V | Bph | Y | F4COO | F4COO | Aib | C | G | -NH2 | |
| 157 | V | Bph | Y | F4COO | F4COO | Hph | C | G | -NH2 | |
| 158 | V | Bph | Y | F4COO | F4COO | H | C | G | -NH2 | |
| 159 | V | Bph | Y | F4COO | F4COO | Hcit | C | G | -NH2 | |
| 160 | V | Bph | Y | F4COO | F4COO | S | C | bA | de | -OH |
| 161 | V | Bph | Y | F4COO | F4COO | S | C | bA | de | -OH |
| 162 | F3aa | Bph | Y | F4COO | F4COO | S | C | bA | de | -OH |
| 163 | Y | Bph | Y | F4COO | F4COO | S | C | bA | de | -OH |
| 164 | V | Bph | Y | F4COO | F4COO | H | C | bA | de | -OH |
| 165 | F3aa | Bph | Y | F4COO | F4COO | S | C | bA | de | -OH |
| 166 | Y | Bph | Y | F4COO | F4COO | S | C | bA | de | -OH |
| 167 | V | Bph | Y | F4COO | F4COO | H | C | bA | de | -OH |
| 168 | V | Bph | Y | F4COO | F4COO | H | C | bA | de | -OH |
| 169 | V | Bph | Y | F4COO | F4COO | H | C | bA | de | -OH |
| 170 | P | Bph | Y | F4COO | F4COO | S | C | bA | de | -OH |
| 171 | V | Bph | Y | F4COO | F4COO | S | C | bA | de | -OH |
| 172 | P | Bph | Y | F4COO | F4COO | S | C | G | -NH2 | |
| 173 | P | Bph | Y | F4COO | F4COO | S | C | G | -NH2 | |
| 174 | P | Bph | Y | F4COO | F4COO | S | C | G | -NH2 | |
| 175 | F3aa | Bph | Y | F4COO | F4COO | S | C | bA | de | -OH |
| 176 | P4Sh | Bph | Y | F4COO | F4COO | S | C | bA | de | -OH |
| 177 | P | Bph | Y | F4COO | F4COO | S | C | bA | de | -OH |
| 178 | V | Bph | Y | F4COO | F4COO | S | C | bA | de | -OH |
| 179 | F3aa | Bph | Y | F4COO | F4COO | S | C | bA | de | -OH |
| 180 | P4Sh | Bph | Y | F4COO | F4COO | S | C | bA | de | -OH |
| 181 | P | Bph | Y | F4COO | F4COO | S | C | bA | de | -OH |
| 182 | F3aa | Bph | Y | F4COO | F4COO | S | C | bA | de | -OH |
| 183 | P4Sh | Bph | Y | F4COO | F4COO | S | C | bA | de | -OH |
| 184 | P | Bph | Y | F4COO | F4COO | S | C | bA | de | -OH |
| 185 | F3aa | Bph | F4F | F4COO | 4Pv | S | C | bA | de | -OH |
| 186 | P4Sh | Bph | F4F | F4COO | 4Pv | S | C | bA | de | -OH |
| 187 | P | Bph | F4F | F4COO | 4Py | S | C | bA | de | -OH |

**[Table 1-15]**

| SEQ ID No. | (Continued) | | | | | |
|---|---|---|---|---|---|---|
| | Ret time | ESI (m/z) | [M+XH]X+ | aLISA BMP4 | aLISA BMP7 | Synthesis |
| 155 | 1.5 | 1294.2 | 2 | D | E | 1 |
| 156 | 1.5 | 1287.1 | 2 | E | E | 1 |
| 157 | 1.5 | 1325.2 | 2 | E | F | 1 |
| 158 | 1.3 | 1313.4 | 2 | D | F | 1 |
| 159 | 1.4 | 1329.9 | 2 | D | E | 1 |
| 160 | 4.264 | 1387.96 | 2 | A | B | 2 |
| 161 | 3.948 | 1382.88 | 2 | A | B | 2 |
| 162 | 3.988 | 1407.9 | 2 | A | B | 2 |
| 163 | 3.908 | 1386.9 | 2 | A | B | 2 |
| 164 | 3.396 | 920.26 | 3 | A | C | 2 |
| 165 | 4.192 | 1435.98 | 2 | A | B | 2 |
| 166 | 4.104 | 1415 | 2 | A | B | 2 |
| 167 | 3.536 | 939.01 | 3 | A | C | 2 |
| 168 | 4.008 | 925.35 | 3 | A | C | 2 |
| 169 | 3.624 | 944.26 | 3 | C | C | 2 |
| 170 | 3.42 | 982.96 | 3 | A | B | 2 |
| 171 | 3.528 | 983.65 | 3 | A | B | 2 |
| 172 | 4.256 | 1279.73 | 2 | C | C | 2 |
| 173 | 4.524 | 1286.35 | 2 | A | C | 2 |
| 174 | 4.092 | 1271.77 | 2 | A | C | 2 |
| 175 | 4.516 | 970.27 | 3 | A | B | 2 |
| 176 | 3.848 | 1408.84 | 2 | A | B | 2 |
| 177 | 4.488 | 1400.91 | 2 | A | B | 2 |
| 178 | 4.612 | 1401.85 | 2 | A | B | 2 |
| 179 | 4.156 | 1031.35 | 3 | C | B | 2 |
| 180 | 3.94 | 1000.66 | 3 | A | B | 2 |
| 181 | 3.708 | 995.24 | 3 | A | B | 2 |
| 182 | 3.784 | 1021.91 | 3 | A | B | 2 |
| 183 | 3.452 | 991.68 | 3 | A | B | 2 |
| 184 | 3.664 | 986.36 | 3 | A | B | 2 |
| 185 | 4.052 | 1004.29 | 3 | C | B | 2 |
| 186 | 3.828 | 973.2 | 3 | A | B | 2 |
| 187 | 4.432 | 967.96 | 3 | C | B | 2 |

### [Example 11]

### Evaluation test of BMP4 signal transduction inhibitory activity

In the present Example, a BMP4 signal transduction inhibitory activity of each peptide consisting of an amino acid sequence set forth in any of SEQ ID NOs: 5 to 240 and 243 to 421 synthesized in Example 10 was verified by phosphorylation of SMAD1 caused by BMP4 stimulus.

A549 cells were cultured in MEM (Thermo Fisher Scientific) containing 10% FBS (Thermo Fisher Scientific), 1 × Non-essential amino acids (Thermo Fisher Scientific), and 50 µg/mL Gentamicin. After TrypLE (Gibco) was used to peel the cells, seeding was performed in a 96-well plate for adhesion cells so that the number of cells would be 20,000 per well, followed by culturing for 48 hours.

After the cells were cultured as described above, the culture medium was replaced with a peptide solution or recombinant Human Noggin Protein (R&D) solution as a positive control diluted to various concentrations in a nutrition-starved culture medium containing 0.1% BSA (Sigma) instead of FBS Thirty minutes after culturing, 0.2 mM (final concentration) of BMP4 was added thereto, to stimulate it for 60 minutes. The culture medium was removed and the cells were lysed with Lysis Buffer provided with AlphaLISA (registered trademark) SureFire (registered trademark) Ultra SMAD1 (p-ser463/465) Assay kit (PerkinElmer). According to the protocol of the kit, a signal was detected using SpectraMax (registered trademark) Paradigm (registered trademark) multimode microplate reader (Molecular Devices). The obtained signal was analysed using GraphPad Prism (GraphPad Software). The % inhibition was calculated by considering non-stimulation as 100% inhibition and BMP stimulation only as 0% inhibition.

Regarding SEQ ID NOs: 5 to 187, the concentrations of the peptides synthesized in the same manner as in Example 2 (shown as 2 in the row of Synthesis) were 0.02 nM, 0.2 nM, and 2 nM, and the concentrations of the peptides synthesized in the same manner as in Example 1 (shown as 1 in the row of Synthesis) were 0.2 nM, 2 nM, and 20 nM.

The evaluation results were based on the results of evaluation using the peptides synthesized in the same manner as in Example 2. Those that showed an inhibitory activity of 50% or more in a zone to which the peptide was added to in an amount of 0.2 nM were defined as A, those that showed an inhibitory activity of less than 50% in 0.2 nM-addition zone and showed an inhibitory activity of 50% or more in 2 nM-addition zone were defined as B or C, and those that showed inhibitory activity of less than 50% in 2 nM-addition zone were defined as D.

In addition, regarding the results evaluated using the peptides synthesized in the same manner as in Example 1, those that showed 50% or more of inhibitory activity in 0.2 nM-addition zone was defined as D, those that showed less than 50% of inhibitory activity in 0.2 nM-addition zone and showed 50% or more of inhibitory activity in 2 nM-addition zone were defined as E, and those that showed less than 50% of inhibitory activity in 2 nM-addition zone and showed 50% or more of inhibitory activity in 20 nM-addition zone were defined as F. The evaluation results of the respective peptides are shown in Table 1.

Regarding SEQ ID NOs: 243 to 321, the test was performed at the concentrations described in Table 6. Regarding the evaluation results, those that showed 30% or more of inhibitory activity in a zone to which the peptide was added in an amount of 0.02 nM were defined as A, those that showed less than 30% of inhibitory activity in 0.02 nM-addition zone and showed 30% or more of inhibitory activity in 0.2 nM-addition zone were defined as B, and those that showed less than 30% of inhibitory activity in 0.2 nM-addition zone and showed 30% or more of inhibitory activity in 2 nM-addition zone were defined as C.

The evaluation results of the respective peptides are shown in Table 6.

Regarding SEQ ID NOs: 322 to 421, the test was performed at the concentrations described in Table 7. First, regarding the evaluation results on BMP 4, those that showed less than 30% of inhibitory activity in 2 nM-peptide-addition zone and showed 30% or more of inhibitory activity in 20 nM-addition zone were defined as A, and those that showed less than 30% of inhibitory activity in 20 nM-addition zone and showed 30% or more of inhibitory activity in in 200 nM-addition zone were defined as B. The evaluation results of the respective peptides are shown in Table 7

Note that ND in the results shows that the inhibitory activity was less than 30% at the maximum concentration evaluated.

According to the present results, it was shown that the peptide of the present invention has a BMP4 signal transduction inhibitory activity.

### [Example 12]

### Evaluation test of BMP7 signal transduction inhibitory activity

In the present Example, BMP7 signal transduction inhibitory activity of each peptide consisting of an amino acid sequence set forth in any of SEQ ID NOs: 5 to 240 and 243 to 421 synthesized in Example 10 was verified by phosphorylation of SMAD 1 caused by BMP7 stimulus.

The evaluation was performed using 10 nM of BMP7 instead of BMP 4.

Regarding SEQ ID NOs: 5 to 240, the concentrations of the peptides synthesized in the same manner as in Example 2 (shown as 2 in the row of Synthesis) were 1 nM, 10 nM, and 100nM, and the concentrations of the peptides synthesized in the same manner as in Example 1 (shown as 1 in the row of Synthesis) were 10 nM, 100 nM, and 1,000 nM, similarly with Example 11.

The peptides synthesized in the same manner as in Example 2 among SEQ ID NOs: 5 to 240 were verified in the same manner as in the verification of Example 11 using A549 cells, except that the peptides were added at molar concentrations of 1 nM, 10 nM, and 100 nM, respectively. Regarding the evaluation results, those that showed 50% or more of inhibitory activity in a zone to which the peptide was added in an amount of 1 nM were defined as A, those that showed less than 50% of inhibitory activity in 1 nM-addition zone and showed 50% or more of inhibitory activity in 10 nM-addition zone were defined as B, those that showed less than 50% of inhibitory activity in 10 nM-addition zone and showed 50% or more of inhibitory activity in 100 nM-addition zone were defined as C, and those that showed less than 50% of inhibitory activity in 100 nM-addition zone were defined as D.

Regarding the results obtained by evaluating the peptide synthesized in the same manner as in Example 1, the evaluation was performed in the same manner except that the peptides were added at molar concentrations of 10 nM, 100 nM, and 1,000 nM, respectively. Regarding the evaluation results, those that showed 50% or more of inhibitory activity in a zone to which the peptide was added in an amount of 10 nM were defined as E, those that showed less than 50% of inhibitory activity in 10 nM-addition zone and showed 50% or more of inhibitory activity in 100 nM-addition zone were defined as F, those that showed less than 50% of inhibitory activity in 100 nM-addition zone and showed 50% or more of inhibitory activity in 1,000 nM-addition zone were defined as G, and those that showed 30% or more and less than 50% of inhibitory activity in 1,000 nM-addition zone were defined as H. The evaluation results of the respective peptides are shown in Table 1.

Note that ND in the results shows that the inhibitory activity was less than 30% at the maximum concentration evaluated.

Regarding SEQ ID NOs: 243 to 273 and SEQ ID NOs: 299 to 321, the test was performed at the concentrations (10, 100, 1,000 nM) described in Table 6. Regarding the evaluation results, those that showed less than 30% of inhibitory activity in a zone to which the peptide was added in an amount of 10 nM and showed 30% or more of inhibitory activity in 100 nM-addition zone were defined as D, and those that showed less than 30% of inhibitory activity in 100 nM-addition zone and showed 30% or more of inhibitory activity in 1,000 nM-addition zone were defined as E.

Regarding SEQ ID NOs: 274 to 298, the test was performed at the concentrations (100, 300, 1,000 nM) described in Table 6.

Those that showed 30% or more of inhibitory activity in 100 nM-addition zone was defined as F, those that showed less than 30% of inhibitory activity in 100 nM-addition zone and showed 30% or more of inhibitory activity in 300 nM-addition zone were defined as G, and those that showed less than 30% of inhibitory activity in 300 nM-addition zone and showed 30% or more of inhibitory activity in 1,000 nM-addition zone were defined as H. The evaluation results of the respective peptides are shown in Table 6.

Note that ND in the results shows that the inhibitory activity was less than 30% at the maximum concentration evaluated.

Regarding SEQ ID NOs: 322 to 370 and SEQ ID NOs: 393 to 421, the test was performed at the concentrations (1, 10, 100 nM). Regarding the evaluation results, those that showed less than 30% of inhibitory activity in a zone to which the peptide was added in an amount of 1 nM and showed 30% or more of inhibitory activity in 10 nM-addition zone were defined as C, and those that showed less than 30% of inhibitory activity in 10 nM-addition zone and showed 30% or more of inhibitory activity in 100 nM-addition zone were defined as D.

Regarding SEQ ID NOs: 371 to 391, the test was performed at the concentrations (10, 30, 90 nM). Regarding the evaluation results, those that showed 30% or more of inhibitory activity in 10 nM-addition zone were defined as E, those that showed less than 30% of inhibitory activity in 10 nM-addition zone and showed 30% or more of inhibitory activity in 30 nM-addition zone were defined as F, and those that showed less than 30% of inhibitory activity in 30 nM-addition zone and showed 30% or more of inhibitory activity in 90 nM-addition zone were defined as G.

The evaluation results of the respective peptides are shown in Table 7.

The results showed that the peptide of the present invention includes those that have signal transduction inhibitory activities of both BMP4 and BMP7 or those that have BMP7 signal transduction inhibitory activity.

### [Example 13]

### IC50 value against BMP4 and BMP7

In the present Example, the respective signal transduction inhibition peptides of BMP4 and BMP7 synthesized in Examples 1 to 10 were used to calculate signal inhibitory activities (IC50) of BMP4 and BMP7.

The evaluation tests of the signal transduction inhibitory activities of SEQ ID NOs: 87, 177, and 181 are as follows. Specifically, the inhibitory activity of BMP4 was evaluated in the same manner as in Example 11 that used A549 cells. Eight concentrations of the peptides were used at a common ratio of 1/3 starting from 2 nM. The inhibitory activity of BMP7 was evaluated in the same manner as in Example 12. Eight concentrations of the peptides were used at a common ratio of 1/3 starting from 100 nM. Note that recombinant Human Noggin Protein (R&D) as a positive control was used at the same concentration as that of the peptide.

In addition, regarding SEQ ID NO: 277 (Example 8), the inhibitory activity of BMP4 was evaluated using eight concentrations of the peptides at a common ratio of 1/3 starting from 20 nM in the same manner as in Example 11. The inhibitory activity of BMP7 was evaluated using eight concentrations of the peptides at a common ratio of 1/3 starting from 1,000 nM in the same manner as in Example 12.

In addition, regarding SEQ ID NO: 417 (Example 6), the inhibitory activity of BMP4 was evaluated using eight concentrations of the peptides at a common ratio of 1/4 starting from 300 nM in the same manner as in Example 11. The inhibitory activity of BMP7 was evaluated using eight concentrations of the peptides at a common ratio of 1/4 starting from 300 nM in the same manner as in Example 12.

The results are shown in FIG. 1, Table 2-1, and Table 2-2.

Note that ND in the results shows that IC50 was not calculated in the evaluation systems.

**[Table 2-1]**

| Table 2 | | |
|---|---|---|
| Peptide SEQ ID No. | IC50 (nM) | |
| | BMP4 | BMP7 |
| 87 | 0.08 | ND |
| 177 | 0.053 | 1.4 |
| 181 | 0.052 | 1.6 |
| Noggin | 0.08 | 2.7 |

### [Table 2-2]

**Table 2-2**

| Peptide SEQ ID No. | IC50(nM) | |
|---|---|---|
| | BMP4 | BMP7 |
| 277 | 0.073 | 455 |
| 417 | ND | 15.6 |
| Noggin | 0.13 | 5.74 |

From the results of Table 2-1, it was shown that the BMP4 signal transduction inhibition peptides No. 87, No. 177, No. 181 have the same level of the BMP4 inhibitory activity as Noggin. In addition, it was shown that the BMP4 signal transduction inhibition peptides No. 177 and No. 181 have the same level of the BMP7 inhibitory activity as Noggin. Note that it was shown that No. 87 does not inhibit signal transduction of BMP7.

From the results of Table 2-2, it was shown that the BMP4 signal transduction inhibition peptide SEQ ID NO: 277 (Example 8) has the same level of the BMP4 inhibitory activity as Noggin. It was shown that the BMP7 signal transduction inhibition peptide SEQ ID NO: 417 (Example 6) has the same level of the BMP7 inhibitory activity as Noggin and does not inhibit signal transduction of BMP 4.

### [Example 14]

### Evaluation test of other BMP family signal transduction inhibitory activities

In the present Example, BMP4 signal transduction inhibition peptide No. 177, peptide No.29986 (SEQ ID NO: 277 (Example 8)), and peptide SEQ ID NO: 417 (Example 6) synthesized in Example 2, Example 8, and Example 6 were verified in the same manner as in Example 13 in order to confirm presence or absence of signal transduction inhibitory activities obtained by BMPs 2, 5, 6, 9, 12, and 14 belonging to the respective BMP families.

Regarding the concentrations of the respective BMPs in the evaluation, 0.3 nM of BMP2, 2 nM of BMP5, 3 nM of BMP6, 10 nM of BMP9 and BMP12, and 0.6 nM of BMP14 were added, followed by application of stimulus.

Regarding No. 177, the concentrations of the peptides were as follows. Specifically, eight concentrations of the peptides were used at a common ratio of 1/3 starting from 10 nM (BMP2), starting from 200 nM (BMP5), starting from 100 nM (BMP6), starting from 1,000 nM (BMP9 and BMP12), and starting from 60 nM (BMP14). The evaluation was performed in the same manner as in Example 11 except for the above points. Note that recombinant Human Noggin Protein (R&D) as a positive control was used at the same concentration as the peptide.

Regarding the peptide SEQ ID NO: 277 (Example 8), the concentrations of the peptides were as follows. Specifically, eight concentrations of the peptides were used at a common ratio of 1/3 starting from 30 nM (BMP2), starting from 200 nM (BMP5), starting from 300 nM (BMP6), starting from 1,000 nM (BMP12), and starting from 60 nM (BMP14).

Regarding the peptide SEQ ID NO: 417 (Example 6), the concentrations of the peptides were as follows. Specifically, eight concentrations of the peptides were used at a common ratio of 1/3 starting from 300 nM (BMP2), starting from 200 nM (BMP5), starting from 300 nM (BMP6), starting from 1,000 nM (BMP12), and starting from 60 nM (BMP14).

The results obtained by calculating IC50 of each BMP family from the obtained measurement values are described in Table 3.

Note that ND in the results shows that IC50 was not calculated in the evaluation systems, and NT in the results shows that the test was not performed.

The inhibition curve of the BMP4 signal transduction inhibition peptide SEQ ID NO: No. 177 was described in FIG. 2. The triangle in the figure indicates the BMP4 signal transduction inhibition peptide No. 177 and the square indicates Noggin. From the results, it was showed that the BMP4 signal transduction inhibition peptide No. 177 has not only the signal transduction inhibitory activities of BMP4 and BMP7 but also the signal transduction inhibitory activities of BMP2, BMP5, and BMP6.

**[Table 3]**

| Table 3 | | | | | | | | | |
|---|---|---|---|---|---|---|---|---|---|
| Seq. ID. No. | Ligand | BMP2 | BMP4 | BMP5 | BMP6 | BMP7 | BMP9 | BMP12 | BMP14 |
| | Conc. | 0.3 nM | 0.2 nM | 2nM | 3nM | 10 nM | 10 nM | 10 nM | 0.6 nM |
| 177 | | 0.21 nM | 0.053 nM | 9.17 nM | 3.9 nM | 1.43 nM | ND | ND | ND |
| 277 | | 0.45 nM | 0.073 nM | ND | ND | 455 nM | NT | ND | ND |
| 417 | | ND | ND | 19.3 nM | 28.7 nM | 15.6 nM | NT | ND | ND |

### [Example 15]

Evaluation test for intermolecular interaction between BMP4/BMP7 and peptide by surface plasmon resonance (SPR)

The test of the intermolecular interaction by the surface plasmon resonance (SPR) of each of the peptides synthesized in Examples 2 and 3 against BMP4/BMP7 was performed by the following manner. The test method is specifically shown as follows.

### [SPR measurement]

A CM5 sensor chip (Cytiva) was inserted into a BiacoreT200 (Cytiva). Three priming runs were performed using running buffer: HBS-EP+(Cytiva). The system was then equilibrated at a flow rate of 30 µL/min. Subsequently, 50 µL of 60 mM EDC solution (Cytiva) and 50 µL of 650 mM NHS solution (Cytiva) were mixed and were allowed to react at a flow rate of 10 µL/min for 420 seconds. The resultant was diluted with a 10 mM acetic acid solution (pH 5.0), to prepare 150 µL of 0.2 µM BMP4/BMP7 solution. The solution was allowed to react at a flow rate of 10 µL/min for 420 seconds, and the BMP4/BMP7 was immobilized in a CM5 sensor chip. After immobilization, capping was performed by reacting with 1.0 M ethanolamine aqueous solution (Cytiva) at a flow rate of 10 µL/min for 420 seconds. A liquid prepared by dissolving each peptide at 10 mM in a DMSO solution was diluted with a running buffer so that the final concentration of the peptide-dissolved liquid would be 10 µM. Then, the peptide solutions (100 nM, 50 nM, 25 nM, 10 nM, and 5 nM) were prepared. Using the above samples, the kinetics of each peptide against BMP4/BMP7 was obtained by SPR measurement.

The kinetics evaluation model was Single Cycle Kinetics, and curve fitting was performed using Biacore T200 Evaluation Software Version 3.0 (Cytiva). The binding of peptides to BMP4/BMP7 was evaluated by performing least-squares curve fitting on the obtained sensorgram to calculate the KD value. The obtained results are shown in Table 4.

### [Table 4]

**Table 4**

| Peptide SEQ ID No. | BMP4 (nM) | BMP7 (nM) |
|---|---|---|
| 84 | 2.1 | 21.7 |
| 177 | 0.4 | 2.3 |

### [Example 16]

### Synthesis of various peptides

The peptides prepared in Example 10 are shown in Table 5, Table 6, and Table 7.

### Table 5

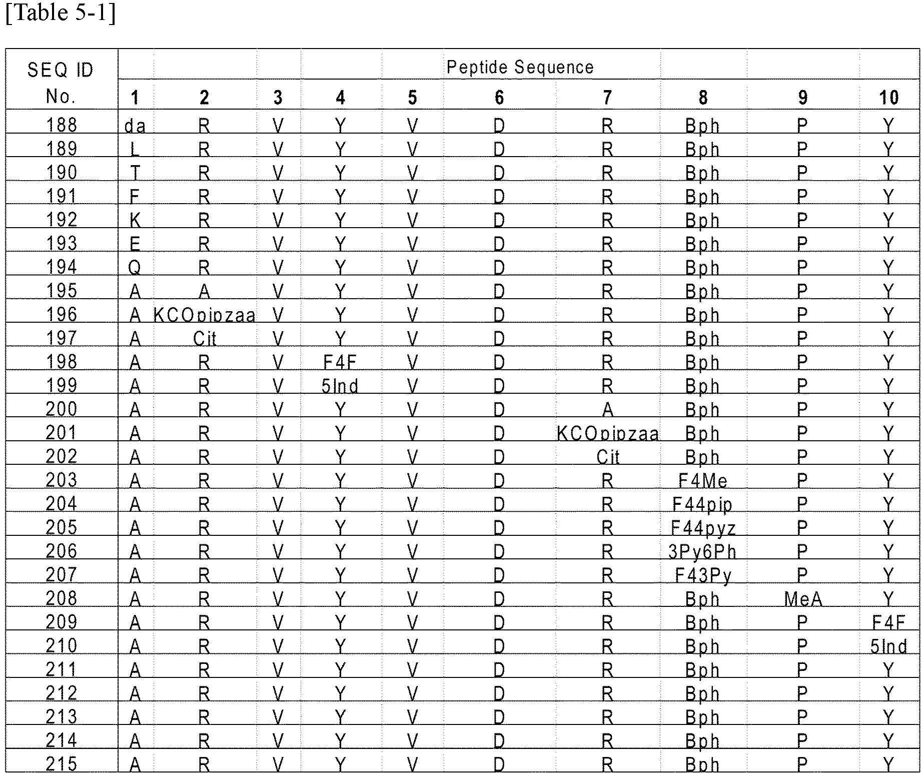

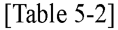

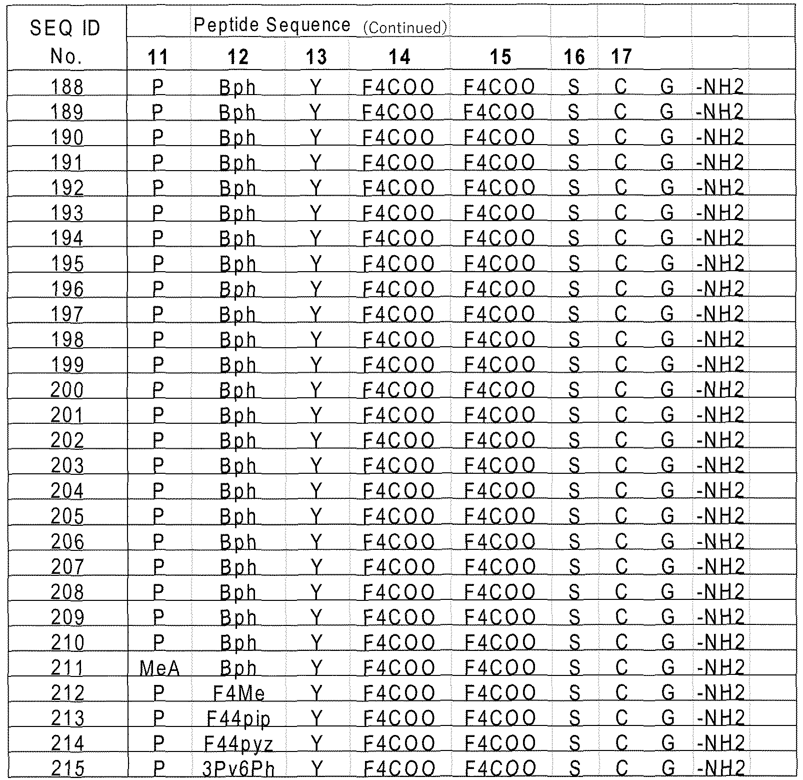

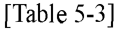

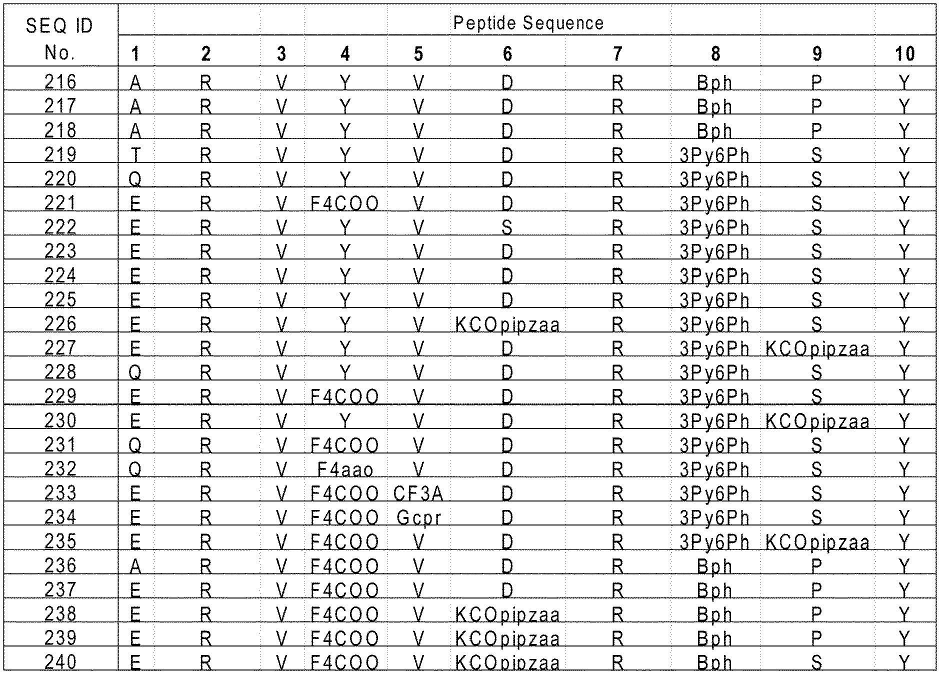

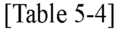

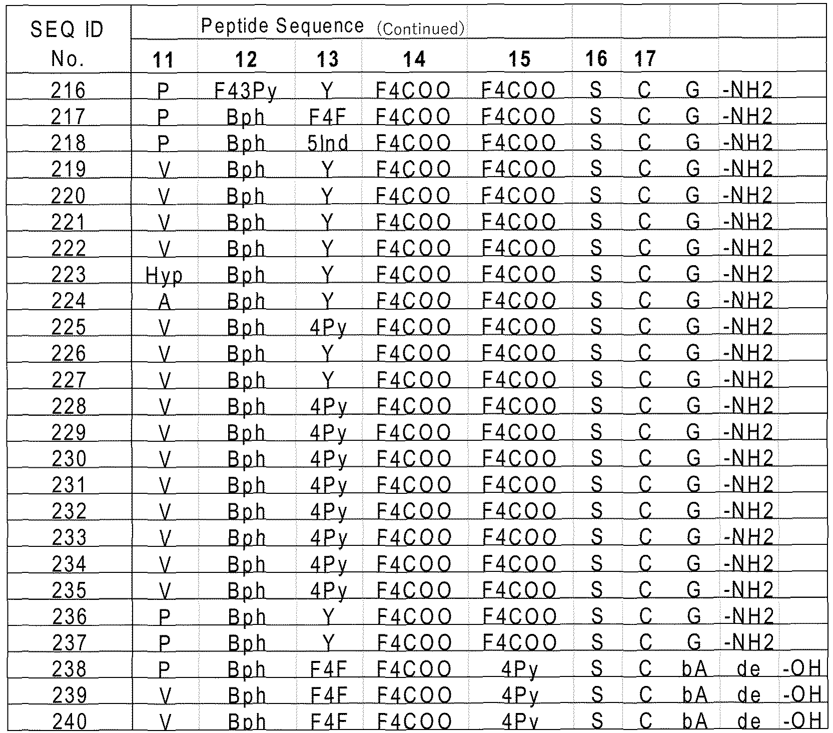

### Table 6

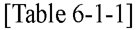

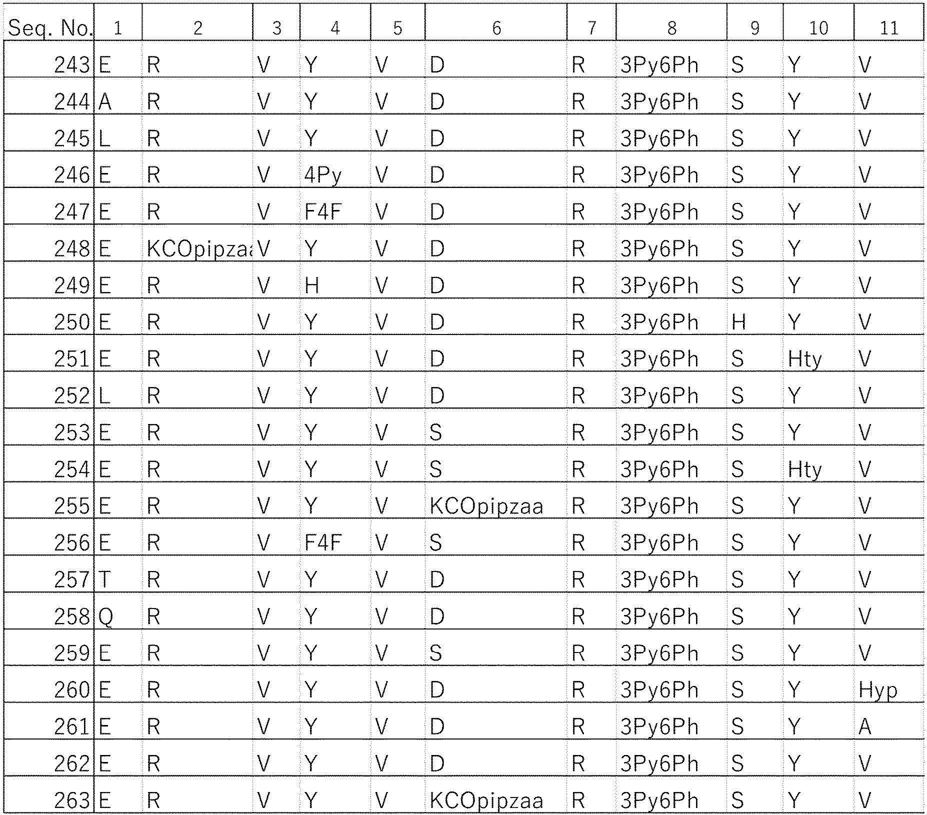

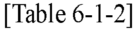

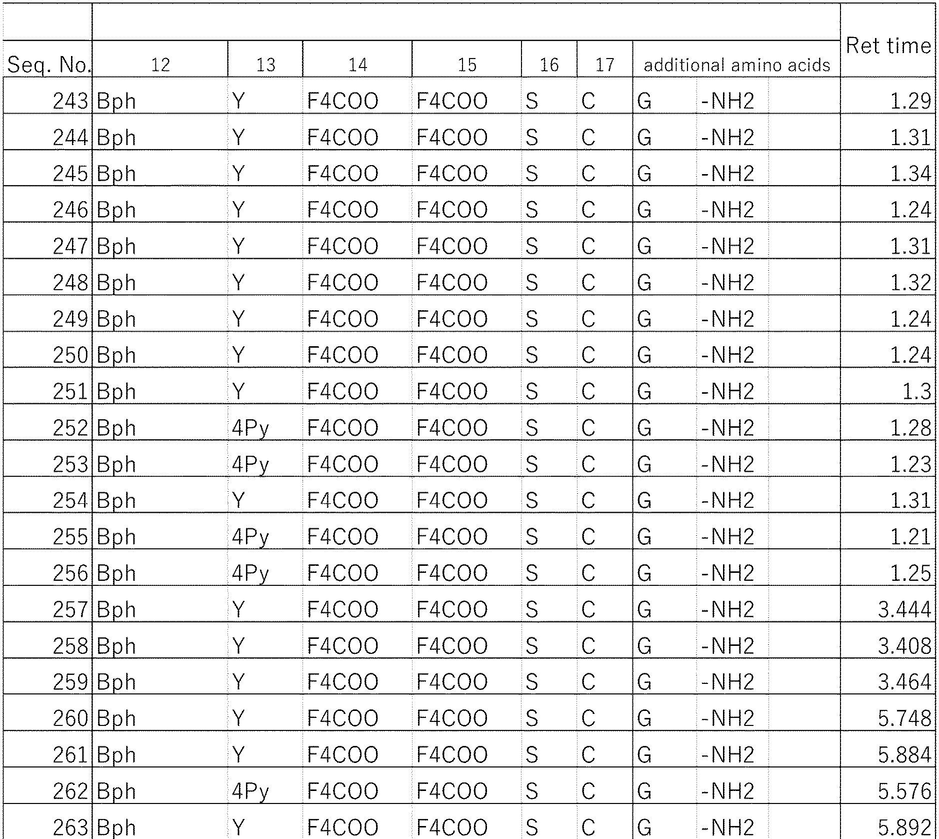

**[Table 6-1-3]**

| | ESI (m/z) | [M+XH]X+ | aLIZA BMP4 (0.2 nM) % inh | aLIZA BMP7 (10 nM) % inh | Synthesis | Peptide Conc. in Assay (nM) | |
|---|---|---|---|---|---|---|---|
| Seq. No. | | | | | | BMP4 Assay | BMP7 Assay |
| 243 | 855.9 | 3 | C | E | 1 | 0.02, 0.2, 2 | 10, 100, 1000 |
| 244 | 836.5 | 3 | C | ND | 1 | 0.02, 0.2, 2 | 10, 100, 1000 |
| 245 | 850.7 | 3 | C | ND | 1 | 0.02, 0.2, 2 | 10, 100, 1000 |
| 246 | 850.9 | 3 | C | E | 1 | 0.02, 0.2, 2 | 10, 100, 1000 |
| 247 | 856.5 | 3 | C | E | 1 | 0.02, 0.2, 2 | 10, 100, 1000 |
| 248 | 903.1 | 3 | C | ND | 1 | 0.02, 0.2, 2 | 10, 100, 1000 |
| 249 | 847.2 | 3 | C | E | 1 | 0.02, 0.2, 2 | 10, 100, 1000 |
| 250 | 872.5 | 3 | C | E | 1 | 0.02, 0.2, 2 | 10, 100, 1000 |
| 251 | 860.5 | 3 | C | ND | 1 | 0.02, 0.2, 2 | 10, 100, 1000 |
| 252 | 845.6 | 3 | C | ND | 1 | 0.02, 0.2, 2 | 10, 100, 1000 |
| 253 | 841.6 | 3 | C | ND | 1 | 0.02, 0.2, 2 | 10, 100, 1000 |
| 254 | 851.3 | 3 | C | ND | 1 | 0.02, 0.2, 2 | 10, 100, 1000 |
| 255 | 912 | 3 | C | ND | 1 | 0.02, 0.2, 2 | 10, 100, 1000 |
| 256 | 842.4 | 3 | C | ND | 1 | 0.02, 0.2, 2 | 10, 100, 1000 |
| 257 | 846.52 | 3 | C | E | 2 | 0.02, 0.2, 2 | 10, 100, 1000 |
| 258 | 855.54 | 3 | B | E | 2 | 0.02, 0.2, 2 | 10, 100, 1000 |
| 259 | 846.56 | 3 | C | E | 2 | 0.02, 0.2, 2 | 10, 100, 1000 |
| 260 | 860.54 | 3 | B | E | 2 | 0.02, 0.2, 2 | 10, 100, 1000 |
| 261 | 846.52 | 3 | C | E | 2 | 0.02, 0.2, 2 | 10, 100, 1000 |
| 262 | 850.89 | 3 | C | ND | 2 | 0.02, 0.2, 2 | 10, 100, 1000 |
| 263 | 916.96 | 3 | C | E | 2 | 0.02, 0.2, 2 | 10, 100, 1000 |

**[Table 6-2-1]**

| Seq.No. | 1 | 2 | 3 | 4 | 5 | 6 | 7 | 8 | 9 | 10 | 11 |
|---|---|---|---|---|---|---|---|---|---|---|---|
| 264 | E | R | V | Y | V | D | R | 3Py6Ph | KCOpipzaa | Y | V |
| 265 | E | R | V | F4COO | V | D | R | 3Py6Ph | S | Y | V |
| 266 | Q | R | V | Y | V | D | R | 3Py6Ph | S | Y | V |
| 267 | E | R | V | F4COO | V | D | R | 3Py6Ph | S | Y | V |
| 268 | E | R | V | Y | V | D | R | 3Py6Ph | KCOpipzaa | Y | V |
| 269 | Q | R | V | F4COO | V | D | R | 3Py6Ph | S | Y | V |
| 270 | Q | R | V | F4aao | V | D | R | 3Py6Ph | S | Y | V |
| 271 | E | R | V | F4COO | CF3A | D | R | 3Py6Ph | S | Y | V |
| 272 | E | R | V | F4COO | Gcpr | D | R | 3Py6Ph | S | Y | V |
| 273 | E | R | V | F4COO | V | D | R | 3Py6Ph | KCOpipzaa | Y | V |
| 274 | E | R | V | F4COO | V | D | R | 3Py6Ph | KCOpipzaa | Y | Hyp |
| 275 | E | R | V | F4COO | V | D | R | 3Py6Ph | KCOpipzaa | Y | Hyp |
| 276 | E | R | V | F4COO | V | KCOpipzaa | R | 3Py6Ph | KCOpipzaa | Y | Hyp |
| 277 | E | R | V | F4COO | V | KCOpipzaa | R | 3Py6Ph | KCOpipzaa | Y | Hyp |
| 278 | E | KCOpipzaa | V | F4COO | V | KCOpipzaa | R | 3Py6Ph | KCOpipzaa | Y | Hyp |
| 279 | E | KCOpipzaa | V | F4COO | V | KCOpipzaa | R | 3Py6Ph | KCOpipzaa | Y | Hyp |
| 280 | E | R | V | F4COO | V | D | R | 3Pv6Ph | KCOpipzaa | Y | Hyp |
| 281 | E | R | V | F4COO | V | D | R | 3Py6Ph | KCOpipzaa | Y | V |
| 282 | E | R | V | F4COO | V | D | R | 3Py6Ph | KCOpipzaa | Y | Hyp |
| 283 | E | R | V | F4COO | V | KCOpipzaa | R | 3Py6Ph | KCOpipzaa | Y | Hyp |
| 284 | E | R | V | F4COO | V | KCOpipzaa | R | 3Py6Ph | KCOpipzaa | Y | V |

**[Table 6-2-2]**

| | | | | | | | | | | Ret time | ESI (m/z) | [M+XH]X+ |
|---|---|---|---|---|---|---|---|---|---|---|---|---|
| Seq.No. | 12 | 13 | 14 | 15 | 16 | 17 | additional amino acids | | | | | |
| 264 | Bph | Y | F4COO | F4COO | S | C | G | -NH2 | | 5.888 | 926.3 | 3 |
| 265 | Bph | Y | F4COO | F4COO | S | C | G | -NH2 | | 3.372 | 865.2 | 3 |
| 266 | Bph | 4Py | F4COO | F4COO | S | C | G | -NH2 | | 5.576 | 850.54 | 3 |
| 267 | Bph | 4Py | F4COO | F4COO | S | C | G | -NH2 | | 5.656 | 860.21 | 3 |
| 268 | Bph | 4Py | F4COO | F4COO | S | C | G | -NH2 | | 5.024 | 921.67 | 3 |
| 269 | Bph | 4Py | F4COO | F4COO | S | C | G | -NH2 | | 5.548 | 859.85 | 3 |
| 270 | Bph | 4Py | F4COO | F4COO | S | C | G | -NH2 | | 5.268 | 870.21 | 3 |
| 271 | Bph | 4Py | F4COO | F4COO | S | C | G | -NH2 | | 5.684 | 873.43 | 3 |
| 272 | Bph | 4Py | F4COO | F4COO | S | C | G | -NH2 | | 5.552 | 859.41 | 3 |
| 273 | Bph | 4Py | F4COO | F4COO | S | C | G | -NH2 | | 5.032 | 930.99 | 3 |
| 274 | Bph | 4Py | F4COO | F4COO | S | C | G | -NH2 | | 5.068 | 935.15 | 3 |
| 275 | Bph | Y | F4COO | F4COO | S | C | G | -NH2 | | 5.556 | 940.22 | 3 |
| 276 | Bph | 4Py | F4COO | F4COO | S | C | G | -NH2 | | 4.884 | 996.22 | 3 |
| 277 | Bph | Y | F4COO | F4COO | S | C | G | -NH2 | | 5.356 | 1001.3 | 3 |
| 278 | Bph | 4Py | F4COO | F4COO | S | C | G | -NH2 | | 5.036 | 1043.6 | 3 |
| 279 | Bph | Y | F4COO | F4COO | S | C | G | -NH2 | | 5.472 | 1048.6 | 3 |
| 280 | Bph | 4Py | F4COO | F4COO | S | C | bA | OH | | 4.984 | 940.26 | 3 |
| 281 | Bph | 4Py | F4COO | F4COO | S | C | bA | OH | | 5.412 | 935.55 | 3 |
| 282 | Bph | Y | F4COO | F4COO | S | C | bA | OH | | 5.528 | 945.28 | 3 |
| 283 | Bph | 4Py | F4COO | F4COO | S | C | bA | OH | | 4.808 | 1001.3 | 3 |
| 284 | Bph | 4Py | F4COO | F4COO | S | C | bA | OH | | 5.14 | 996.64 | 3 |

**[Table 6-2-3]**

| | aLIZA BMP4 (0.2 nM) % inh | aLIZA BMP7 (10 nM) % inh | Synthesis | Peptide Conc. in Assay (nM) | |
|---|---|---|---|---|---|
| Seq.No. | | | | BMP4 Assay | BMP7 Assay |
| 264 | C | E | 2 | 0.02, 0.2, 2 | 10, 100, 1000 |
| 265 | B | E | 2 | 0.02, 0.2, 2 | 10, 100, 1000 |
| 266 | C | ND | 2 | 0.02, 0.2, 2 | 10, 100, 1000 |
| 267 | C | ND | 2 | 0.02, 0.2, 2 | 10, 100, 1000 |
| 268 | C | ND | 2 | 0.02, 0.2, 2 | 10, 100, 1000 |
| 269 | C | ND | 2 | 0.02, 0.2, 2 | 10, 100, 1000 |
| 270 | C | ND | 2 | 0.02, 0.2, 2 | 10, 100, 1000 |
| 271 | C | ND | 2 | 0.02, 0.2, 2 | 10, 100, 1000 |
| 272 | C | ND | 2 | 0.02, 0.2, 2 | 10, 100, 1000 |
| 273 | C | ND | 2 | 0.02, 0.2, 2 | 10, 100, 1000 |
| 274 | B | ND | 2 | 0.02, 0.2, 2 | 100, 300, 1000 |
| 275 | B | G | 2 | 0.02, 0.2, 2 | 100, 300, 1000 |
| 276 | B | ND | 2 | 0.02, 0.2, 2 | 100, 300, 1000 |
| 277 | B | H | 2 | 0.02, 0.2, 2 | 100, 300, 1000 |
| 278 | C | ND | 2 | 0.02, 0.2, 2 | 100, 300, 1000 |
| 279 | B | H | 2 | 0.02, 0.2, 2 | 100, 300, 1000 |
| 280 | B | H | 2 | 0.02, 0.2, 2 | 100, 300, 1000 |
| 281 | C | ND | 2 | 0.02, 0.2, 2 | 100, 300, 1000 |
| 282 | B | G | 2 | 0.02, 0.2, 2 | 100, 300, 1000 |
| 283 | B | H | 2 | 0.02, 0.2, 2 | 100, 300, 1000 |
| 284 | C | ND | 2 | 0.02, 0.2, 2 | 100, 300, 1000 |

**[Table 6-3-1]**

| Seq.No. | 1 | 2 | 3 | 4 | 5 | 6 | 7 | 8 | 9 | 10 |
|---|---|---|---|---|---|---|---|---|---|---|
| 285 | E | KCOpipzaa | V | F4COO | V | KCOpipzaa | R | 3Py6Ph | KCOpipzaa | Y |
| 286 | E | R | V | F4COO | V | D | R | 3Py6Ph | KCOpipzaa | Y |
| 287 | E | R | V | F4COO | Atp | KCOpipzaa | R | 3Py6Ph | KCOpipzaa | Y |
| 288 | E | R | V | F4COO | Cha4tH | KCOpipzaa | R | 3Py6Ph | KCOpipzaa | Y |
| 289 | E | R | V | F4COO | Gthp | KCOpipzaa | R | 3Py6Ph | KCOpipzaa | Y |
| 290 | E | R | V | F4COO | V | KCOpipzaa | R | Bph4C | KCOpipzaa | Y |
| 291 | E | R | V | F4COO | V | KCOpipzaa | R | pBph2F | KCOpipzaa | Y |
| 292 | E | R | V | F4COO | V | KCOpipzaa | R | 3Py6Ph | KCOpipzaa | Y |
| 293 | E | R | V | F4COO | Cha4tH | KCOpipzaa | R | 3Py6Ph | KCOpipzaa | Y |
| 294 | E | R | V | F4COO | Cha4tH | KCOpipzaa | R | 3Py6Ph | KCOpipzaa | Y |
| 295 | E | R | V | F4COO | V | KCOpipzaa | R | Bph4C | KCOpipzaa | Y |
| 296 | E | R | V | F4COO | V | KCOpipzaa | R | 3Py6Ph | KCOpipzaa | Y |
| 297 | E | R | V | F4COO | Atp | KCOpipzaa | R | 3Py6Ph | KCOpipzaa | Y |
| 298 | E | R | V | F4COO | Cha4tH | KCOpipzaa | R | 3Py6Ph | KCOpipzaa | Y |
| 299 | E | R | V | F4COO | V | KCOpipzaa | R | 3Py6Ph | KCOpipzaa | Y |
| 300 | E | R | V | F4COO | V | KCOpipzaa | R | 3Py6Ph | KCOpipzaa | Y |
| 301 | E | R | V | F4COO | V | KCOpipzaa | R | 3Py6Ph | KCOpipzaa | Y |
| 302 | E | R | V | F4COO | V | KCOpipzaa | R | 3Py6Ph | KCOpipzaa | Y |
| 303 | E | R | V | F4COO | V | KCOpipzaa | R | Bph3C | KCOpipzaa | Y |
| 304 | E | R | V | F4COO | V | KCOpipzaa | R | pBph2F | KCOpipzaa | Y |
| 305 | E | R | V | F4COO | A1Ac4pip | KCOpipzaa | R | 3Py6Ph | KCOpipzaa | Y |

**[Table 6-3-2]**

| | | | | | | | | | | | Ret time | ESI (m/z) |
|---|---|---|---|---|---|---|---|---|---|---|---|---|
| Seq.No. | 11 | 12 | 13 | 14 | 15 | 16 | 17 | additi onal am ino acids | | | | |
| 285 | Hyp | Bph | 4Py | F4COO | F4COO | S | C | bA | OH | | 5 | 1048.7 |
| 286 | Hyp | Bph | 4Py | F4COO | F4COO | S | C | bA | de | OH | 4.956 | 983.31 |
| 287 | Hyp | Bph | 4Py | F4COO | F4COO | S | C | G | -NH2 | | 4.824 | 1014.9 |
| 288 | Hyp | Bph | 4Py | F4COO | F4COO | S | C | G | -NH2 | | 4.74 | 1019.6 |
| 289 | Hyp | Bph | 4P y | F4COO | F4COO | S | C | G | -NH2 | | 4.808 | 1010.2 |
| 290 | Hyp | Bph | 4Py | F4COO | F4COO | S | C | G | -NH2 | | 5.284 | 1007.9 |
| 291 | Hyp | Bph | 4Py | F4COO | F4COO | S | C | G | -NH2 | | 5.504 | 1001.9 |
| 292 | Hyp | Bph3OMe | 4Py | F4COO | F4COO | S | C | G | -NH2 | | 4.924 | 1006.3 |
| 293 | Hyp | Bph3OMe | 4Py | F4COO | F4COO | S | C | G | -NH2 | | 4.744 | 1029.6 |
| 294 | Hyp | Bph | Y | F4COO | F4COO | S | C | G | -NH2 | | 5.228 | 1024.6 |
| 295 | Hyp | Bph | Y | F4COO | F4COO | S | C | G | -NH2 | | 3.396 | 1012.5 |
| 296 | Hyp | Bph3OMe | Y | F4COO | F4COO | S | C | G | -NH2 | | 5.356 | 1011.2 |
| 297 | Hvp | Bph3OMe | Y | F4COO | F4COO | S | C | G | -NH2 | | 5.3 | 1029.9 |
| 298 | Hyp | Bph3OMe | Y | F4COO | F4COO | S | C | G | -NH2 | | 5.22 | 1034.6 |
| 299 | Hyp | Bph4F | Y | F4COO | F4COO | S | C | G | -NH2 | | 1.34 | 1007.6 |
| 300 | Hyp | Bph3Me | Y | F4COO | F4COO | S | C | G | -NH2 | | 1.35 | 1006.3 |
| 301 | Hyp | pBph3F | Y | F4COO | F4COO | S | C | G | -NH2 | | 1.34 | 1007.6 |
| 302 | Hyp | pBph2F | Y | F4COO | F4COO | S | C | G | -NH2 | | 1.34 | 1007.7 |
| 303 | Hyp | Bph | Y | F4COO | F4COO | S | C | G | -NH2 | | 1.42 | 1012.4 |
| 304 | Hyp | Bph | Y | F4COO | F4COO | S | C | G | -NH2 | | 1.4 | 1007.3 |
| 305 | Hyp | Bph | Y | F4COO | F4COO | S | C | G | -NH2 | | 1.31 | 1033.8 |

**[Table 6-3-3]**

| | [M+XH]X+ | aLIZA BMP4 (0.2 nM) % inh | aLIZA BMP7 (10 nM) % inh | Synthesis | Peptide Conc. in Assay (nM) | |
|---|---|---|---|---|---|---|
| Seq.No. | | | | | BMP4 Assay | BMP7 Assay |
| 285 | 3 | C | H | 2 | 0.02, 0.2, 2 | 100, 300, 1000 |
| 286 | 3 | B | G | 2 | 0.02, 0.2, 2 | 100, 300, 1000 |
| 287 | 3 | C | H | 2 | 0.02, 0.2, 2 | 100, 300, 1000 |
| 288 | 3 | B | F | 2 | 0.02, 0.2, 2 | 100, 300, 1000 |
| 289 | 3 | C | ND | 2 | 0.02, 0.2, 2 | 100, 300, 1000 |
| 290 | 3 | B | G | 2 | 0.02, 0.2, 2 | 100, 300, 1000 |
| 291 | 3 | B | G | 2 | 0.02, 0.2, 2 | 100, 300, 1000 |
| 292 | 3 | C | ND | 2 | 0.02, 0.2, 2 | 100, 300, 1000 |
| 293 | 3 | C | ND | 2 | 0.02, 0.2, 2 | 100, 300, 1000 |
| 294 | 3 | B | F | 2 | 0.02, 0.2, 2 | 100, 300, 1000 |
| 295 | 3 | B | F | 2 | 0.02, 0.2, 2 | 100, 300, 1000 |
| 296 | 3 | B | ND | 2 | 0.02, 0.2, 2 | 100, 300, 1000 |
| 297 | 3 | C | ND | 2 | 0.02, 0.2, 2 | 100, 300, 1000 |
| 298 | 3 | B | H | 2 | 0.02, 0.2, 2 | 100, 300, 1000 |
| 299 | 3 | C | ND | 1 | 0.02, 0.2, 2 | 10, 100, 1000 |
| 300 | 3 | C | E | 1 | 0.02, 0.2, 2 | 10, 100, 1000 |
| 301 | 3 | C | E | 1 | 0.02, 0.2, 2 | 10, 100, 1000 |
| 302 | 3 | C | E | 1 | 0.02, 0.2, 2 | 10, 100, 1000 |
| 303 | 3 | C | E | 1 | 0.02, 0.2, 2 | 10, 100, 1000 |
| 304 | 3 | C | E | 1 | 0.02, 0.2, 2 | 10, 100, 1000 |
| 305 | 3 | C | E | 1 | 0.02, 0.2, 2 | 10, 100, 1000 |

**[Table 6-4-1]**

| Seq.No. | 1 | 2 | 3 | 4 | 5 | 6 | 7 | 8 | 9 | 10 | 11 |
|---|---|---|---|---|---|---|---|---|---|---|---|
| 306 | E | R | V | F4COO | Cba | KCOpipzaa | R | 3Py6Ph | KCOpipzaa | Y | Hyp |
| 307 | E | R | V | F4COO | Chg | KCOpipzaa | R | 3Py6Ph | KCOpipzaa | Y | Hyp |
| 308 | E | R | V | F4COO | Cha | KCOpipzaa | R | 3Py6Ph | KCOpipzaa | Y | Hyp |
| 309 | E | R | V | F4COO | Gthp | KCOpipzaa | R | 3Py6Ph | KCOpipzaa | Y | Hyp |
| 310 | E | R | V | F4COO | Atp | KCOpipzaa | R | 3Py6Ph | KCOpipzaa | Y | Hyp |
| 311 | E | R | V | F4COO | V | KCOpipzaa | R | 3Py6Ph | KCOpipzaa | Y | Hyp |
| 312 | E | R | V | F4COO | V | KCOpipzaa | R | 3Py6Ph | KCOpipzaa | Y | Hyp |
| 313 | E | R | V | F4COO | Cba | KCOpipzaa | R | 3Py6Ph | KCOpipzaa | Y | Hyp |
| 314 | E | R | V | F4COO | Cba | KCOpipzaa | R | 3Py6Ph | KCOpipzaa | Y | Hyp |
| 315 | E | R | V | F4COO | Cba | KCOpipzaa | R | 3Py6Ph | KCOpipzaa | Y | Hyp |
| 316 | E | R | V | F4COO | Cba | KCOpipzaa | R | 3Py6Ph | KCOpipzaa | Y | Hyp |
| 317 | E | R | V | F4COO | Cba | KCOpipzaa | R | 3Py6Ph | KCOpipzaa | Y | Hyp |
| 318 | E | R | V | F4COO | Cha4tH | KCOpipzaa | R | 3Py6Ph | KCOpipzaa | Y | Hyp |
| 319 | E | R | V | F4COO | Cha4tH | KCOpipzaa | R | 3Py6Ph | KCOpipzaa | Y | Hyp |
| 320 | E | R | V | F4COO | Cha4tH | KCOpipzaa | R | 3Py6Ph | KCOpipzaa | Y | Hyp |
| 321 | E | R | V | F4COO | Cha4tH | KCOpipzaa | R | 3Py6Ph | KCOpipzaa | Y | Hyp |

**[Table 6-4-2]**

| | | | | | | | | | | Ret time | ESI (m/z) |
|---|---|---|---|---|---|---|---|---|---|---|---|
| Seq.No. | 12 | 13 | 14 | 15 | 16 | 17 | additional amino acids | | | | |
| 306 | Bph | Y | F4COO | F4COO | S | C | G | -NH2 | | 1.35 | 1010.2 |
| 307 | Bph | Y | F4COO | F4COO | S | C | G | -NH2 | | 1.35 | 1014.9 |
| 308 | Bph | Y | F4COO | F4COO | S | C | G | -NH2 | | 1.37 | 1019.5 |
| 309 | Bph | Y | F4COO | F4COO | S | C | G | -NH2 | | 1.31 | 1015.7 |
| 310 | Bph | Y | F4COO | F4COO | S | C | G | -NH2 | | 1.31 | 1020.2 |
| 311 | Bph3F | Y | F4COO | F4COO | S | C | G | -NH2 | | 5.044 | 1007.7 |
| 312 | Bph3C | Y | F4COO | F4COO | S | C | G | -NH2 | | 5.212 | 1013.2 |
| 313 | Bph3F | Y | F4COO | F4COO | S | C | G | -NH2 | | 5.528 | 1015.9 |
| 314 | Bph3C | Y | F4COO | F4COO | S | C | G | -NH2 | | 5.68 | 1021.5 |
| 315 | Bph | 4Py | F4COO | F4COO | S | C | G | -NH2 | | 5.028 | 1005 |
| 316 | Bph3F | 4Py | F4COO | F4COO | S | C | G | -NH2 | | 4.984 | 1011 |
| 317 | Bph3C | 4Py | F4COO | F4COO | S | C | G | -NH2 | | 5.248 | 1016.3 |
| 318 | Bph3F | Y | F4COO | F4COO | S | C | G | -NH2 | | 5.132 | 1030.6 |
| 319 | Bph3C | Y | F4COO | F4COO | S | C | G | -NH2 | | 5.304 | 1036.1 |
| 320 | Bph3F | 4Py | F4COO | F4COO | S | C | G | -NH2 | | 4.592 | 1025.6 |
| 321 | Bph3C | 4Py | F4COO | F4COO | S | C | G | -NH2 | | 4.768 | 1031.1 |

**[Table 6-4-3]**

| | [M+XH]X+ | aLIZA BMP4 (0.2 nM) % inh | aLIZA BMP7 (10 nM) % inh | Synthesis | Peptide Conc. in Assay (nM) | |
|---|---|---|---|---|---|---|
| Seq.No. | | | | | BMP4 Assay | BMP7 Assay |
| 306 | 3 | C | E | 1 | 0.02, 0.2, 2 | 10, 100, 1000 |
| 307 | 3 | C | E | 1 | 0.02, 0.2, 2 | 10, 100, 1000 |
| 308 | 3 | C | E | 1 | 0.02, 0.2, 2 | 10, 100, 1000 |
| 309 | 3 | C | ND | 1 | 0.02, 0.2, 2 | 10, 100, 1000 |
| 310 | 3 | C | E | 1 | 0.02, 0.2, 2 | 10, 100, 1000 |
| 311 | 3 | B | E | 2 | 0.02, 0.2, 2 | 10, 100, 1000 |
| 312 | 3 | A | E | 2 | 0.02, 0.2, 2 | 10, 100, 1000 |
| 313 | 3 | A | E | 2 | 0.02, 0.2, 2 | 10, 100, 1000 |
| 314 | 3 | B | E | 2 | 0.02, 0.2, 2 | 10, 100, 1000 |
| 315 | 3 | B | E | 2 | 0.02, 0.2, 2 | 10, 100, 1000 |
| 316 | 3 | C | ND | 2 | 0.02, 0.2, 2 | 10, 100, 1000 |
| 317 | 3 | B | ND | 2 | 0.02, 0.2, 2 | 10, 100, 1000 |
| 318 | 3 | B | D | 2 | 0.02, 0.2, 2 | 10, 100, 1000 |
| 319 | 3 | B | D | 2 | 0.02, 0.2, 2 | 10, 100, 1000 |
| 320 | 3 | B | E | 2 | 0.02, 0.2, 2 | 10, 100, 1000 |
| 321 | 3 | B | E | 2 | 0.02, 0.2, 2 | 10, 100, 1000 |

### Table 7

**[Table 7-1-1]**

| Seq ID No. | | | | | | | | | | |
|---|---|---|---|---|---|---|---|---|---|---|
| | 1 | 2 | 3 | 4 | 5 | 6 | 7 | 8 | 9 | 10 |
| 322 | 4Py | G | MeF | G | Bph | G | D | D | Cha | A |
| 323 | 3Py | G | MeF | G | Bph | G | D | D | Cha | A |
| 324 | F | G | MeF | G | F | G | D | D | Cha | A |
| 325 | F | G | MeF | G | F44Py | G | D | D | Cha | A |
| 326 | F | G | MeF | G | F43Py | G | D | D | Cha | A |
| 327 | F | G | MeF | G | F42Py | G | D | D | Cha | A |
| 328 | F | G | MeF | G | 3Pv6Ph | G | D | D | Cha | A |
| 329 | F | G | MeF | G | F44thp | G | D | D | Cba | A |
| 330 | F | G | MeF | da | Bph | G | D | D | Cha | A |
| 331 | F | G | MeF | ds | Bph | G | D | D | Cha | A |
| 332 | F | G | MeF | G | Bph | A | D | D | Cha | A |
| 333 | F | G | MeF | G | Bph | da | D | D | Cha | A |
| 334 | F | G | MeF | G | Bph | G | N | D | Cha | A |
| 335 | F | G | MeF | G | Bph | G | D | D | Acpe | A |
| 336 | F | G | MeF | G | Bph | G | D | D | Cha | E |
| 337 | F | G | MeF | G | Bph | G | D | D | Cha | Hgl |
| 338 | F | G | MeF | G | Bph | G | D | D | Cha | Ahp |
| 339 | F | G | MeF | G | Bph | G | D | D | Cha | Abu |
| 340 | F | G | MeF | G | Bph | G | D | D | Cha | Q |
| 341 | F | G | MeF | G | Bph | G | D | D | Cha | S |
| 342 | F | G | MeF | G | Bph | G | D | D | Cha | T |
| 343 | F | G | MeF | G | Bph | G | D | D | Cha | Hgn |
| 344 | F | G | MeF | G | Bph | G | D | D | Cha | A |
| 345 | F | G | MeF | G | Bph | G | D | D | Cha | A |
| 346 | F | G | MeF | G | Bph | G | D | D | Cha | A |

**[Table 7-1-2]**

| Seq ID | | | | | | | | Ret time | ESI (m/z) |
|---|---|---|---|---|---|---|---|---|---|
| No. | 11 | 12 | 13 | 14 | additional amino acids | | | | |
| 322 | MeF | L | H | C | G | -NH2 | | 1.46 | 894.4 |
| 323 | MeF | L | H | C | G | -NH2 | | 1.47 | 894.6 |
| 324 | MeF | L | H | C | G | -NH2 | | 1.57 | 855.9 |
| 325 | MeF | L | H | C | G | -NH2 | | 1.38 | 894.4 |
| 326 | MeF | L | H | C | G | -NH2 | | 1.4 | 894.4 |
| 327 | MeF | L | H | C | G | -NH2 | | 1.43 | 894.4 |
| 328 | MeF | L | H | C | G | -NH2 | | 1.48 | 894.4 |
| 329 | MeF | L | H | C | G | -NH2 | | 1.59 | 898 |
| 330 | MeF | L | H | C | G | -NH2 | | 1.66 | 900.9 |
| 331 | MeF | L | H | C | G | -NH2 | | 1.64 | 908.9 |
| 332 | MeF | L | H | C | G | -NH2 | | 1.65 | 900.9 |
| 333 | MeF | L | H | C | G | -NH2 | | 1.65 | 900.9 |
| 334 | MeF | L | H | C | G | -NH2 | | 1.64 | 893.3 |
| 335 | MeF | L | H | C | G | -NH2 | | 1.62 | 886.8 |
| 336 | MeF | L | H | C | G | -NH2 | | 1.62 | 922.9 |
| 337 | MeF | L | H | C | G | -NH2 | | 1.63 | 929.9 |
| 338 | MeF | L | H | C | G | -NH2 | | 1.73 | 922 |
| 339 | MeF | L | H | C | G | -NH2 | | 1.66 | 900.8 |
| 340 | MeF | L | H | C | G | -NH2 | | 1.61 | 922.4 |
| 341 | MeF | L | H | C | G | -NH2 | | 1.63 | 901.8 |
| 342 | MeF | L | H | C | G | -NH2 | | 1.64 | 908.9 |
| 343 | MeF | L | H | C | G | -NH2 | | 1.62 | 929.4 |
| 344 | MeF | Cha | H | C | G | -NH2 | | 1.78 | 914.1 |
| 345 | MeF | Ahp | H | C | G | -NH2 | | 1.68 | 900.9 |
| 346 | MeF | I | H | C | G | -NH2 | | 1.63 | 893.9 |

**[Table 7-1-3]**

| Seq ID | [M+XH]X+ | aLISA BMP4 (0.2 nM) % inh | aLISA BMP7 (10nM) % inh | Synthesis | Peptide Conc. in Assay (nM) | |
|---|---|---|---|---|---|---|
| No. | | | | | BMP4 Assay | BMP7 Assay |
| 322 | 2 | ND | D | 1 | 0.2, 2, 20 | 1, 10, 100 |
| 323 | 2 | ND | D | 1 | 0.2, 2, 20 | 1, 10, 100 |
| 324 | 2 | ND | D | 1 | 0.2, 2, 20 | 1, 10, 100 |
| 325 | 2 | ND | D | 1 | 0.2, 2, 20 | 1, 10, 100 |
| 326 | 2 | ND | D | 1 | 0.2, 2, 20 | 1, 10, 100 |
| 327 | 2 | A | D | 1 | 0.2, 2, 20 | 1, 10, 100 |
| 328 | 2 | ND | D | 1 | 0.2, 2, 20 | 1, 10, 100 |
| 329 | 2 | ND | D | 1 | 0.2, 2, 20 | 1, 10, 100 |
| 330 | 2 | ND | D | 1 | 0.2, 2, 20 | 1, 10, 100 |
| 331 | 2 | ND | D | 1 | 0.2, 2, 20 | 1, 10, 100 |
| 332 | 2 | ND | D | 1 | 0.2, 2, 20 | 1, 10, 100 |
| 333 | 2 | ND | D | 1 | 0.2, 2, 20 | 1, 10, 100 |
| 334 | 2 | ND | D | 1 | 0.2, 2, 20 | 1, 10, 100 |
| 335 | 2 | ND | D | 1 | 0.2, 2, 20 | 1, 10, 100 |
| 336 | 2 | ND | D | 1 | 0.2, 2, 20 | 1, 10, 100 |
| 337 | 2 | ND | D | 1 | 0.2, 2, 20 | 1, 10, 100 |
| 338 | 2 | ND | D | 1 | 0.2, 2, 20 | 1, 10, 100 |
| 339 | 2 | ND | D | 1 | 0.2, 2, 20 | 1, 10, 100 |
| 340 | 2 | ND | D | 1 | 0.2, 2, 20 | 1, 10, 100 |
| 341 | 2 | ND | D | 1 | 0.2, 2, 20 | 1, 10, 100 |
| 342 | 2 | ND | D | 1 | 0.2, 2, 20 | 1, 10, 100 |
| 343 | 2 | ND | D | 1 | 0.2, 2, 20 | 1, 10, 100 |
| 344 | 2 | A | D | 1 | 0.2, 2, 20 | 1, 10, 100 |
| 345 | 2 | A | D | 1 | 0.2, 2, 20 | 1, 10, 100 |
| 346 | 2 | ND | D | 1 | 0.2, 2, 20 | 1, 10, 100 |

**[Table 7-2-1]**

| Seq ID | | | | | | | | | | |
|---|---|---|---|---|---|---|---|---|---|---|
| No. | 1 | 2 | 3 | 4 | 5 | 6 | 7 | 8 | 9 | 10 |
| 347 | F | G | MeF | G | Bph | G | D | D | Cha | A |
| 348 | F | G | MeF | G | Bph | G | D | D | Cha | A |
| 349 | F | G | MeF | G | Bph | G | D | D | Cha | A |
| 350 | F | G | MeF | G | Bph | G | D | D | Cha | A |
| 351 | F | G | MeF | G | Bph | G | D | D | Cha | A |
| 352 | F | G | MeF | G | Bph | G | D | D | Cha | A |
| 353 | F | G | MeF | G | Bph | G | D | D | Cha | A |
| 354 | F | G | MeF | G | Bph | G | D | D | Cha | A |
| 355 | F | G | MeF | G | Bph | G | D | D | Cha | A |
| 356 | F | G | MeF | G | Bph | G | D | D | Cha | A |
| 357 | F | G | MeF | G | Bph | G | D | D | Cha | A |
| 358 | F | G | MeF | G | Bph | G | D | E | Cha | A |
| 359 | F | G | MeF | G | Bph | G | D | E | Cha | A |
| 360 | F | G | MeF | G | Bph | G | D | E | Cha | A |
| 361 | F | G | MeF | G | Bph | G | D | E | Cha | A |
| 362 | F | G | MeF | G | F | G | D | E | Cha | A |
| 363 | F | G | MeF | G | 3Py6Ph | G | D | E | Cha | A |
| 364 | F | G | MeF | G | Bph | G | D | E | Cha | Q |
| 365 | F | G | MeF | G | Bph | G | D | E | Cha | Abu |
| 366 | F | G | MeF | G | Bph | G | D | E | Cha | Hgl |
| 367 | F | G | MeF | G | F | G | D | E | Cha | Q |
| 368 | F | G | MeF | G | Bph | G | D | E | Cha | Q |
| 369 | F | G | MeF | G | F | G | D | E | Cha | Q |
| 370 | F | G | MeF | G | 3Py6Ph | G | D | E | Cha | Q |
| 371 | F | G | MeF | G | Bph | G | D | D | Cha | A |

**[Table 7-2-2]**

| Seq ID | | | | | | | | Ret time | **ESI** (m/z) |
|---|---|---|---|---|---|---|---|---|---|
| No. | 11 | 12 | 13 | 14 | additional amino acids | | | | |
| 347 | MeF | Chg | H | C | G | -NH2 | | 1.66 | 906.9 |
| 348 | MeF | L | A | C | G | -NH2 | | 1.84 | 860.8 |
| 349 | MeF | L | L | C | G | -NH2 | | 1.9 | 881.8 |
| 350 | MeF | L | Abu | C | G | -NH2 | | 1.86 | 867.8 |
| 351 | MeF | L | 4Py2NH2 | C | G | -NH2 | | 1.65 | 906.8 |
| 352 | MeF | L | G | C | G | -NH2 | | 1.84 | 853.8 |
| 353 | MeF | L | S | C | G | -NH2 | | 1.82 | 868.8 |
| 354 | MeF | L | N | C | G | -NH2 | | 1.81 | 882.3 |
| 355 | MeF | L | Hyp | C | G | -NH2 | | 1.8 | 881.9 |
| 356 | MeF | L | P4Sh | C | G | -NH2 | | 1.82 | 881.8 |
| 357 | MeF | L | F4COO | C | G | -NH2 | | 1.82 | 920.9 |
| 358 | MeF | L | H | C | G | -NH2 | | 6.092 | 900.9 |
| 359 | MeF | L | Abu | C | G | -NH2 | | 3.747 | 875.2 |
| 360 | MeF | L | 4Py2NH2 | C | G | -NH2 | | 6.176 | 914 |
| 361 | MeF | L | F4COO | C | G | -NH2 | | 3.447 | 928.3 |
| 362 | MeF | L | H | C | G | -NH2 | | 5.004 | 863.3 |
| 363 | MeF | L | H | C | G | -NH2 | | 4.72 | 901.5 |
| 364 | MeF | L | H | C | G | -NH2 | | 5.792 | 929.5 |
| 365 | MeF | L | H | C | G | -NH2 | | 6.228 | 907.9 |
| 366 | MeF | L | H | C | G | -NH2 | | 6.056 | 937 |
| 367 | MeF | L | H | C | G | -NH2 | | 5.016 | 891.5 |
| 368 | MeF | L | F4COO | C | G | -NH2 | | 3.064 | 956.8 |
| 369 | MeF | L | F4COO | C | G | -NH2 | | 6.3 | 918.4 |
| 370 | MeF | L | F4COO | C | G | -NH2 | | 5.088 | 957.4 |
| 371 | MeF | L | H | C | G | -NH2 | | 1.6 | 894.2 |

**[Table 7-2-3]**

| Seq ID | [M+XH]X+ | aLISA BMP4 (0.2 nM) % inh | aLISA BMP7 (10nM) % inh | Synthesis | Peptide Conc. in Assay (nM) | |
|---|---|---|---|---|---|---|
| No. | | | | | BMP4 Assay | BMP7 Assay |
| 347 | 2 | ND | D | 1 | 0.2, 2, 20 | 1, 10, 100 |
| 348 | 2 | A | D | 1 | 0.2, 2, 20 | 1, 10, 100 |
| 349 | 2 | ND | D | 1 | 0.2, 2, 20 | 1, 10, 100 |
| 350 | 2 | ND | D | 1 | 0.2, 2, 20 | 1, 10, 100 |
| 351 | 2 | ND | D | 1 | 0.2, 2, 20 | 1, 10, 100 |
| 352 | 2 | ND | D | 1 | 0.2, 2, 20 | 1, 10, 100 |
| 353 | 2 | ND | D | 1 | 0.2, 2, 20 | 1, 10, 100 |
| 354 | 2 | ND | D | 1 | 0.2, 2, 20 | 1, 10, 100 |
| 355 | 2 | A | D | 1 | 0.2, 2, 20 | 1, 10, 100 |
| 356 | 2 | A | C | 1 | 0.2, 2, 20 | 1, 10, 100 |
| 357 | 2 | ND | D | 1 | 0.2, 2, 20 | 1, 10, 100 |
| 358 | 2 | ND | D | 2 | 0.2, 2, 20 | 1, 10, 100 |
| 359 | 2 | ND | D | 2 | 0.2, 2, 20 | 1, 10, 100 |
| 360 | 2 | ND | D | 2 | 0.2, 2, 20 | 1, 10, 100 |
| 361 | 2 | ND | D | 2 | 0.2, 2, 20 | 1, 10, 100 |
| 362 | 2 | ND | D | 2 | 0.2, 2, 20 | 1, 10, 100 |
| 363 | 2 | ND | D | 2 | 0.2, 2, 20 | 1, 10, 100 |
| 364 | 2 | ND | D | 2 | 0.2, 2, 20 | 1, 10, 100 |
| 365 | 2 | ND | D | 2 | 0.2, 2, 20 | 1, 10, 100 |
| 366 | 2 | ND | D | 2 | 0.2, 2, 20 | 1, 10, 100 |
| 367 | 2 | ND | D | 2 | 0.2, 2, 20 | 1, 10, 100 |
| 368 | 2 | ND | C | 2 | 0.2, 2, 20 | 1, 10, 100 |
| 369 | 2 | ND | D | 2 | 0.2, 2, 20 | 1, 10, 100 |
| 370 | 2 | ND | D | 2 | 0.2, 2, 20 | 1, 10, 100 |
| 371 | 2 | A | E | 1 | 2, 20, 200 | 10, 30, 90 |

**[Table 7-3-1]**

| Seq ID | | | | | | | | | | |
|---|---|---|---|---|---|---|---|---|---|---|
| No. | 1 | 2 | 3 | 4 | 5 | 6 | 7 | 8 | 9 | 10 |
| 372 | F3C | G | MeF | G | Bph | G | D | D | Cha | A |
| 373 | F4C | G | MeF | G | Bph | G | D | D | Cha | A |
| 374 | F3Me | G | MeF | G | Bph | G | D | D | Cha | A |
| 375 | F4Me | G | MeF | G | Bph | G | D | D | Cha | A |
| 376 | F | G | MeF | G | 3Pv | G | D | D | Cha | A |
| 377 | F | G | MeF | G | 4Py | G | D | D | Cha | A |
| 378 | F | G | MeF | G | F4F | G | D | D | Cha | A |
| 379 | F | G | MeF | G | F3C | G | D | D | Cha | A |
| 380 | F | G | MeF | G | F4C | G | D | D | Cha | A |
| 381 | F | G | MeF | G | F3Me | G | D | D | Cha | A |
| 382 | F | G | MeF | G | F4Me | G | D | D | Cha | A |
| 383 | F | G | MeF | G | Y | G | D | D | Cha | A |
| 384 | F | G | MeF3C | G | Bph | G | D | D | Cha | A |
| 385 | F | G | MeF4C | G | Bph | G | D | D | Cha | A |
| 386 | F | G | MeF3Me | G | Bph | G | D | D | Cha | A |
| 387 | F | G | MeY | G | Bph | G | D | D | Cha | A |
| 388 | F | G | MeF | G | Bph | G | D | D | Cha | A |
| 389 | F | G | MeF | G | Bph | G | D | D | Cha | A |
| 390 | F | G | MeF | G | Bph | G | D | D | Cha | A |
| 391 | F | G | MeF | G | Bph | G | D | D | Cha | A |
| 392 | F4F | G | MeF | G | Bph | G | D | D | Cha | A |
| 393 | F | G | MeF4Me | G | Bph | G | D | D | Cha | A |
| 394 | F | G | MeF | G | Bph | G | D | D | Cha | A |
| 395 | F4F | G | MeF4Me | G | Bph | G | D | D | Cha | A |
| 396 | F4F | G | MeF | G | Bph | G | D | D | Cha | A |

**[Table 7-3-2]**

| Seq ID | | | | | | | | Ret time | ESI (m/z) |
|---|---|---|---|---|---|---|---|---|---|
| No. | 11 | 12 | 13 | 14 | additional amino acids | | | | |
| 372 | MeF | L | H | C | G | -NH2 | | 1.63 | 911.4 |
| 373 | MeF | L | H | C | G | -NH2 | | 1.63 | 911.5 |
| 374 | MeF | L | H | C | G | -NH2 | | 1.63 | 901.2 |
| 375 | MeF | L | H | C | G | -NH2 | | 1.63 | 901.2 |
| 376 | MeF | L | H | C | G | -NH2 | | 1.31 | 856.6 |
| 377 | MeF | L | H | C | G | -NH2 | | 1.3 | 856.5 |
| 378 | MeF | L | H | C | G | -NH2 | | 1.52 | 864.8 |
| 379 | MeF | L | H | C | G | -NH2 | | 1.54 | 873.4 |
| 380 | MeF | L | H | C | G | -NH2 | | 1.55 | 873.3 |
| 381 | MeF | L | H | C | G | -NH2 | | 1.54 | 863.1 |
| 382 | MeF | L | H | C | G | -NH2 | | 1.54 | 863.1 |
| 383 | MeF | L | H | C | G | -NH2 | | 1.44 | 864.1 |
| 384 | MeF | L | H | C | G | -NH2 | | 1.64 | 911.5 |
| 385 | MeF | L | H | C | G | -NH2 | | 1.64 | 911.5 |
| 386 | MeF | L | H | C | G | -NH2 | | 1.63 | 901.2 |
| 387 | MeF | L | H | C | G | -NH2 | | 1.54 | 902.1 |
| 388 | MeF3C | L | H | C | G | -NH2 | | 1.63 | 911.7 |
| 389 | MeF4C | L | H | C | G | -NH2 | | 1.63 | 911.7 |
| 390 | MeF3Me | L | H | C | G | -NH2 | | 1.62 | 901.2 |
| 391 | MeY | L | H | C | G | -NH2 | | 1.55 | 902.1 |
| 392 | MeF | L | H | C | G | -NH2 | | 5.804 | 903.2 |
| 393 | MeF | L | H | C | G | -NH2 | | 5.968 | 901.2 |
| 394 | MeF4Me | L | H | C | G | -NH2 | | 5.932 | 901.3 |
| 395 | MeF | L | H | C | G | -NH2 | | 6.024 | 910.4 |
| 396 | MeF4Me | L | H | C | G | -NH2 | | 6 | 910.3 |

**[Table 7-3-3]**

| Seq ID | [M+XH]X+ | aLISA BMP4 (0.2 nM) % inh | aLISA BMP7 (10nM) % inh | Synthesis | Peptide Conc. in Assay (nM) | |
|---|---|---|---|---|---|---|
| No. | | | | | BMP4 Assay | BMP7 Assay |
| 372 | 2 | ND | G | 1 | 2, 20, 200 | 10, 30, 90 |
| 373 | 2 | A | F | 1 | 2, 20, 200 | 10, 30, 90 |
| 374 | 2 | ND | G | 1 | 2, 20, 200 | 10, 30, 90 |
| 375 | 2 | B | F | 1 | 2, 20, 200 | 10, 30, 90 |
| 376 | 2 | ND | G | 1 | 2, 20, 200 | 10, 30, 90 |
| 377 | 2 | ND | F | 1 | 2, 20, 200 | 10, 30, 90 |
| 378 | 2 | ND | F | 1 | 2, 20, 200 | 10, 30, 90 |
| 379 | 2 | ND | G | 1 | 2, 20, 200 | 10, 30, 90 |
| 380 | 2 | B | F | 1 | 2, 20, 200 | 10, 30, 90 |
| 381 | 2 | ND | G | 1 | 2, 20, 200 | 10, 30, 90 |
| 382 | 2 | ND | G | 1 | 2, 20, 200 | 10, 30, 90 |
| 383 | 2 | ND | F | 1 | 2, 20, 200 | 10, 30, 90 |
| 384 | 2 | B | E | 1 | 2, 20, 200 | 10, 30, 90 |
| 385 | 2 | B | F | 1 | 2, 20, 200 | 10, 30, 90 |
| 386 | 2 | B | F | 1 | 2, 20, 200 | 10, 30, 90 |
| 387 | 2 | A | F | 1 | 2, 20, 200 | 10, 30, 90 |
| 388 | 2 | B | E | 1 | 2, 20, 200 | 10, 30, 90 |
| 389 | 2 | B | E | 1 | 2, 20, 200 | 10, 30, 90 |
| 390 | 2 | B | E | 1 | 2, 20, 200 | 10, 30, 90 |
| 391 | 2 | A | F | 1 | 2, 20, 200 | 10, 30, 90 |
| 392 | 2 | A | D | 2 | 0.2, 2, 20 | 1, 10, 100 |
| 393 | 2 | A | D | 2 | 0.2, 2, 20 | 1, 10, 100 |
| 394 | 2 | ND | D | 2 | 0.2, 2, 20 | 1, 10, 100 |
| 395 | 2 | A | D | 2 | 0.2, 2, 20 | 1, 10, 100 |
| 396 | 2 | A | C | 2 | 0.2, 2, 20 | 1, 10, 100 |

**[Table 7-4-1]**

| Seq ID | | | | | | | | | | |
|---|---|---|---|---|---|---|---|---|---|---|
| No. | 1 | 2 | 3 | 4 | 5 | 6 | 7 | 8 | 9 | 10 |
| 397 | F | G | MeF4Me | G | Bph | G | D | D | Cha | A |
| 398 | F4F | G | MeF4Me | G | Bph | G | D | D | Cha | A |
| 399 | F | G | MeF | G | Bph | G | D | E | Cha | Q |
| 400 | F | G | MeF | G | Bph | G | D | E | Cha | Q |
| 401 | F | G | MeF | G | Bph | G | D | E | Cha | Hgl |
| 402 | F | G | MeF | G | Bph | G | D | E | Cha | Hgl |
| 403 | F | G | MeF | G | Bph | G | D | E | Cha | Hgl |
| 404 | F | G | MeF | G | Bph | G | D | E | Cha | Q |
| 405 | F | G | MeF | G | Bph | G | D | E | Cha | Q |
| 406 | F | G | MeF | G | Bph | G | D | E | Cha | Q |
| 407 | F | G | MeF | G | Bph | G | D | E | Cha | Q |
| 408 | F | G | MeF | G | Bph | G | D | E | Cha | Hgl |
| 409 | F | G | MeF | G | Bph | G | D | E | Cha | Hgl |
| 410 | F | G | MeF | G | 3Pv6Ph | G | D | E | Cha | Hgl |
| 411 | F | G | MeF | G | 3Pv6Ph | G | D | E | Cha | Hgl |
| 412 | F4F | G | MeF | G | 3Pv6Ph | G | D | E | Cha | Hgl |
| 413 | F4F | G | MeF | G | 3Pv6Ph | G | D | E | Cha | Hgl |
| 414 | F | G | MeF | G | 3Py6Ph | G | D | E | Cha | Hgl |
| 415 | F | G | MeF | G | 3Py6Ph | G | D | E | Cha | Hgl |
| 416 | F4F | G | MeF | G | 3Pv6Ph | G | D | E | Cha | Hgl |
| 417 | F4F | G | MeF | G | 3Pv6Ph | G | D | E | Cha | Hgl |
| 418 | F | G | MeF | G | 3Pv6Ph | G | D | E | Cha | Hgl |
| 419 | F | G | MeF | G | 3Pv6Ph | G | D | E | Cha | Hgl |
| 420 | F4F | G | MeF | G | 3Pv6Ph | G | D | E | Cha | Hgl |
| 421 | F4F | G | MeF | G | 3Pv6Ph | G | D | E | Cha | Hgl |

**[Table 7-4-2]**

| Seq ID | | | | | | | | Ret time | **ESI** (m/z) |
|---|---|---|---|---|---|---|---|---|---|
| No. | 11 | 12 | 13 | 14 | additional amino acids | | | | |
| 397 | MeF4Me | L | H | C | G | -NH2 | | 6.156 | 908.3 |
| 398 | MeF4Me | L | H | C | G | -NH2 | | 6.204 | 917.3 |
| 399 | MeF | L | Abu | C | G | -NH2 | | 3.652 | 903.3 |
| 400 | MeF | L | P4Sh | C | G | -NH2 | | 3.256 | 917.3 |
| 401 | MeF | L | F4COO | C | G | -NH2 | | 3.56 | 963.8 |
| 402 | MeF | L | Abu | C | G | -NH2 | | 3.844 | 910.8 |
| 403 | MeF | L | P4Sh | C | G | -NH2 | | 3.44 | 924.8 |
| 404 | MeF | L | F4COO | C | -NH2 | | | 3.596 | 927.8 |
| 405 | MeF | L | F4COO | C | G | OH | | 3.476 | 956.9 |
| 406 | MeF | L | F4COO | C | bA | OH | | 3.504 | 963.9 |
| 407 | MeF | L | F4COO | C | bA | de | OH | 3.168 | 1028 |
| 408 | MeF | L | F4COO | C | -NH2 | | | 3.857 | 935.4 |
| 409 | MeF | L | F4COO | C | bA | de | OH | 3.317 | 1036 |
| 410 | MeF | L | F4COO | C | -NH2 | | | 5.7 | 935.8 |
| 411 | MeF | L | F4COO | C | bA | de | OH | 5.044 | 1037 |
| 412 | MeF4Me | L | F4COO | C | -NH2 | | | 6.216 | 952 |
| 413 | MeF4Me | L | F4COO | C | bA | de | OH | 5.88 | 1053 |
| 414 | MeF | L | P4Sh | C | -NH2 | | | 5.14 | 897.2 |
| 415 | MeF | L | P4Sh | C | bA | de | OH | 5.396 | 997.5 |
| 416 | MeF4Me | L | P4Sh | C | -NH2 | | | 6.012 | 912.9 |
| 417 | MeF4Me | L | P4Sh | C | bA | de | OH | 5.48 | 1013 |
| 418 | MeF | L | Abu | C | -NH2 | | | 5.948 | 882.9 |
| 419 | MeF | L | Abu | C | bA | de | OH | 5.576 | 983.5 |
| 420 | MeF4Me | L | Abu | C | -NH2 | | | 5.852 | 899.2 |
| 421 | MeF4Me | L | Abu | C | bA | de | OH | 6.04 | 999.5 |

**[Table 7-4-3]**

| Seq ID | [M+XH]X+ | aLISA BMP4 (0.2 nM) % inh | aLISA BMP7 (10nM) % inh | Synthesis | Peptide Conc. in Assay (nM) | |
|---|---|---|---|---|---|---|
| No. | | | | | BMP4 Assay | BMP7 Assay |
| 397 | 2 | ND | D | 2 | 0.2, 2, 20 | 1, 10, 100 |
| 398 | 2 | A | D | 2 | 0.2, 2, 20 | 1, 10, 100 |
| 399 | 2 | ND | C | 2 | 0.2, 2, 20 | 1, 10, 100 |
| 400 | 2 | ND | C | 2 | 0.2, 2, 20 | 1, 10, 100 |
| 401 | 2 | ND | C | 2 | 0.2, 2, 20 | 1, 10, 100 |
| 402 | 2 | ND | C | 2 | 0.2, 2, 20 | 1, 10, 100 |
| 403 | 2 | ND | C | 2 | 0.2, 2, 20 | 1, 10, 100 |
| 404 | 2 | ND | C | 2 | 0.2, 2, 20 | 1, 10, 100 |
| 405 | 2 | ND | D | 2 | 0.2, 2, 20 | 1, 10, 100 |
| 406 | 2 | ND | D | 2 | 0.2, 2, 20 | 1, 10, 100 |
| 407 | 2 | ND | C | 2 | 0.2, 2, 20 | 1, 10, 100 |
| 408 | 2 | ND | C | 2 | 0.2, 2, 20 | 1, 10, 100 |
| 409 | 2 | ND | D | 2 | 0.2, 2, 20 | 1, 10, 100 |
| 410 | 2 | ND | D | 2 | 0.2, 2, 20 | 1, 10, 100 |
| 411 | 2 | ND | D | 2 | 0.2, 2, 20 | 1, 10, 100 |
| 412 | 2 | ND | D | 2 | 0.2, 2, 20 | 1, 10, 100 |
| 413 | 2 | ND | D | 2 | 0.2, 2, 20 | 1, 10, 100 |
| 414 | 2 | ND | D | 2 | 0.2, 2, 20 | 1, 10, 100 |
| 415 | 2 | ND | D | 2 | 0.2, 2, 20 | 1, 10, 100 |
| 416 | 2 | ND | D | 2 | 0.2, 2, 20 | 1, 10, 100 |
| 417 | 2 | ND | C | 2 | 0.2, 2, 20 | 1, 10, 100 |
| 418 | 2 | ND | D | 2 | 0.2, 2, 20 | 1, 10, 100 |
| 419 | 2 | ND | D | 2 | 0.2, 2, 20 | 1, 10, 100 |
| 420 | 2 | ND | D | 2 | 0.2, 2, 20 | 1, 10, 100 |
| 421 | 2 | ND | D | 2 | 0.2, 2, 20 | 1, 10, 100 |

### [Example 17]

### Synthesis of (2S)-2-({[(9H-fluoren-9-yl)methoxy]carbonyl}amino)-3-{3-fluoro-[1,1'-biphenyl]-4-yl}propanoic acid (pBph3F)

In the present Example, an amino acid having the following structure used in the present invention was synthesized.

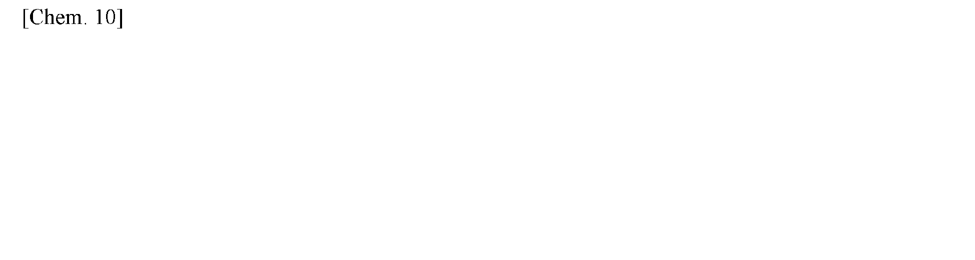

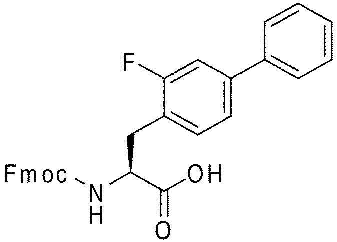

Under nitrogen atmosphere, to a mixture solution of toluene (200 mL), water (200 mL), and ethanol (50 mL) of 1-bromo-2-fluoro-4-iodobenzene (15.0 g, 49.9 mmol) and phenylboronic acid, sodium carbonate (10.6 g, 54.9 mmol) and [1,1'-bis(diphenylphosphino)ferrocene]palladium(II)dichloride dichloromethane adduct (2.04 g, 5.50 mmol) were added, and the mixture was stirred at 110°C for 16 hours. Then, the resultant was extracted with ethyl acetate (100 mL) three times. The combined organic extracted product was washed with saturated saline (100 mL) three times, dried over anhydrous sodium sulfate, and filtrated. Then, the filtrate was concentrated. The obtained residues were purified by silica gel chromatography (ethyl acetate:petroleum ether = 1:25).

Under nitrogen atmosphere, to a DMF (200 mL) suspension of zinc (6.25 g, 95.6 mmol), methyl(2R)-2-({[(9H-fluoren-9-yl)methoxy]carbonyl}amino)-3-iodopropanate (17.3 g, 38.2 mmol) and iodine (2.44 g, 9.61 mmol) were added, and the mixture was stirred at room temperature for 30 minutes. To this reaction liquid, a part of the obtained product described above (8.00 g, 31.9 mmol), tris((1E,4E)-1,5-diphenylpenta-1,4-dien-3-one)dipalladium (0.730 g, 0.797 mmol), and dicyclohexyl ({2',6'-dimethoxy-[1,1'-biphenyl]-2-yl})phosphane (0.640 g, 1.56 mmol) were added, and the mixture was stirred at 50°C for about 16 hours. The solid was filtered, and 600 mL of water was added to the filtrate, to stop reaction. Then, the resultant was extracted with ethyl acetate (200 mL) three times. The combined organic extracted product was washed with saturated saline (200 mL) three times, dried over anhydrous sodium sulfate, and filtrated. Then, the filtrate was concentrated. The obtained residues were purified by silica gel column chromatography (ethyl acetate:petroleum ether = 1:3).

To a mixture solution of 2-propanol (120 mL), water (40 mL), and THF (40 mL) of the obtained product (12 g, 24.4 mmol), calcium chloride (43.0 g, 387 mmol) was added, and the mixture was stirred at 0°C for 10 minutes. To this reaction liquid, lithium hydroxide monohydrate (4.06 g, 96.8 mmol) was added, and the mixture was stirred at room temperature for about 16 hours. After the reaction liquid was adjusted to pH 7 using an aqueous solution of citric acid, the solid was filtered, and was washed with methanol:dichloromethane = 1/10 (200 mL). The filtrate and the washing liquid were combined, and the organic layer was dried over anhydrous sodium sulfate, followed by filtration. Then, the filtrate was concentrated. The obtained residues were washed with diethyl ether and were dried under reduced pressure, to obtain a compound as shown above. ¹H NMR(500MHz,DMSO-d₆) δ7.88(d,J=7.5Hz,2H), 7.69-7.53(m,4H),7.48-7.21(m,11H),6.76-6.14(m,1H),4.25-3.85(m,4H),3.31-3.11(m,1H),2.88-2.66(m,1H).LC-MS(ESI,m/z):[M-H]⁻ 480.

If any discrepancy between the tables and the sequence listing in this specification is found, the statements in the tables shall be interpreted as being correct.

### INDUSTRIAL APPLICABILITY

This invention may be used in the pharmaceutical and biotechnology industries.

## Claims

1. A peptide or a pharmaceutically acceptable salt thereof, the peptide comprising:
an amino acid sequence described below:
E-R-V-F4COO-Atp-D-R-Bph-P-Y-P-Bph-Y-F4COO-F4COO-S-C (SEQ ID NO: 1); or
an amino acid sequence resulting from substitution, addition, deletion, or insertion of 1 to 15 amino acid residues selected from the group consisting of amino acid residues at positions 1, 2, 4, 5, 6, 7, 8, 9, 10, 11, 12, 13, 14, 15, and 16 of the amino acid sequence described above.

2. A peptide or a pharmaceutically acceptable salt thereof, the peptide comprising:
an amino acid sequence described below:
X1-X2-X3-X4-X5-X6-X7-X8-X9-X10-X11-X12-X13-X14-X15-X16-X17 (SEQ ID NO: 2); or
an amino acid sequence resulting from substitution, deletion, addition, or insertion of 1 to 10 amino acid sequences from the amino acid sequence set forth in SEQ ID NO: 2,
wherein X1 is E, A, T, Q, or L,
X2 is R, K, Hgl, any polar amino acid, or any aliphatic amino acid,
X3 is V,
X4 is an amino acid having an aromatic ring that may be a heterocycle at a side chain,
X5 is any aliphatic amino acid, Y, D, Atp, Cha4tH, Gthp, or A1Ac4pip,
X6 is D, any basic amino acid, or any polar amino acid,
X7 is any basic amino acid or any polar amino acid,
X8 is biphenylalanine that may have a substituent and in which C on an aromatic ring may be substituted with N,
X9 is any amino acid,
X10 is Y, Hty, or 4Py,
X11 is any amino acid,
X12 is biphenylalanine that may have a substituent and in which C on an aromatic ring may be substituted with N,
X13 is unsubstituted or substituted F, 4Py, or 3Py6NH2,
X14 is F4COO, D, or G,
X15 is F4COO, Y, 4Py, 3Py6NH2, W7N, Hph, or N,
X16 is any amino acid, and
X17 is C.

3. The peptide or the pharmaceutically acceptable salt thereof according to claim 2,
wherein X1 is E,
X2 is R or any polar amino acid,
X3 is V,
X4 is unsubstituted or substituted Y or F4COO, X5 is V or Atp,
X6 is D or KCOpipzaa,
X7 is R,
X8 is Bph,
X9 is P or S,
X10 is Y or Hty,
X11 is any aliphatic amino acid,
X12 is unsubstituted or substituted Bph,
X13 is unsubstituted or substituted F,
X14 is F4COO,
X15 is F4COO or 4Py,
X16 is any amino acid, and
X17 is C.

4. The peptide or the pharmaceutically acceptable salt thereof according to claim 2,
wherein X1 is E, A, T, Q, or L,
X2 is R or KCOpipzaa,
X3 is V,
X4 is Y, F4F, F4COO, 4Py, H, or F4aaO,
X5 is any aliphatic amino acid, Atp, Cha4tH, Gthp, or A1Ac4pip,
X6 is D, S, or KCOpipzaa,
X7 is R or K,
X8 is 3Py6Ph, Bph, Bph4C, pBph2F, or Bph3C,
X9 is S, P, KCOpipzaa, or H,
X10 is Y or Hty,
X11 is any aliphatic amino acid or Hyp,
X12 is biphenylalanine that may have a substituent,
X13 is unsubstituted or substituted F or 4Py,
X14 is F4COO,
X15 is F4COO or 4Py,
X16 is S, and
X17 is C.

5. The peptide or the pharmaceutically acceptable salt thereof according to claim 2,
wherein the peptide includes an amino acid sequence described below: X1-X2-X3-X4-X5-X6-X7-X8-X9-X10-X11-X12-X13-X14-X15-X16-C (SEQ ID NO: 4), or an amino acid sequence resulting from substitution, deletion, addition, or insertion of 1 to 10 amino acid sequences from the amino acid sequence set forth in SEQ ID NO: 4, and
wherein X1 is A,
X2 is R, KAc, Q, or KCOpipzaa,
X3 is V,
X4 is Y, F4F, or 4Py,
X5 is V,
X6 is D, KCOpipzaa, K, R, G, or H,
X7 is R, K, KAc, 4Py, S, or Cit,
X8 is 3Py6Ph or Bph,
X9 is P, S, K, H, G, S, or KCOpipzaa,
X10 is Y,
X11 is any aliphatic amino acid,
X12 is Bph or 3Py6Ph,
X13 is Y or F4F,
X14 is F4COO,
X15 is F4COO or 4Py, and
X16 is S.

6. The peptide or the pharmaceutically acceptable salt thereof according to any one of claims 1 to 5,
wherein the peptide or the pharmaceutically acceptable salt thereof has binding ability to BMP4.

7. The peptide or the pharmaceutically acceptable salt thereof according to any one of claims 1 to 5,
wherein the peptide or the pharmaceutically acceptable salt thereof has inhibitory activity of BMP4.

8. A peptide or a pharmaceutically acceptable salt thereof,
wherein the peptide is the peptide according to any one of claims 1 to 7, and includes an amino acid sequence set forth in any one of SEQ ID NOs: 5 to 187 and SEQ ID NOs: 243 to 421, or
wherein the peptide includes an amino acid sequence resulting from substitution, deletion, addition, or insertion of 1 to 10 amino acid sequences from the amino acid sequence set forth in any one of SEQ ID NOs: 5 to 187 and SEQ ID NOs: 243 to 421, and has binding ability to BMP4 or inhibitory activity of BMP4.

9. A peptide or a pharmaceutically acceptable salt thereof, the peptide comprising:
an amino acid sequence described below:
F-G-MeF-G-Bph-G-D-D-Cha-A-MeF-L-H-C (SEQ ID NO: 241); or
an amino acid sequence resulting from substitution, addition, deletion, or insertion of 1 to 12 amino acid residues selected from the group consisting of amino acid residues at positions 1, 3, 4, 5, 6, 7, 8, 9, 10, 11, 12, and 13 of the amino acid sequence described above.

10. A peptide or a pharmaceutically acceptable salt thereof, the peptide comprising:
an amino acid sequence described below:
X1-X2-X3-X4-X5-X6-X7-X8-X9-X10-X11-X12-X13-X14 (SEQ ID NO: 242); or
an amino acid sequence resulting from substitution, deletion, addition, or insertion of 1 to 12 amino acid sequences from the amino acid sequence set forth in SEQ ID NO: 245,
wherein X1 is phenylalanine that may have a substituent and in which C on an aromatic ring may be substituted with N,
X2 is G,
X3 is N-methylphenylalanine that may have a substituent,
X4 is G, an aliphatic amino acid or a polar amino acid that may be a D form,
X5 is biphenylalanine in which C on an aromatic ring may be substituted with N or phenylalanine that may have a substituent,
X6 is G, or A that may be a D form,
X7 is any polar amino acid,
X8 is any acidic amino acid,
X9 is any aliphatic amino acid,
X10 is any amino acid,
X11 is N-methylphenylalanine that may have a substituent,
X12 is any aliphatic amino acid,
X13 is any amino acid, and
X14 is C.

11. The peptide or the pharmaceutically acceptable salt thereof according to claim 9 or 10,
wherein the peptide or the pharmaceutically acceptable salt thereof has inhibitory activity of BMP7.

12. The peptide or the pharmaceutically acceptable salt thereof according to claim 9 or 10,
wherein the peptide includes an amino acid sequence set forth in any one of SEQ ID NOs: 243 to 421, or
wherein the peptide includes an amino acid sequence resulting from substitution, deletion, addition, or insertion of 1 to 10 amino acid sequences from an amino acid sequence set forth in any one of SEQ ID NOs: 243 to 421, and has inhibitory activity of BMP7.

13. The peptide or the pharmaceutically acceptable salt thereof according to any one of claims 1 to 12,
wherein the peptide is a cyclic peptide.

14. The peptide or the pharmaceutically acceptable salt thereof according to claim 13,
wherein the peptide includes a cyclic structure in which a chloroacetylated amino acid and a cysteine residue contained in the peptide are bound.

15. The peptide or the pharmaceutically acceptable salt thereof according to any one of claims 1 to 14, further comprising an additional amino acid residue sequence.

16. A BMP4 and/or BMP7 signal transduction inhibitor comprising
the peptide or the pharmaceutically acceptable salt thereof according to any one of claims 1 to 15.

17. A human stem cell differentiation induction regulator comprising
the peptide or the pharmaceutically acceptable salt thereof according to any one of claims 1 to 15.

18. A culture medium supplement for culturing human stem cells, comprising
one or two or more kinds of the peptide or the pharmaceutically acceptable salt thereof according to any one of claims 1 to 15, the BMP4 and/or BMP7 signal transduction inhibitor according to claim 16, and the human stem cell differentiation induction regulator according to claim 17.

19. A method for inhibiting signal transduction activity of BMP4 and/or BMP7 using the peptide or the pharmaceutically acceptable salt thereof according to any one of claims 1 to 15.

20. A method for regulating differentiation induction of human stem cells using the peptide or the pharmaceutically acceptable salt thereof according to any one of claims 1 to 15.
